# EUROPEAN PATENT SPECIFICATION

(11) **EP 1 928 537 B1**
(45) Date of publication and mention of the grant of the patent: **21.01.2015**
(21) Application number: 06814198.5
(22) Date of filing: 05.09.2006
(51) Int. Cl.: A61M 37/00, A61M 35/00, A61B 17/00, A61B 17/20, A61B 19/00, A61F 13/40

(54) **Devices for transcutaneous delivery of vaccines and transdermal delivery of drugs**
Vorrichtungen für die transkutane Abgabe von Vakzine und transdermale Abgabe von Arzneimittel
Dispositifs d'administration transcutanée de vaccins et d'administration transdermique de médicaments

(30) Priority: 02.09.2005 US 714073 P
(43) Date of publication of application: 11.06.2008
(60) Divisional application: 10011210.1
(73) Proprietor: Intercell USA, Inc., Gaithersburg, MD 20878 (US)
(72) Inventor: GLENN, Gregory, M., Poolesville, MD 20837 (US); DOERKSEN, Kyle, J., Los ALtos Hills, CA 94022 (US); ELLINGSWORTH, Larry, R., Rockville, MD 20850 (US); EPPERSON, Diane, E., Bethesda, MD 20816 (US); FRERICHS, David, Poolesville, MD 20837 (US); HAMER, Mervyn, L., Gaithersburg, MD 20878 (US); HENNINGSGAARD, Aaron, A., San Francisco, CA 94115, (US); INOUYE, Matthew, D., Foster City, CA 94404 (US); JAMES, Adrian, B., Oakland, CA 94618 (US); KAPLAN, Jonathan, I., Palo Alto, CA 94306 (US); LAI, John, W., San Bruno, CA 94066 (US); LISTER, Robert, I., Dronfield, S18 2FN (GB); LUBENSKY, David, A., San Francisco, CA 94114 (US); MACDONALD, Peter, S., Palo Alto, CA 94303 (US); MADSEN, Sally, H., San Francisco, CA 94107 (US); MASON, Brian, J., Simsbury, CT 06070 (US); MCELHANEY, Christine, W., Palo Alto, CA 94301 (US); MOHRI, Lionel, Santa Clara, CA 95051 (US); PIGLIACAMPO, Anthony, C., Menlo Park, CA 94025 (US); ROMERO, Gina, L., Soquel, CA 95073 (US); STEEVES, Charles, F., III, Pte. du Chene, New Brunswick, E4P 4A8 (CA); VILLAR, Christina, P., North Potomac, MD 20878 (US); YU, Jianmei, Bethesda, MD 20817 (US)
(74) Representative: Vossius & Partner
(86) International application number: PCT/US2006/034620
(87) International publication number: WO 2007/028167

(56) References cited:
- WO-A-03/074102
- WO-A-03/084597
- WO-A-2004/009172
- WO-A-2004/019777
- WO-A-2004/098684
- WO-A-2005/049107
- WO-A-2006/055795
- WO-A-2007/002523
- WO-A2-02/074244
- US-A- 5 599 545
- US-A1- 2002 193 729
- US-B1- 6 797 276
- RICHARD W. ET AL.: 'Parenteral Adjuvant Activities of Escherichia coli Heat-Labile Toxin and its B Subunit for Immunization of Mice against Gastric Helicobacter pylori Infection' INFECT. IMMUN. vol. 68, no. 5, May 2000, pages 2775 - 2782, XP008126642

## Description

The disclosure relates to the fields of vaccine and drug delivery devices.

Needle-free delivery has become a global priority because of the risk of needle-borne diseases associated with re-use and improper disposal of needles. Needle-free delivery of therapeutic agents provides a safe, convenient, and non-invasive alternative to oral or parenteral delivery techniques. Other advantages of needle-free delivery include elimination of needle pain, the risk of introducing infection to treated individuals, the risk of contamination or infection of health care workers caused by accidental needle-sticks and the disposal of used needles.

WO 2004/098684 relates to skin-mountable devices and WO 03/084597 discloses a method and a device for delivering a substance intradermally to a patient.

However, the barrier function of the skin appears to have prevented the effective delivery of therapeutic agents via the skin to date. The reason is that the skin, the largest human organ, plays an important part in the body's defense against invasion by infectious agents and contact with noxious substances.

Accordingly, there is a need to develop a device for physical disruption of one or more layers of the skin to enhance the delivery of therapeutic agents via the skin.

The present invention is characterized by the embodiments as defined in the claims. Thus, it relates to the following items:
1. A system comprising a device for disrupting skin, said device comprising a mask and a disrupting member mechanically coupled to said mask, wherein said mask has an opening, which defines the location and the size of the skin to be disrupted, and comprises a fastener to secure said mask to skin and characterized in that said disrupting member further comprises:
   (a) a snap dome mounted on the top of the mask assembly, and
   (b) a disrupting strip,
      wherein said snap dome is adapted to provide a sufficient amount of pressure, when being depressed, to disrupt the skin as the strip is pulled over said opening in said mask.
2. The system of item 1, characterized in that said device further comprises:
   (a) a bridge bonded onto the mask, and
   (b) a button assembled so that said snap dome is positioned between the bridge and the button.
3. The system of item 1 or 2, characterized in that said device further comprises an interlocking mechanism, comprising a clip bonded to the strip, wherein:
   (a) when no force is applied to the snap dome or button, the strip is captured inside the bridge; and
   (b) when the prescribed force is applied to the snap dome or button, the button depresses and releases the strip to be pulled out by a user.
4. The system of any of items 1 to 3, further comprising a reservoir comprising a formulation.
5. The system of item 4, wherein said reservoir is a patch.
6. The system of item 4 or 5, wherein the formulation comprises a therapeutic agent.
7. The system of item 4 or 5, wherein said formulation comprises an antigen and a pharmaceutically acceptable carrier, wherein said antigen is capable of inducing an immune response when applied transcutaneously on the area of the subject's skin treated with said system; and wherein treating the area of the skin with said system enhances the immune response.
8. The system of item 7, wherein said formulation further comprises an adjuvant in an amount effective to enhance the immune response to the antigen.
9. The system of item 8, wherein the adjuvant and the antigen is a single molecule having both adjuvant and antigen properties.
10. The system of item 4 or 5, wherein said formulation consists of an adjuvant and a pharmaceutically acceptable carrier and is capable of enhancing an antigen-specific immune response when applied transcutaneously on the area of the subject's skin treated with said system, and wherein the antigen is not administered by a transcutaneous route.
11. The system of any of items 1 to 10, wherein said disrupting strip comprises a metal or ceramic abrasive.
12. The system of any of items 1 to 11, wherein said fastener comprises an adhesive.
13. The system of any of items 1 to 12, wherein said mask further comprises an opening for marking the skin while said mask is adhered to the skin, wherein a marking on the skin made through said opening defines the location of disruption.
14. The system of any of items 1 to 12, wherein said mask further comprises an indicator disposed to transfer to the skin when the mask is fastened thereto, wherein said indicator denotes the location of skin disruption.
15. The system of any of items 5 to 10, wherein said patch further comprises an adhesive for adhering to skin and a release liner disposed over said adhesive.

The disclosure provides devices for the disruption of one or more layers of skin and methods of their use to administer therapeutic agents, e.g., antigens or drugs. The therapeutic agents may be provided in a formulation.

The devices are designed to disrupt a defined area of skin. The defined area can approximate the area that a patch or other suitable vehicle for therapeutic agent, e.g., drug or vaccine, delivery is designed to contact. The treated area may be larger or smaller than the delivery vehicle. The area may be defined by marks, such as those made by a washable ink pen, fingerprint ink and treating the exposed skin in a hole in a mask, over which an disrupting member is passed, or the area may be defined by rotating a disrupting member in place.

For devices that employ a mask that is secured to the skin, the disclosure provides methods of disrupting the stratum corneum by first securing the mask to the skin and then disrupting the skin. For rotating devices, the disrupting member is simply placed against the skin and actuated to effect disruption.

By "antigen" is meant to describe a substance that induces a specific immune response when presented to immune cells of an organism. For example, an antigen may be a protein, a polypeptide, a peptide, a glycoprotein, a carbohydrate, a lipid, a glycolipid, a lipoprotein, or a phospholipid. An antigen may comprise a single immunogenic epitope, or a multiplicity of immunogenic epitopes recognized by a B-cell receptor (i.e., antibody on the membrane of the B cell) or a T-cell receptor. Antigen may be provided as a whole organism such as, for example, a bacterium or virion; antigen may be obtained from an extract or lysate, either from whole cells or membrane alone; or antigen may be chemically synthesized or produced by recombinant means. An antigen may be administered by itself or with an adjuvant. A single molecule may be both an antigen and an adjuvant (e.g., cholera toxin).

By "adjuvant" is meant any substance that is used to specifically or non-specifically potentiate an antigen-specific immune response, perhaps through activation of antigen presenting cells. Examples of adjuvants include an oil emulsion (e.g., complete or incomplete Freund's adjuvant, a chemokine, a cytokine, and an ADP-ribosylating exotoxin (bARE)). An adjuvant may be administered with an antigen or may be administered by itself. A single molecule may have both adjuvant and antigen properties.

By "compressed" is meant folded, rolled, crumpled, or otherwise manipulated to reduce the exposed surface area.

By "disruption" is meant any act resulting in the outer layers of the skin being compromised, for example, abrasion, scraping, rubbing, cutting, dissolution, swelling, or creating grooves of an area of stratum corneum or epidermis. Disruption may result in all or partial removal, cutting, thinning, or gouging of the effected area of the outer layers of skin, e.g., epidermis or stratum corneum. In one embodiment, the disruption results in removal or thinning of the stratum corneum in parts of the area, leaving islands of undisrupted, or less disrupted, stratum corneum. In another embodiment, disruption may include gouging into the skin 1 to 100 µm in depth, e.g., 10 to 50 µm in depth, 10-20 µm in depth, 20-60 µm in depth, or 20-100 µm in depth. As an example, the disruption may include penetration into the skin of 10 µm in depth.

By "draining lymph node field" is meant an anatomic area over which the lymph collected is filtered through a set of defined set of lymph nodes (e.g., cervical, axillary, inguinal, epitrochelear, popliteal, those of the abdomen, and thorax).

By "effective amount" is meant an amount of a therapeutic agent sufficient to induce or enhance an antigen-specific immune response, for an antigen, or treat or diagnose a condition, for a drug. Such induction of an immune response may provide a treatment such as, for example, immunoprotection, desensitization, immunosuppression, modulation of autoimmune disease, potentiation of cancer immunosurveillance, or therapeutic vaccination against an established infectious disease. Treatment includes curing, amelioration, or prevention.

By "epicutaneous" administration is meant administration to the exterior layer of the skin.

By "immunostimulation" is meant stimulation of the immune response induced by a vaccine. As an example, an immune stimulant patch containing an adjuvant may be used to stimulate the immune response of an injected vaccine.

By "therapeutic agent" is meant any chemical species or combination of chemical species capable of use in disease treatment, prevention, or diagnosis.

By "subject" is meant an animal. In one embodiment, the animal may be a mammal. In another embodiment the mammal may be a human being. The subject may be in need of treatment by delivery of a therapeutic agent, for example, transcutaneous delivery of a vaccine or transdermal delivery of a drug.

By "transcutaneous immunization" is meant passage of an antigen into the outer layers of the skin and an induction of an immune response to the antigen.

Other features and advantages will be apparent from the following description and the claims.

The drawings are not necessarily to scale.

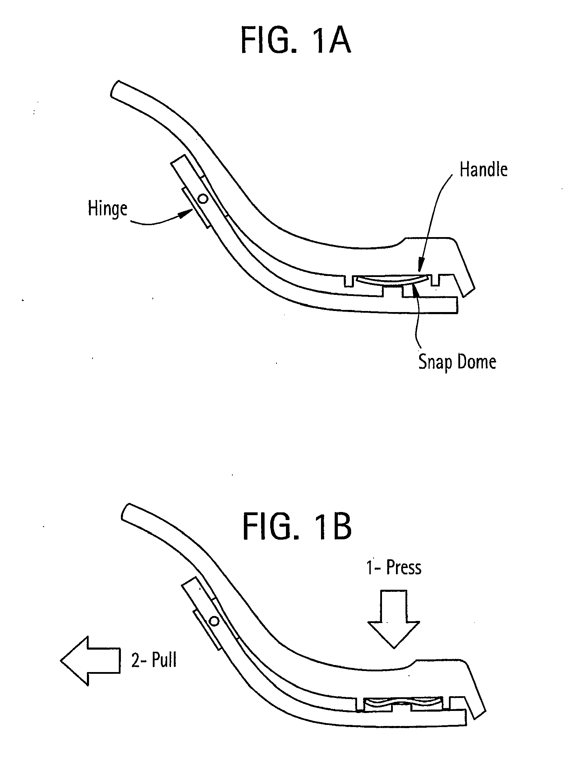

Figures 1A-1B are schematic depictions of a disrupting member that includes a snap dome to indicate when an adequate amount of pressure is applied. In Figure 1A, the disrupting member includes a handle, foot, and snap dome. The foot pivots with respect to the handle at the hinge. The under surface of the foot is covered in a material that disrupts the skin, such as, an abrasive. The snap dome is a bi-stable metal dome that reversibly deforms when a specified force is applied to it. Upon deformation, it emits a clicking sound and provides a snap feel to the user holding the handle. Figure 1B illustrates use of the disrupting member. The user presses down on the handle until a snap and click are produced and then pulls the disrupting member across the skin.

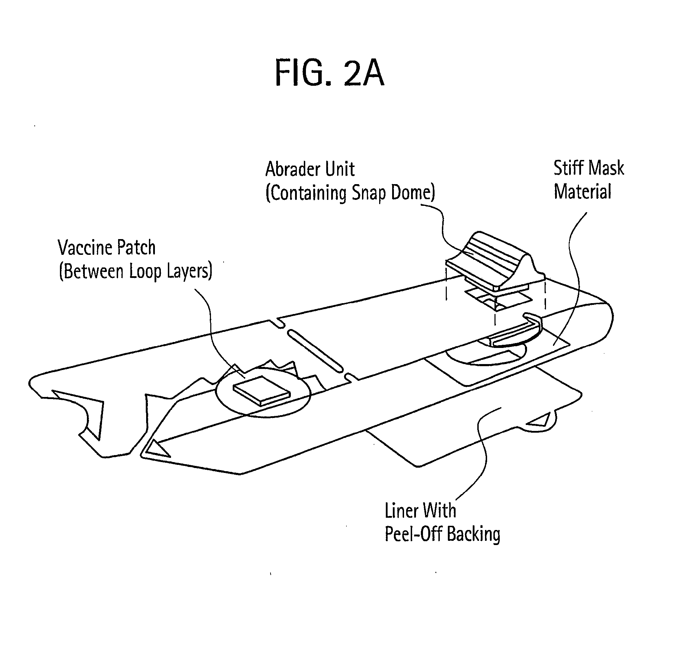

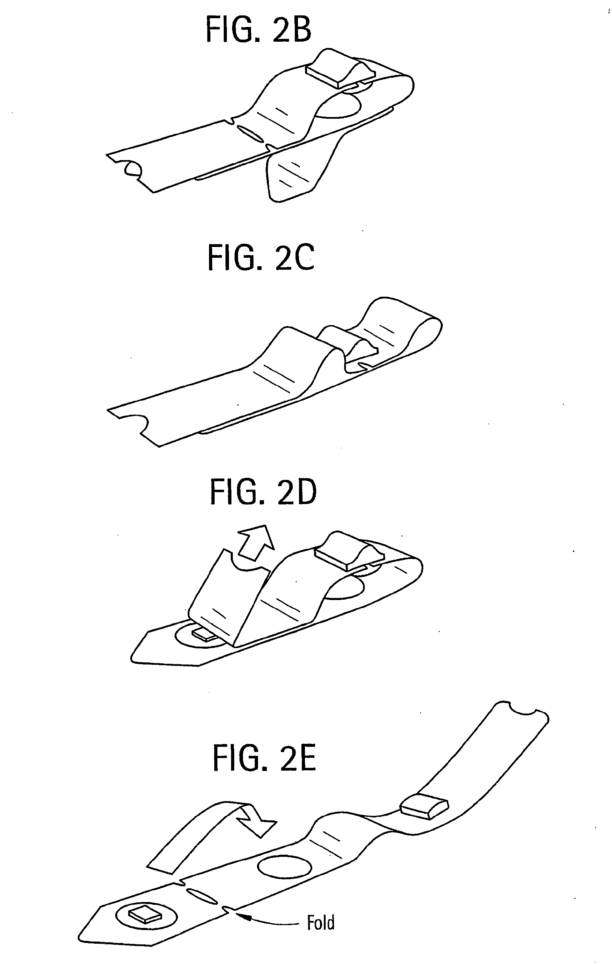

Figures 2A-2E are schematic depictions of an integrated device for disrupting a defined area of skin and applying a therapeutic agent to the disrupted area. Figure 2A shows the device prior to use. In Figure 2B, a liner is removed to expose adhesive, and the device is adhered to the skin. In Figure 2C, the user presses down on the disrupting member using the minimum amount of force and strokes the disrupting member across the opening in the mask. In Figure 2D, the user lifts a tab on the loop to expose a vaccine patch. In Figure 2E, the patch is contacted with the disrupted skin, and the device is removed leaving the patch behind.

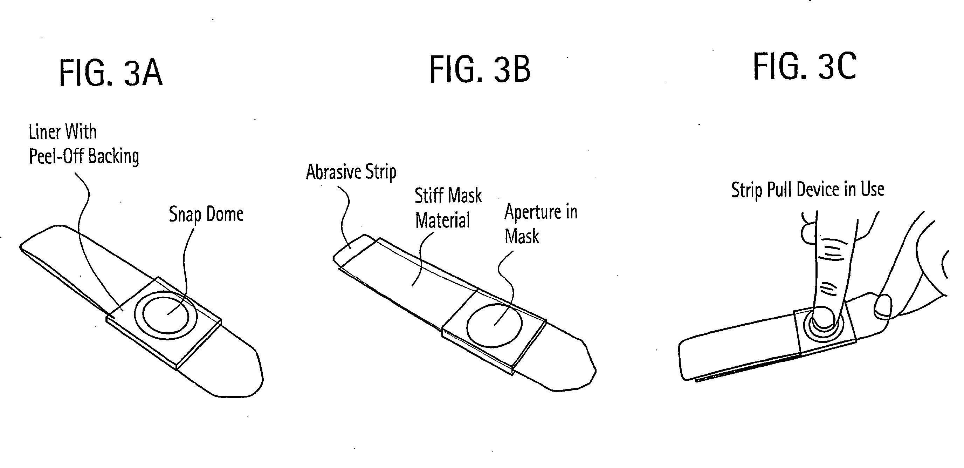

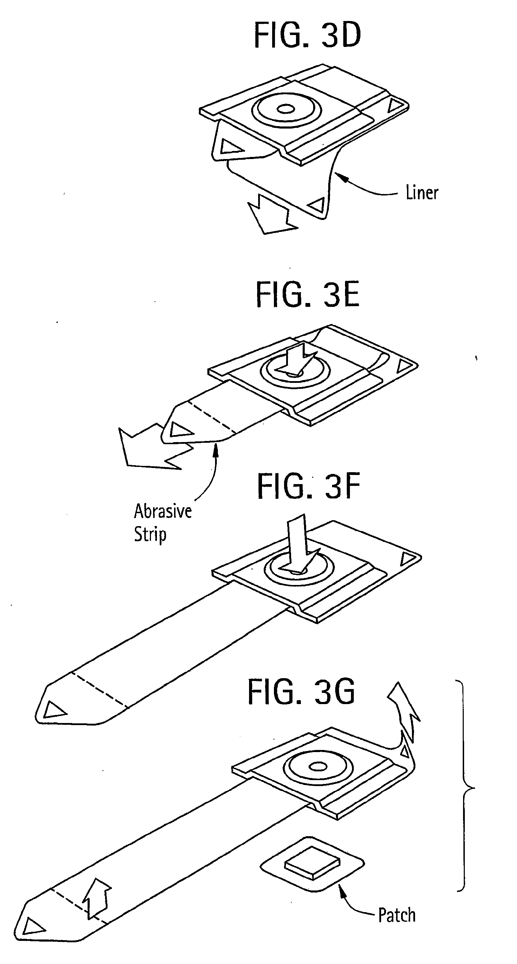

Figures 3A-3G are schematic depictions of devices including a disrupting strip for disrupting a defined area of skin through a mask. Figure 3A illustrates the one side of a strip pull device with an uncompressed strip. Figure 3B illustrates the side that contacts the skin of the same device of 3A. Figure 3C illustrates actuation of the device of 3A-3B. In Figure 3D, a liner is removed to expose adhesive, and the device is adhered to the skin. In Figure 3E, the user presses down on the disrupting member using the minimum amount of force, and pulls the disrupting strip until it is fully extended. In Figure 3F, the user again pushes on the device to attach a vaccine patch to the disrupted skin. In Figure 3G, the device is removed leaving the patch on the skin.
Figures 4A-4D are schematic depictions of a rotationally actuated disrupting device for disrupting a defined area of skin. Figure 4A illustrates a device employing a pull string for rotational actuation. Figure 4B illustrates an abrasive pad mounted on a spinning portion of the device. Figure 4C is a cross section of the device. Figure 4D illustrates how ink spots, or other indicators, may be employed to align a patch with the disrupted area.

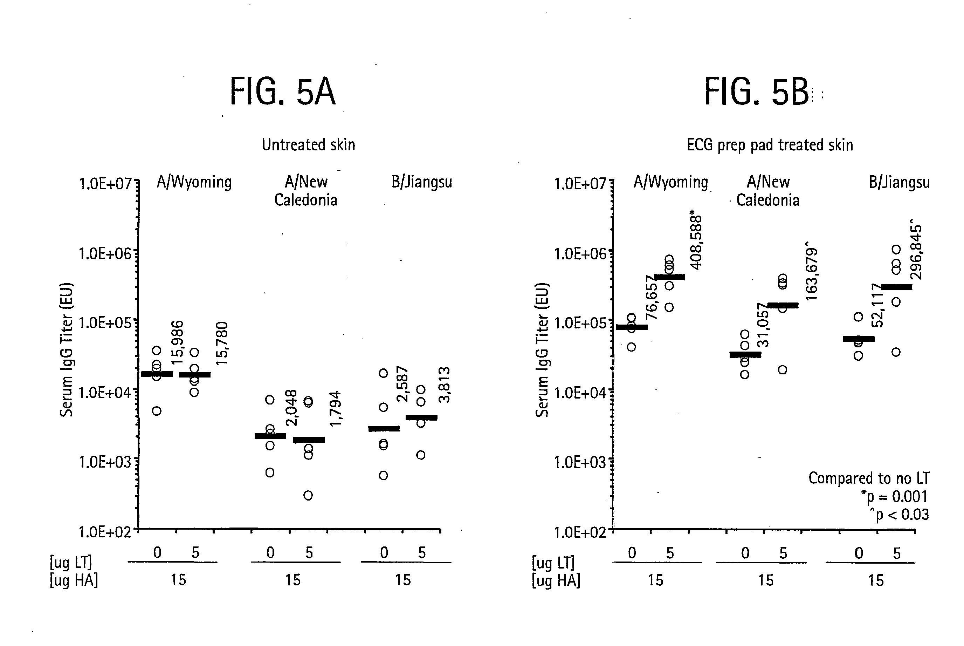

Figures 5A and 5B show the effect of skin pretreatment upon transcutaneous immunization with trivalent influenza (TIV) vaccine. Guinea pigs were immunized by priming with 0.5 µg of HA on study day 1. On study day 22, all animals were shaved and the shaved area hydrated with 10% glycerol/saline. Panel (A) the shaved skin was not pretreated. Panel (B) The shaved, hydrated area was pretreated with 15-strokes using an ECG prep pad. A dry formulated patch containing 15 µg hemagglutinin (HA) (5 µg HA per strain) alone or blended with 5 µg LT was applied to the shaved skin. Tegaderm was applied over the patch and secured with an adhesive wrap. Patches were removed the next day (18-24 hr) and the site rinsed with water. Two weeks after the second immunization (day 36), serum was collected and individual animal (o) antibody titers were determined for each of the influenza strains contained in the vaccine. The geometric mean titer for each group is indicated (bar). A T test was used to compare differences between groups receiving LT-adjuvant to a group receiving no adjuvant. (Protocol 1134-04221.026)

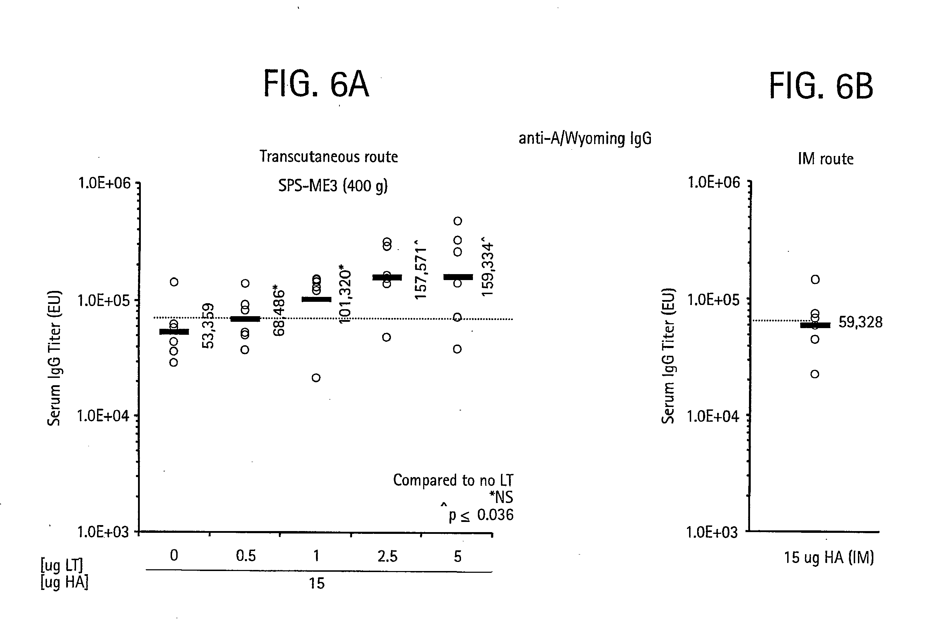

Figures 6A-6F show the immune response to transcutaneous immunization with trivalent influenza vaccine formulated in dry patch. Guinea pigs were immunized by priming with 0.5 µg HA on study day 1. On study day 22, groups receiving patches were pretreated on the shaved abdomen with SPS™-ME³ (skin preparation (or pretreatment) system-microedge cube) (2-strokes). A dry formulated patch containing 15 µg HA (5 µg per strain) was layered with a second placebo patch (no LT) or a dry patch containing 0.5 to 5 µg of LT. Patches were overlayed with Tegaderm and removed the next day. A separate group was immunized by priming with 0.5 µg HA (study day 1) and boosting (study day 22) with 15 µg HA administered by intramuscular (IM) injection. Two weeks after the second immunization (day 36), serum was collected and individual animal (o) antibody titers were determined for each of the influenza strains contained in the vaccine. The geometric mean titer for each group is indicated (bar). The dashed line indicates the geometric mean titer of the IM injected group compared to the transcuatneously immunized groups. (Protocol 5235-052)

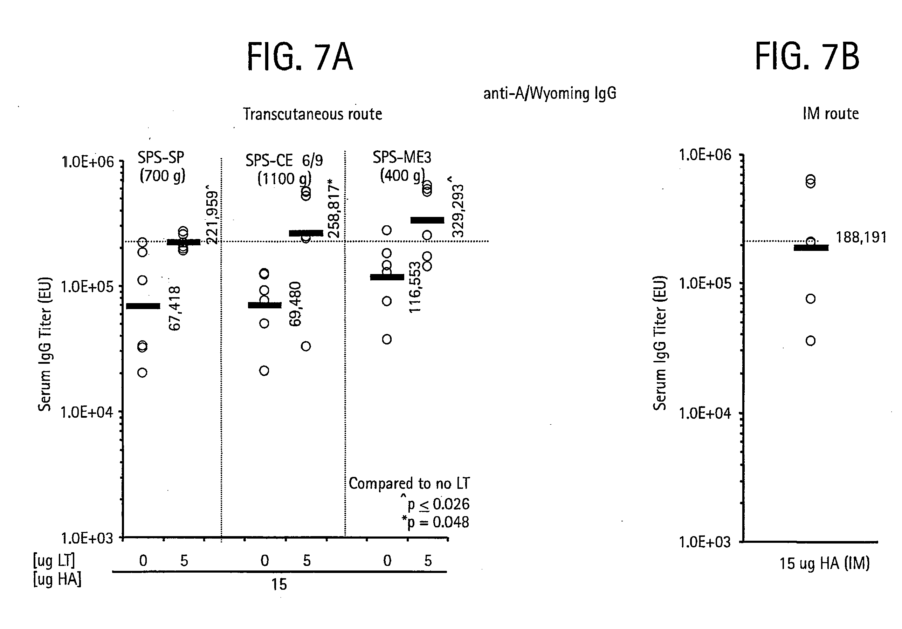

Figures 7A-7F show the comparison of Skin Pretreatment Systems (SPS™) and the immune response elicited by transcutaneous immunizaiton with influenza vaccine. Guinea pigs were primed and boosted as described in Figure 5. Groups receiving patches were pretreated on the abdomen with either the SPS™-SP (180 grit sandpaper), SPS™-CE (chemical etch) 6/9 or SPS™-ME³ and a patch formulated with 15 µg HA alone (no LT) or blended with 5 µg LT was applied to the treated skin. A separate group was immunized by priming with 0.5 µg HA (study day 1) and boosting (study day 22) with 15 µg HA administered by IM injection. Two weeks after the second immunization, serum was collected and evaluated for antibody (IgG) titers to each of the influenza strains. The geometric mean titer for each group is indicated (bar). The dashed line indicates the geometric mean titer of the IM injected group compared to the transcuatneously immunized groups. (Protocol 5235-054)

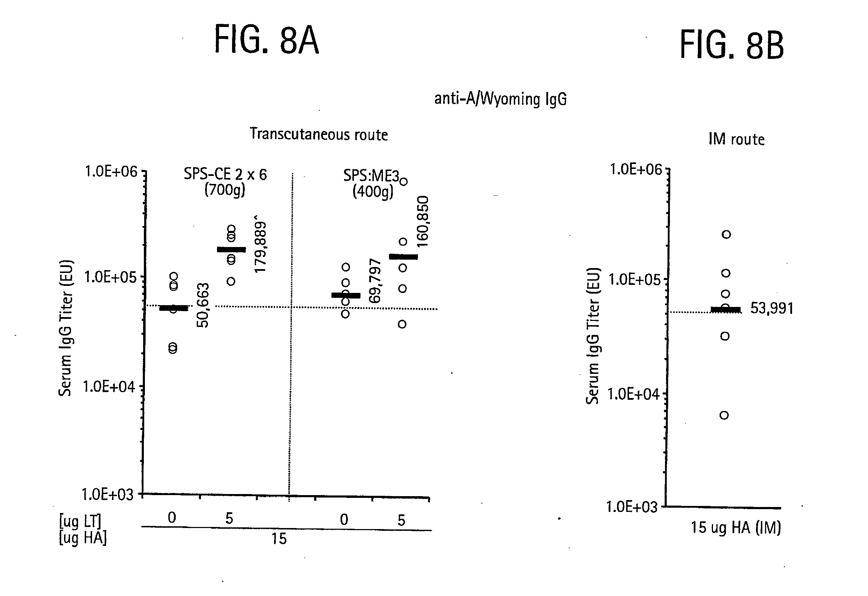

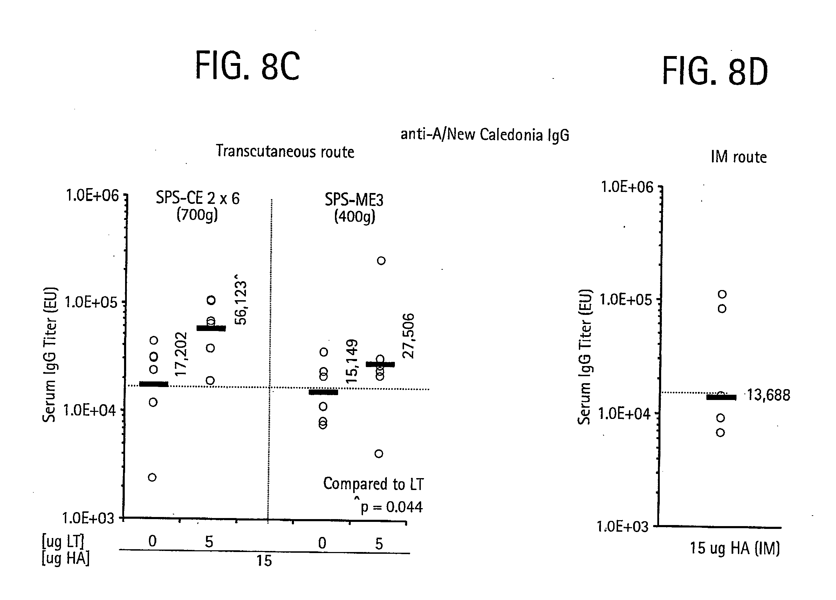

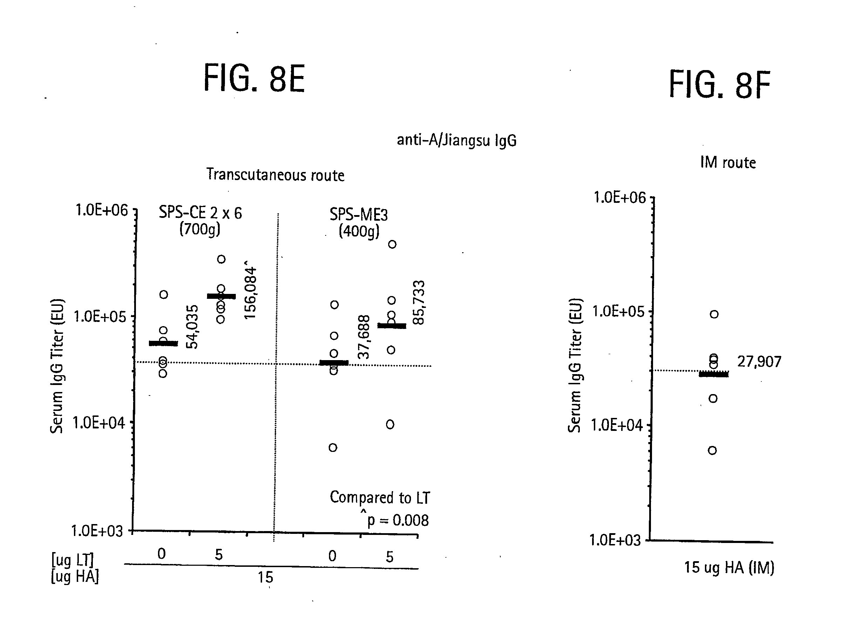

Figures 8A-8F show the comparison of SPS™-ME³ and SPS™-CE 2x6 and the immune response elicited by transcutaneous immunization with influenza vaccine. Guinea pigs were primed and boosted as described in Figure 5 and Methods. Groups receiving patches were pretreated with either the SPS™-ME³ or SPS™-CE 2x6 devices. Groups of 6 received a 1cm² dry patch formulated with 15 µg HA, which was layered with a placebo (no LT) or a dry patch containing 5 µg LT. A separate group was immunized by priming with 0.5 µg HA (study day 1) and boosting (study day 22) with 15 µg HA administered by IM injection. Two weeks after the second immunization, serum was collected and evaluated for serum antibody titers to each of the influenza strains. The dashed line indicates the geometric mean titer of the IM injected group compared to the transcuatneously immunized groups. (Protocol 5235-058)

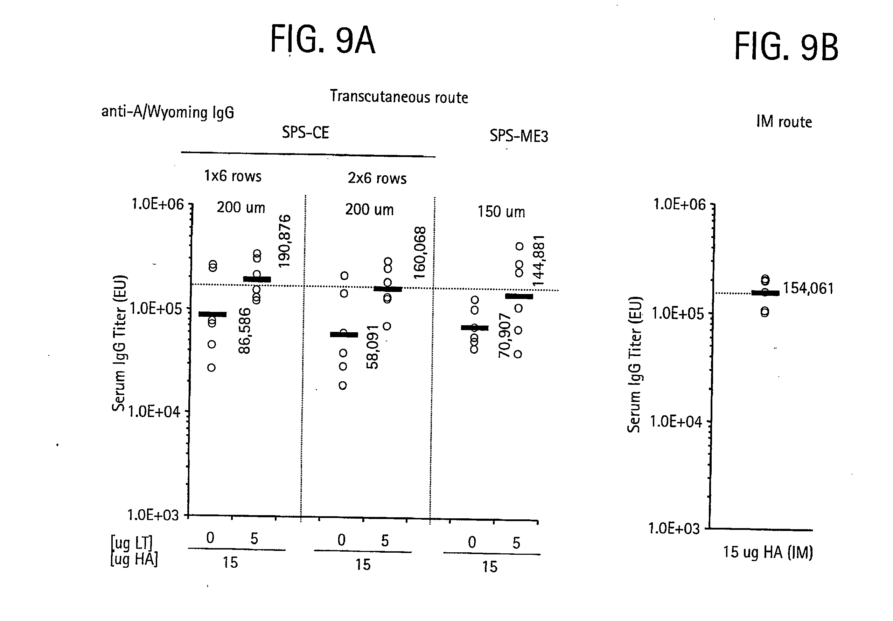

Figures 9A-9F show the comparison of the immune response to transcutaneous immunization with influenza vaccine delivered from a patch on skin pretreated with SPS™-CE (1x6) and SPS™-CE (2x6) devices. Guinea pigs were immunized by priming by IM injection with 0.5 µg HA (study day 1) and boosting with a patch or IM injection on study day 26 as described in Methods. Groups (N = 6/group) receiving patches were pretreated with SPS™-CE (1x6), SPS™-CE (2x6) or SPS™-ME³ devices. A 1cm² dry patch formulated with 15 µg HA was applied and layered with a placebo (no LT) or a dry patch containing 5 µg LT. Two weeks after the second immunization serum was collected and evaluated for serum antibody titers to each of the influenza strains. The dashed line indicates the geometric mean titer of the IM injected group compared to the transcuatneously immunized groups. (Protocol 5235-057)

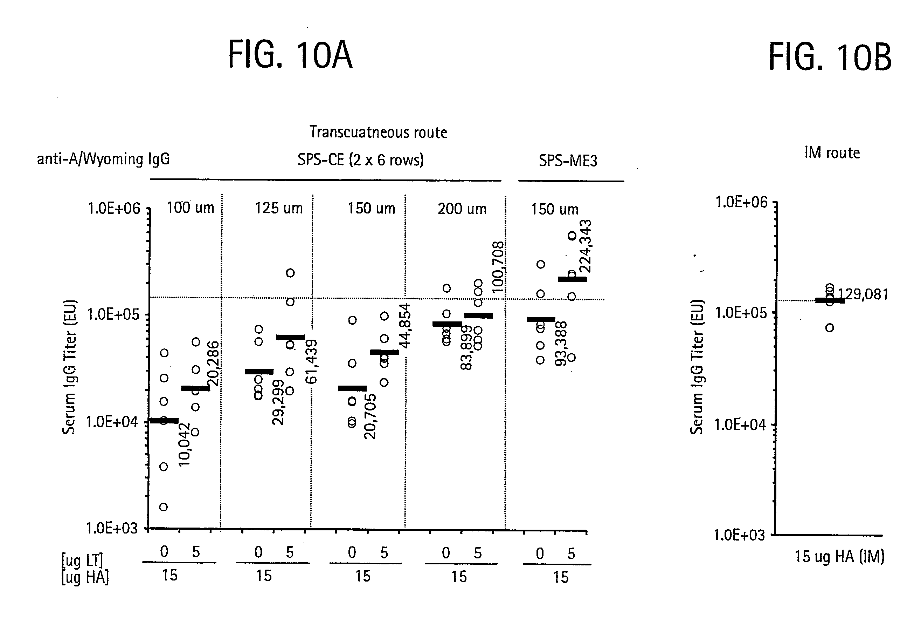

Figures 10A-10F show the evaluation of chemical etch ridge height on transcutaneous delivery of inactived influenza vaccine. Guinea pigs were immunized as described in Figure 5 and Methods. For groups receiving patches, the shaved abdomen was pretreated with SPS™-CE (2x6) device with ridges (edges) of different heights (100 µm to 200 µm) or with SPS™-ME³. A 1cm² patch formulated with 15 µg HA was applied and layered with a placebo (no LT) or a dry patch containing 5 µg LT. Two weeks after the second immunization, serum was collected and evaluated for serum antibody titers to each of the influenza strains. The dashed line indicates the geometric mean titer of the IM injected group compared to the transcuatneously immunized groups. (Protocol 5235-068)

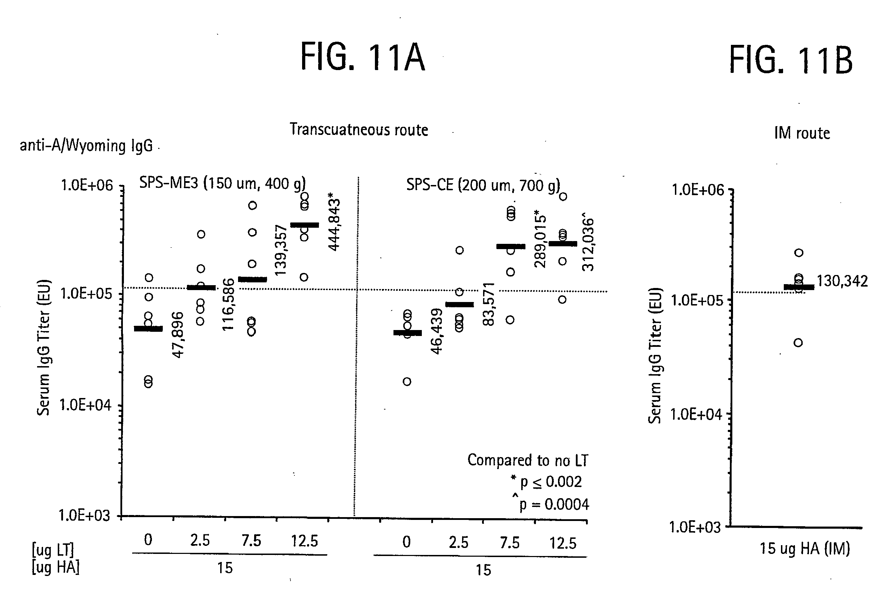

Figures 11A-11F show LT-adjuvant dose response when co-administered with influenza vaccine. Guinea pigs were immunized as described in Figure 5 and Methods. The shaved abdomen was pretreated with SPS™-CE (2x6) or SPS™-ME³: A 1cm² patch formulated with 15 µg HA was applied and layered with a placebo (no LT) or a dry patch containing different amounts of LT (2.5 to 12.5 µg). Serum was collected two weeks after the second immunization and evaluated for serum antibody titers to each of the influenza strains. The dashed line indicates the geometric mean titer of the IM injected group compared to the transcuatneously immunized groups. (Protocol 5235-059)
Figure 12 shows the anti-LT IgG antibody response for clinician administered and self-administered pretreatment. Following pretreatment, the subjects self-vaccinated by administering 50 µg LT dry patch to the anterior thigh.
Figure 13 is a schematic depiction of a strip pull device of the disclosure.
Figure 14 is a schematic depiction of the mask component of a strip pull device.
Figures 15A-15C are schematic depictions of the bridge component of a strip pull device.
Figures 16A-16C are schematic depictions of the button component of the bridge.
Figure 17 is a schematic depiction of a profile of a strip pull device.
Figure 18A is a schematic depiction of a strip pull device prior to actuation. This device includes a hoop to surround the strip and graphical instructions. Figure 18B is a schematic depiction of a strip pull device after actuation.
Figures 19A-19B are schematic depictions of both sides of a strip for a strip pull device.
Figures 20A-20B are schematic depictions of an interlock in a strip pull device.
Figure 21 is a schematic depiction of a treatment registration system of the invention.
Figures 22A-22C are schematic depictions of use of a treatment registration system of the invention in treatment registration.
Figure 23 is a schematic depiction of a mask and separate disrupting member in a Mask and Abrade System of the disclosure.
Figure 24 is a schematic depiction of the top surface of a mask for use in a mask and abrade system of the disclosure.
Figures 25A-25B are schematic depictions of actuation of the disrupting member of Figure 23.

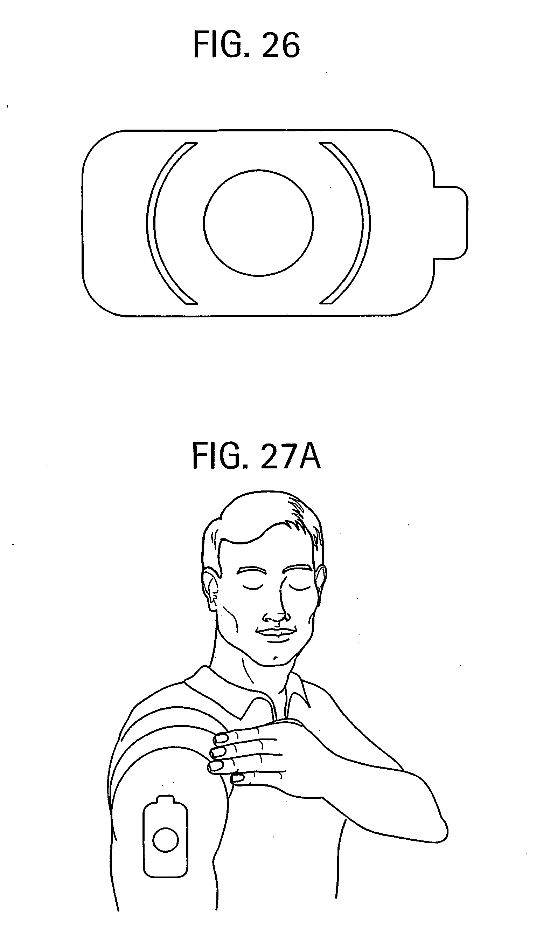

Figures 26 is a schematic depiction of exemplary treatment registration system integrated into the bottom surface of a mask

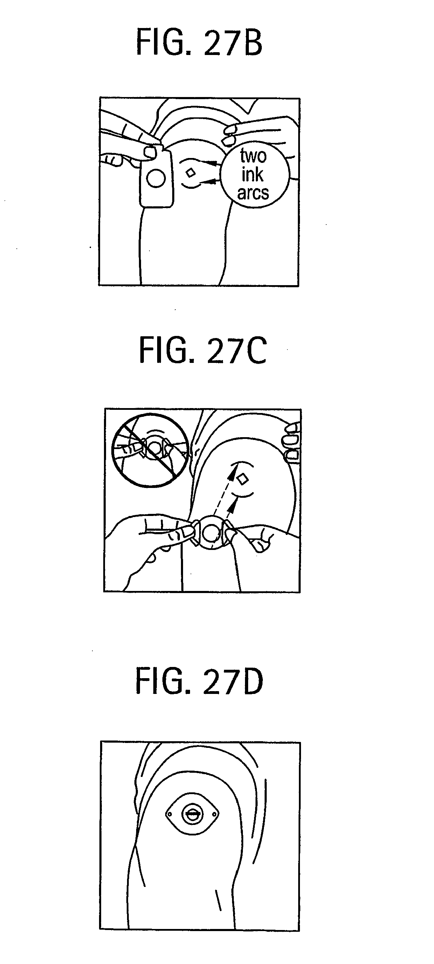

Figures 27A to 27D are schematic depictions of use of a mask and disrupting member with a treatment registration system.
Figures 28A-28B are schematic depictions of an exemplary patch of the disclosure.
Figure 29 is a schematic depiction of features of a patch of the disclosure to aid in use and alignment.
Figures 30A-30B are schematic depictions of application of a patch of the disclosure.
Figure 31 is a schematic depiction of a patch of the disclosure having two tabs and a three component release liner.
Figures 32A-32D are schematic depictions of application of the patch illustrated in Figure 31.
Figure 33 is a schematic depiction of a treatment registration system of the invention.
Figures 34A-34C are schematic depictions of use of a treatment registration system of the disclosure in treatment registration.
Figures 35A-D are photographs and confocal micrographs of a chemically etched metal disrupting member.

In general, devices of the disclosure are capable of disrupting a defined area of skin to deliver a therapeutic agent or a formulation including a therapeutic agent to one or more layers of the skin. A formulation may include one or more therapeutic agents and a pharmaceutically acceptable carrier. The formulation, for example, may be in liquid form, in dry form, or in the form of a gel or emulsion.

The present devices increase the ability to deliver therapeutic agents or therapeutic formulations transcutaneously and/or transdermally. Disruption of the outermost layers of the skin such as the stratum corneum and/or upper epidermis enhances skin penetration or absorption of the therapeutic agent into the skin. Devices of the disclosure may be configured in various ways to improve transcutaneous and/or transdermal delivery of therapeutic agents.

### Devices

The disclosure provides devices that can be used in systems that prepare skin for administration of therapeutic agents, i.e., skin preparation or pretreatment systems (SPS™). These systems employ various methods to disrupt the skin for delivery of therapeutic agents, e.g., vaccine or drug. Exemplary components and SPS™'s are described herein.

**Disrupting Member.** All of the devices include a disrupting member capable of disrupting one or more layers of the skin, e.g., the stratum corneum. Any device known in the art for disruption of the skin, e.g., stratum corneum, maybe employed. These devices may include, for example, abrasives, microelectromechanical (MEMS) components with arrays of short microneedles or microprotrusions, microblades, sandpaper-like components, scrapers, cutters, shavers, punched metal, stamped and formed metal, nylon loops (i.e., Velcro), injection molded plastic, chemically etched metal, thermally sprayed metal, sharpened ceramic edges, and the like. Exemplary materials for disrupting members include metals, plastics, silicon, silicon dioxide, silicon carbide, aluminum oxide, diamond, and ceramics. Other disrupting members include metal microprotrusions, e.g., as described in U.S. Publication No. 2002/0128599. A disrupting member may include abrasives disposed on a surface, e.g., sandpaper, particles embedded in a plastic surface, or a wire brush, or the disrupting member may be fabricated in one piece, e.g., a file (formed, for example, by impact deformation), a rasp, or microneedle array, such as described in U.S. Publication No. 2004/0077994 and U.S. Patent No. 6,595,947. Steel wool and abrasive pads may also be employed. The particle or protrusion size employed in the disrupting member ranges, for example, from about 10 - 500 µm, e.g., about 50 µm-150 µm, or about 200-300 µm or about 80 - 600 P-grit, e.g., about 180-600 P-grit or 180-320 P-grit. Other methods for skin disruption are known in the art.

The disrupting member may also include surgical blades, razor blades, or the like incorporated into a structure to limit the penetration or cutting to the skin, such as described in Example 16. These disrupting members are differentiated from standard razors used for shaving, which are specifically designed to avoid skin abrasion or cutting. Similarly, surgical blades are typically designed to cut through the skin. In the current disclosure, the disruption is limited to the skin.

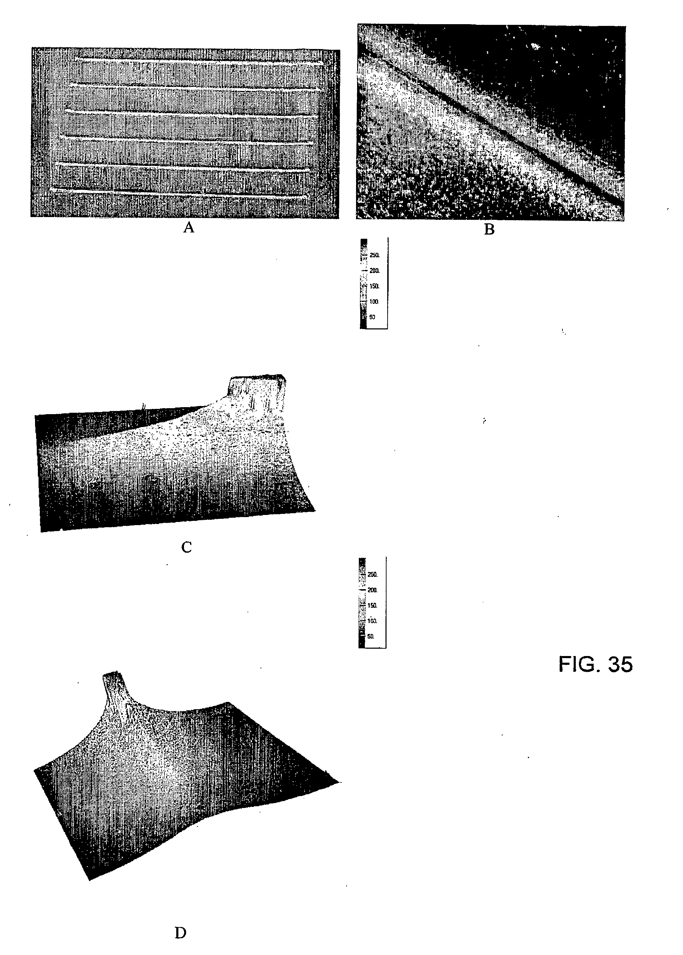

Another disrupting member may be manufactured by chemically etching metal, i.e., SPS™-CE. This disrupting surface as designed [1] provides a single part with multiple protrusions manufactured with precise geometric control, [2] provides a single part that replaces a potential multi-part assembly, [3] provides multiple protrusions that are fabricated with specific 3D geometry, locations, surface profile, and alignment, [4] produces a reproducible skin disruption, [5] produces an effective skin disruption for the delivery of therapeutic agents, [6] produces an effective skin disruption in one gliding motion, [7] controls the depth and directionality of the skin disruption, [8] minimizes patient discomfort and bleeding, [9] is disposable, [10] is single-use, and [11] is fabricated with a configurable manufacturing process that can be specified to create other protrusion designs that match specific skin disruption and medical treatment requirements. Figures 35A-D shows a chemically etched disrupting surface configured for a strip pull device. Figures 35A and 35B are photographs of a chemically etched disrupting member. Figures 35C and 35D are confocal micrographs of a single chemically etched protrusion.

In this example, the disrupting surface is manufactured by a photo-chemical etch process that selectively removes material from a plate or sheet of medical grade stainless steel. The metal is coated with a negative photoresist and exposed to a controlled UV light source. The photoresist is developed, and the unexposed areas are stripped away from the metal. The bare metal is etched with an acid mixture, typically 30-45% Ferric Chloride, leaving the desired design. This process is customizable according to the application; it is possible to create triangles, circles, thin lines and irregular shapes easily in any width, length and complex geometry. The depth of the etching is dependent on the design and the thickness of the material which can range from less than 0.005" to over 0.040" (0.127 mm to over 1.016 mm); it is possible to remove material entirely from the sheet or to etch to certain depths within the material. The flexibility of this process allows the metal to be etched to form blade-like protrusions on a single sheet. For example, some protrusions have been designed to be ¼" - 1"(6.35 - 25.4 mm) in length, 0.002" - 0.008" (0.0508 - 0.2032 mm) in width at the center progressively decreasing to create a 26 degree angle at the ends and have been etched to depths from 0.004" - 0.010" (0.1016 - 0.254 mm) in a 0.015" (0.381 mm) thick material. The material is removed without altering the integrity or properties of the metal. Utilizing this manufacturing process, all geometric features can be precisely reproduced from part to part. The manufacturing process is extremely accurate at forming the disrupting surface to the specified geometry and surface profile. Length, height, width, profile and approach angle can all be controlled. This precise control provides the capability to generate the specific skin disruption required by the medical treatment.

When used with the devices of the disclosure, taller metal protrusions contact the skin, e.g., when protruding through an aperture. As the device is pulled over the skin, the sharp edges of the protrusions slice through the top layers of the skin and create channels in the skin. These channels open up the skin to allow large molecules of a medical treatment, such as a vaccine, to access the underlying cell layers. The skin disruption produced by this device is painless, non-threatening, and controlled.

Compared to other systems which incorporate blades, the advantages of this disrupting surface can be grouped into the following categories: (1) This single-piece part has multiple sharp blade-like protrusions that have precise geometry and locations. (2) This single part eliminates the manufacturing labor and cost associated with a multi-blade, multi-part assembly. (3) The height, width, length, approach angle, and alignment of the multiple protrusions can be precisely controlled and reproduced with this manufacturing process. (4) The protrusions are non-threatening to touch and are easily handled and manipulated. The significantly large flat surface area at the top of each protrusion helps protect users from contact with the sharp leading edge of each protrusion. (5) The surface disrupts the skin painlessly and effectively delivers vaccine without the use of needle injection. A considerable portion of the population has a phobia of needles and others are apprehensive of receiving a traditional vaccination procedure (e.g. intramuscular injection or subcutaneous injection). (6) The device is safe for both the clinician and the patient. Since the skin disruption typically does not result in bleeding, it reduces the risk of accidental contaminations and other risks associated with needles. (7) The device is an element of a simple to use system that is for single-use and may be disposable without any precautions needed. (8) The substrate is adaptable for a variety of aperture designs. (9) The device is fabricated with a configurable manufacturing process that can be specified to create other protrusion designs that match specific skin disruption requirements.

Any disrupting member may include an indicator for the amount of pressure being applied. Typically, an indicator will provide tactile, auditory, or visual feedback, or a combination thereof. Such an indicator may report the actual pressure, or it may indicate that a minimum amount of pressure has been reached. Pressure ranges, for example, from 200 g to 2000 g, e.g., 700 g, applied over a 1-5 in² (6-32 cm²) area. An exemplary indicator of a minimum amount of pressure is a snap dome that depresses when pressure above a set limit is applied (Figure 1). The snap dome may indicate that an appropriate amount of pressure is applied by visual, auditory, or tactile factors. Disrupting members that contain snap domes may be fabricated out of at least two pieces that are capable of moving relative to one another to allow the compression necessary to depress the dome. In another embodiment, the disrupting member may contain elastomeric elements that allow compression of the dome. Another method to indicate applied pressure is an LED indicator light, which is assembled inside two moving parts of the disrupting member. A spring of particular strength is employed to transmit force to the pretreatment site. When the spring is compressed, an electrical circuit to an included battery is completed, and a colored light turns on and is visible to the administrator. Another indicator is a snap spring.

Preferably, the minimum amount of disruption to produce the desired result should be used. Determination of the appropriate amount of disruption for delivery of a selected substance, e.g., drug or antigen, is within the ordinary skill in the art. To achieve a desired amount of disruption, a disrupting member may be passed over an area of skin one or more times, e.g., at least 2, 5, 10, 15, or 20 times. Passes over the skin may be in the same direction, or the direction may be altered during disruption, including perpendicular or across the previous stroke in a different direction. The application of cross-hatched grooves or furrows may improve antigen or drug delivery as shown in Example 10. In other embodiments, the disrupting member may be rotated or vibrated in place to achieve a desired amount of disruption. In another embodiment, the disrupting surface of the member may be sized such that a single pass of the disrupting member yields sufficient disruption, e.g., between 0.5-3 inches in width, for example, 1 inch, (25.4 mm) and 0.5-3 inches (12.7-76.2 mm) in length, for example, 1.5 inches (38.1 mm). The length and width of the disrupting surface can control the extent of the stratum corneum (SC) disruption. The length of the disrupting surface, e.g., a strip, may also be used to control the relative delivery of the antigen. Table 13, described in more detail in Example 14, shows that increasing the length of the disrupting surface from half an inch to one and a half inches increases the amount of antigen delivered into the skin as indicated by an increase in anti-LT IgG titers from about 5,600 to 23,000.

Methods known in the art may be used to determine whether a sufficient amount of disruption has occurred. Such methods include monitoring electrical impedance, electrical capacitance, pH, optical fluorescence or reflectance, e.g., infrared (IR) or visible, thermal diffusivity and conductivity, transepidermal water loss, ultrasonic reflection, confocal microscopy, biopsy, or gaseous efflux of the abraded skin. The monitor may be integrated with the device of the disclosure or may be separate.

A therapeutic agent, e.g., vaccine or drug, may be coated onto a disrupting member and delivered during disruption. Alternatively, the disrupting member may be coupled to a reservoir from which the therapeutic agent, e.g., vaccine or drug, is administered before, during, or after disruption. The therapeutic agent may also be stored in a reservoir separate from the disrupting member. The therapeutic agent may also be formulated to cling to the disrupting member. For example a dry formulation could be applied to the surface that delivers during the disrupting step. If the disrupting member does not include a reservoir for containment and discharge of fluids from the device, a substance-containing liquid or reconstituting liquid may be separately applied to the skin, for example from a separate dispenser, patch, or pump. However, reservoirs may be an integral part of the disrupting member. Typically the reservoir is in fluid communication with the disrupting surface of the member, for example via channels through needles or protrusions, or via channels which exit the reservoir between such needles or protrusions or abrasive particles, or via porous materials. In this embodiment, the substance or reconstituting liquid is contained in the reservoir of the disrupting member and is dispensed to the skin surface, e.g., prior to abrasion or simultaneously with abrasion. The disrupting member may also include means for controlling the rate of delivery of the substance or reconstituting liquid, or for controlling the amount of substance or reconstituting liquid delivered. Therapeutic agents may be stored in a reservoir or adhered to the disrupting member in any suitable manner, e.g., dry form, solution, or suspension.

**Mask and Abrade Device.** In one embodiment, the device includes a mask and a disrupting member, as described herein. One of the many features of the mask and abrade device includes masking the sensation of the disrupting improving the tolerability of the skin disruption. Although this device is referred to as Mask and Abrade, any disrupting member may be employed. The disrupting member may, or may not be, mechanically coupled to the mask. The mask may include an adhesive to adhere to the skin. Alternatively, straps or other mechanical fasteners may be employed to secure the mask to the skin. The mask contains an opening that defines the area of the skin to be disrupted. The opening may expose 1-5 cm², e.g., via a circular shape or square shape with rounded edges. An exemplary opening is a circular opening of ~3 cm². In certain embodiments, the disrupting member is not constrained to move only within the area of the opening. In one embodiment, the disrupting member is used to disrupt the skin in one to five strokes. In another embodiment, the disrupting member is used to disrupt the skin in two strokes or in five strokes.

Preferably, a mask and abrade device prepares the skin to receive a therapeutic agent by disrupting thin layers of skin and provides the following outcomes [1] a reproducible skin disruption [2] effective delivery, [3] a skin disruption with defined surface area [4] controlled application of force to the disrupting member, [5] controlled placement of therapeutic agent, [6] controlled directionality of the disrupting member, [7] minimized patient discomfort, [8] reinforced correct clinician behavior, [9] an element of a needle-free vaccination system and therefore an alternative vaccination method for a less painful treatment [10] disposability, [11] self-adherence of the mask to the skin upon application, [12] marks lefts on skin to guide therapeutic agent vehicle placement (described below), [13] ergonomic, e.g., addresses a wide range of hand geometries and requires less than 5 lbs actuation force to operate, design [14] single-use system, and [15] a configurable system that can be modified to produce different skin disruptions.

A mask of the disclosure may also reduce the sensation of discomfort or pain associated with disrupting the skin. Methods for determining the amount of discomfort are known in the art, e.g., patient questionnaires or visual analog scales. Without being bound by any theory, we believe the mask, by being fastened to the skin, provides sensations preceding skin disruption that reduce any sensation of pain or discomfort from the disruption. The mask also stabilizes the skin, i.e., reduces local stretching or bunching of the skin surface, during disruption allowing for controlled and reproducible disruption of a defined area. A defined and reproducible area aids in placement of therapeutic agents after treatment. The mask also reduces adverse effects of disruption, such as local rash or irritation.

A mask and abrade device may be supplied as a kit with mechanically uncoupled mask and disrupting member. To employ the kit, the mask is attached to the skin, e.g., by an adhesive, and the disrupting member is passed over the opening in the mask at least once. A patch or other reservoir containing a vaccine or drug may then be placed over the disrupted area, and this patch may, or may not, be mechanically coupled to the mask. Whether or not mechanically coupled to the mask, the patch may remain adhered to the skin while the mask is removed, including detaching the mask from the patch, if necessary.

The mask may also be a thin film, for example, paper, metal, or plastic, e.g., polyester tape or polycarbonate tape that has an adhesive portion for attachment to the skin. The disrupting member may be attached to the film so that it can be passed over the opening in the mask. A patch, or other reservoir, containing the vaccine or drug may also be attached to the film so that it can be contacted with the skin after disruption. Alternatively, the disrupting member may be coated with a vaccine or drug, which is delivered during disruption. An exemplary integrated mask and disrupting member is shown in Figures 2A-2E. In this example, the device is in the form of a loop. To operate the device, an adhesive backing is first exposed, and the device is adhered to the skin. The disrupting member is then passed over the opening in the mask at least once. The loop is then opened to expose a patch containing the therapeutic agent, e.g., vaccine, which is then contacted with the disrupted skin. The patch may also contain an adhesive so that it adheres to the skin, and the rest of the device may be removed and discarded.

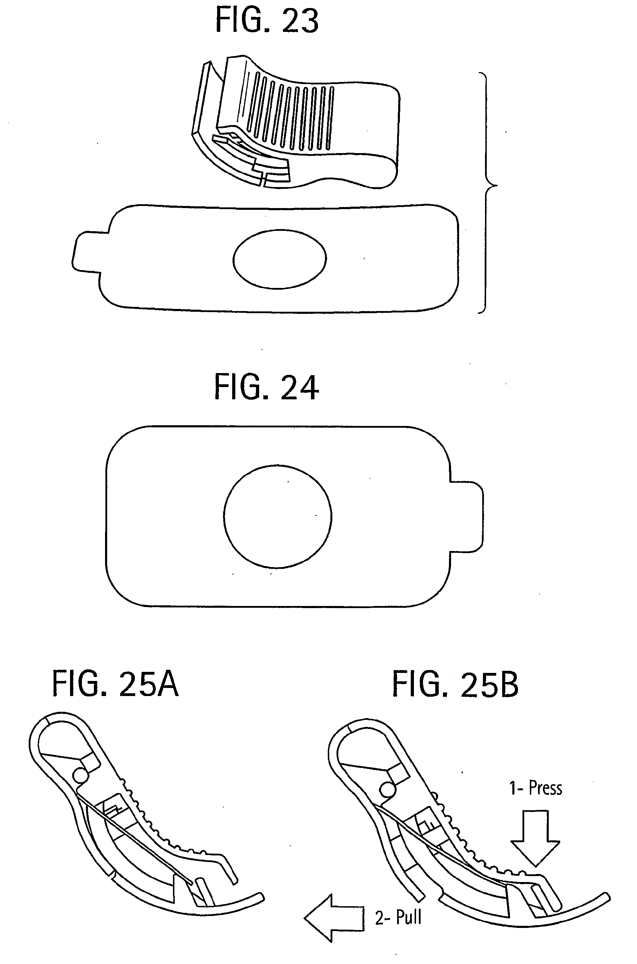

Another exemplary mask and abrade system is shown in Figure 23. The plastic mask is a medical grade plastic with effective resistance to disruption to minimize potential contact of plastic residue (resulting from use of the disrupting member) with disrupted skin. The plastic mask contains a medical grade pressure sensitive adhesive (PSA) that permits temporary adhesion to skin during administration. An opening in the plastic mask prescribes the location and the size of the preparation site. Patient discomfort is minimized through the selection of the thickness of the mask combined with the properties of the chosen material. Example 11 shows that improved tolerability with the use of masking. Figure 24 illustrates the mask, with release liner peel tab.

In this exemplary system, the disrupting member includes four components: the "handle," the "foot," the force indicator such as a metal snap spring, which is actuated by movement between the two previous components, and an abrasive. The snap spring is permanently attached to the "handle" and enclosed within it. The "foot" is assembled via snap fit to the handle, but retains the ability to move through a limited range of motion. The abrasive is bonded to the outer, convex surface of the foot. The shape of the handle is designed to control directionality of disruption. Using any hand grip, the user is guided to stroke only in one direction instead of using the disrupting member in a back and forth manner. The surface of the foot onto which the abrasive is bonded is shaped to maintain constant contact between abrasive and mask or skin. The constant radius shape and the size of the surface ensure that the user can hold the device with a variety of hand grips and at a range of angles with respect to the skin and still maintain appropriate contact and application force. The force indicator assembled between the "foot" and the "handle" changes shape when the correct force is applied pressing the disrupting member onto the mask and skin (See figures 25A and 25B). The force indicator has the characteristic of providing the user with significant tactile and audible feedback as to when the minimum required force has been applied. The force indicator also gives feedback when force is removed. The minimum continuous force applied to the disrupting member is nominally about 1.54 lbs (0.7 kg); however, this force may be modified to match required performance. The force may be in the range of about 0.22 lbs to 4.4 lbs (0.1 kg to 2.0 kg). As discussed, the skilled user may determine an appropriate amount of force based on the desired amount of disruption and the materials used in the disrupting member. The disrupting member can also accommodate a variety of comfortable hand, wrist, and arm positions for the administrator while still providing for a controlled skin disruption.

For any mask and abrade device, the properties of the disrupting member are selected to produce the skin disruption required for the specific application. For example, the snap dome, method of disruption, disrupting materials, and mask are all elements of which the mechanical, geometric, and material properties may be modified, e.g., to produce skin disruptions to meet the specific requirements of other applications.

**Strip Pull Device.** In a similar embodiment, the device includes a mask and a disrupting member that is a strip and including disrupting materials as described herein. The strip is disposed so that it can be pulled across the opening in the mask to disrupt skin in one pass. In some embodiments, a portion of the strip, e.g., the leading end or the edges, does not contain a disrupting material, e.g., abrasive, so that the strip experiences less friction upon first being pulled. The strip may be attached to the device assembly by any suitable means, such as a loop or grooves through which the strip slides. The device assembly includes a mask, with the accompanying properties and advantages as described with the mask and abrade embodiment.

In one embodiment, a strip pull device prepares the skin to receive the delivery of a therapeutic agent by disrupting thin layers of skin and provides the following outcomes[1] reproducible skin disruption, [2] effective delivery for therapeutic agents, [3] a skin disruption that has a prescribed surface area and depth, [4] controlled application of force to the disrupting member, [5] controlled placement of therapeutic agent, [6] controlled directionality of disrupting member, [7] minimized patient discomfort, [8] protection of the disrupting material from contamination until treatment, [9] protection of the clinician, the patient, and their clothing from the disrupting member, except at the preparation site, [10] reinforced correct user behavior, [11] cues, through mechanical design and instructions, offered as to its use, [12] an element of a needle-free vaccination system and therefore an alternative vaccination method for a less painful treatment [13] integration of multiple manual steps and components into one single system, [14] disposability without any disposal requirements (in contrast to injection needles), [15] space-efficiency, [16] self-adherence of the mask to skin upon application, [17] marks left on skin to guide therapeutic agent vehicle placement, [18] ergonomic, e.g., addresses a wide range of hand geometries and requires less than 5 lbs actuation force to operate, design [19] single-use device, and [20] a configurable system that can be modified to produce different skin disruptions.

Exemplary strip pull devices are shown in Figures 3A-3G. Each device includes a snap dome disposed over the opening in the mask. Depressing the snap dome provides a sufficient amount of pressure to disrupt the skin as the strip is pulled over the opening. As with the mask and abrade device, a patch or other reservoir of therapeutic agent to be contacted with the disrupted area may, or may not be attached to the mask. In one embodiment, the strip is un-compressed prior to actuation (Figures 3A-3D). In another embodiment, the strip is folded, rolled, or otherwise compressed in size in the device, and, as the strip is pulled, it is extended (Figures 3D-3G).

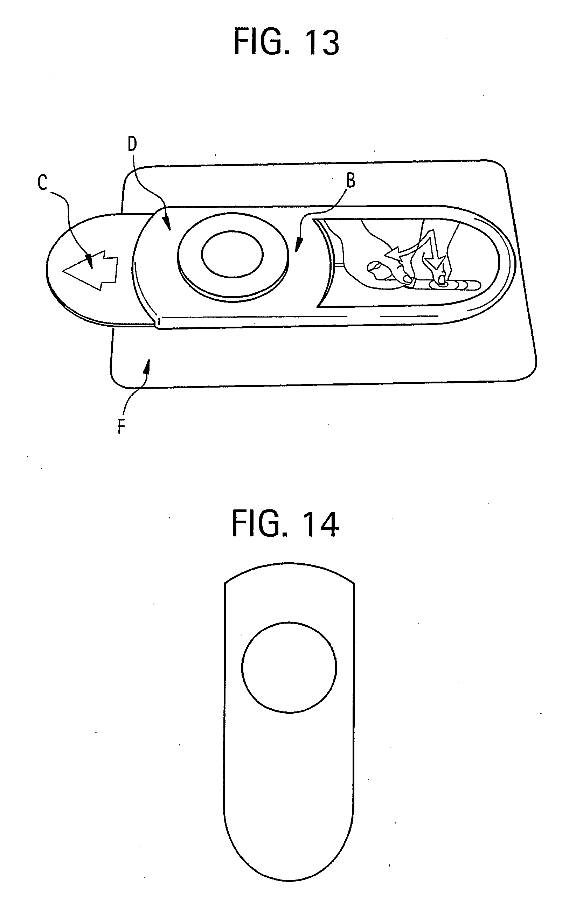

Another strip pull device is shown in Figure 13. This device employs [A] a plastic mask, [B] a bridge assembly, [C] a strip assembly (including skin disrupting material), [D] locking mechanism, [E] a treatment registration system (described below), and [F] oversized release liner.

The plastic mask (Figure 14) is an inert medical grade plastic with effective resistance to disruption to minimize potential contact of plastic residue (resulting from use of the disrupting member) with disrupted skin. The plastic mask is fabricated with a medical grade pressure sensitive adhesive (PSA) that permits temporary adhesion to skin during administration, aids in marking the skin to denote the location of disruptions, and prevents slippage. An opening in the plastic mask defines the location and the size of the preparation site.

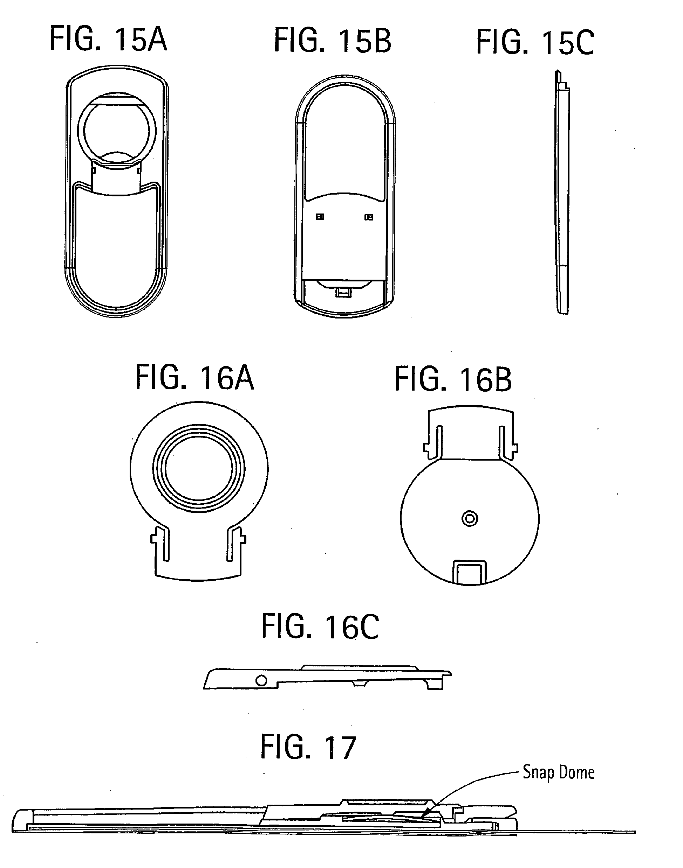

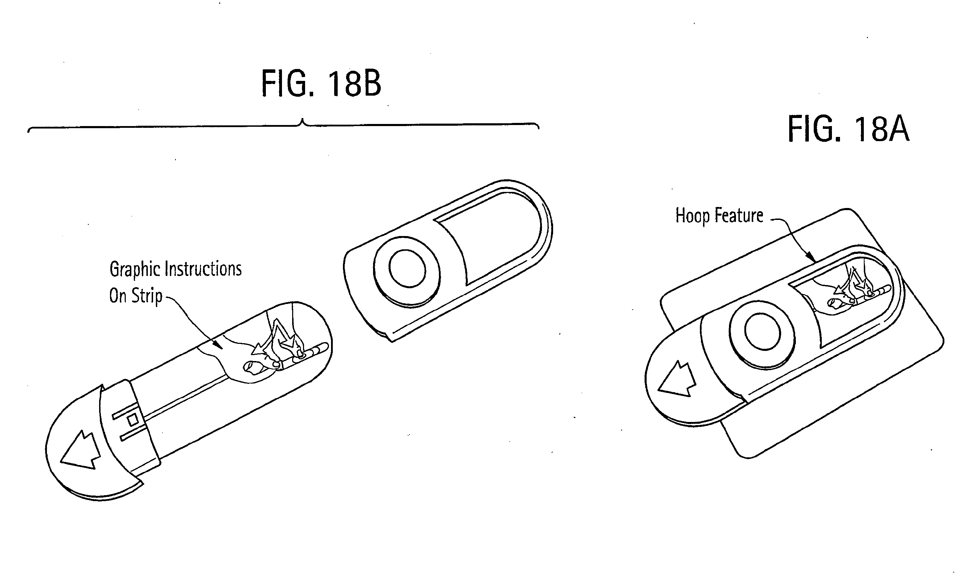

The bridge assembly (Figures 15A-15C) includes three components: the bridge, the button, and the metal snap dome, which is positioned between the two other components. Figure 17 illustrates a profile of the bridge. The bridge assembly is bonded onto the plastic mask and provides a platform to define the directionality of the abrasive during application. The design of the bridge is such that a force indicator, such as a snap dome, can provide an indication of the application force. The snap dome provides tactile and audible feedback for the user to know when the minimum required force has been applied and also when the force has been removed. The snap dome force specification, i.e., force required to depress the snap dome, represents the force needed for a consistent and reproducible skin disruption and surface area. The bridge also contains a hoop feature that constrains the strip to ensure that the abrasive does not come in contact with outside elements such as the patient's clothing. This hoop geometry of the bridge when combined with the mask, serves to guide the translation of the abrasive strip. The hoop feature may also provide viewing access to graphical instructions located on the strip. Figures 18A-18B show how the hoop feature of the bridge allows additional graphical instructions.

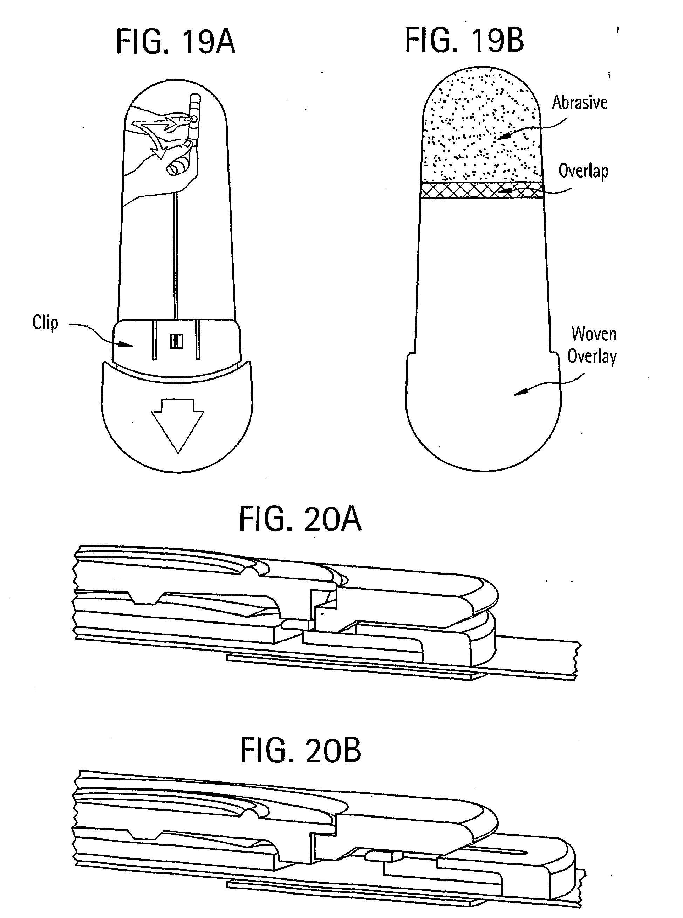

The strip assembly slides between the bridge assembly and the mask and includes four components: the clip, the strip backing, the abrasive, and the woven overlay. The strip backing is a medical grade plastic material. Two materials are bonded to one side of the strip backing: the abrasive and a woven overlay. The length and the abrasive properties are selected to provide the abrasion characteristics required for proper skin treatment. The remaining surface of the strip is covered with a medical grade woven material that a) reduces patient discomfort when the strip is pulled, b) facilitates pulling the strip at a controlled speed, and c) slightly overlaps the abrasive to prevent it from peeling when the strip assembly is pulled through the bridge/mask assembly. The clinician pulls the strip while maintaining pressure on the snap dome. The applied force is translated to the moving abrasive which disrupts the skin exposed through the mask opening. Figures 19A-19B show the strip assembly.

In this device, the user maintains pressure on the snap dome while simultaneously pulling the strip. The clip bonded to the strip prevents motion of the strip unless the proper amount of force is being applied. When the user applies a force to the button of the bridge assembly, the snap dome will compress, and the button will be able to move. The button is illustrated in Figures 16A-16C. The button motion interacts with the clip of the strip assembly in the following ways: a) When no force is applied to the button and snap dome, the strip is mechanically captured inside the bridge assembly and b) When the prescribed force is applied to the button and snap dome, the button depresses and releases the strip to be pulled out by the user. This interlocking mechanism prevents the user from initiating the strip actuation pulling the strip until they have correctly applied the prescribed force to the button. When the user applies an initial load that is less than specified, the interlock mechanism prevents the user from pulling the abrasive strip over the skin. Figures 20A-20B illustrate the interlock mechanism.

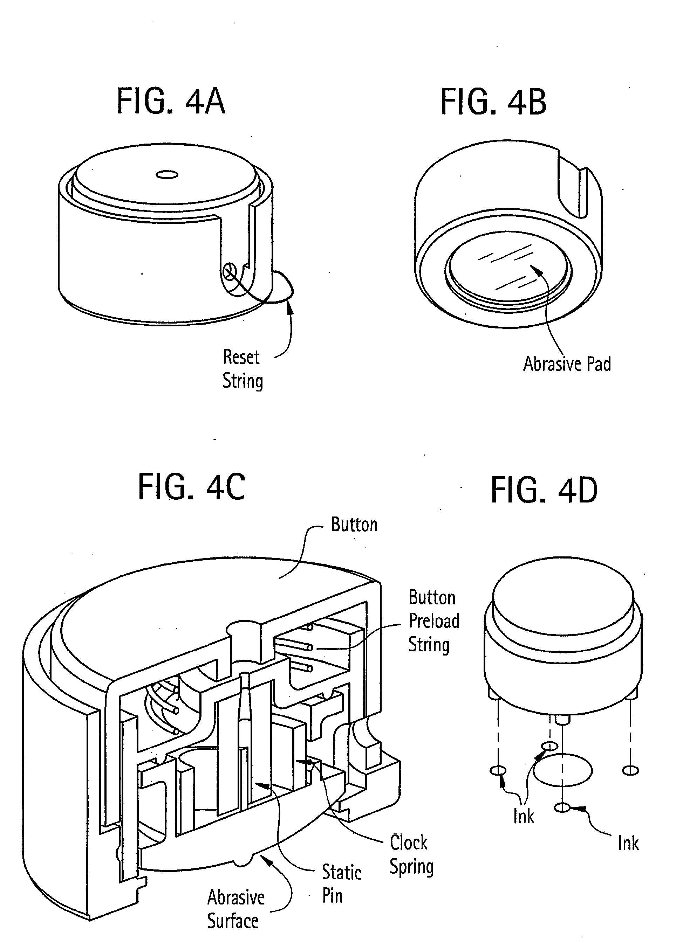

**Site and Spin.** Another device includes a disrupting member, as described herein, that is rotationally actuated to disrupt the skin. The area disrupted may be determined by the size of the disrupting surface or by a mask surrounding the disrupting surface. Using a mask around the edges of the disrupting surface reduces potential cutting by the edge of the surface. The rotational motion may be achieved by standard means, including motors, springs, e.g., clock springs, and pull strings. The device may be designed to be activated only once, or it may be used indefinitely. The device may include one or more switches to prevent actuation prior to use. Switches may be disposed on the surface that contacts the skin to ensure proper contact between the device and the skin. The device may also include a dye or other indicator that marks the skin to indicate the region that has been disrupted. Such markings are useful when the vaccine or drug is applied after removal of the device from the skin. Indicators include inks and adhesive markings, e.g., stickers. An exemplary site and spin device is shown in Figure 4. Although a site and spin device may not employ a mask as employed in the mask and abrade and strip pull devices, placing the device against the skin for actuation helps stabilize the skin, reduce patient discomfort, and provide similar advantages to the masks in the other systems.

**Delivery System for Therapeutic Agents.** The invention also features a delivery system for therapeutic agents for use in conjunction with the devices described herein. Preferably, the delivery system [1] facilitates accurate placement, [2] is safe for skin contact, [3] maintains appropriate adhesion to the skin for at least a duration of 6 hours, [4] reinforces correct user behavior, [5] minimizes risks of peel-off during wear, [6] suggests the orientation of the delivery vehicle on the skin, [7] protects the administrator from the therapeutic agent, [8] protects the therapeutic agent from coming in contact with fingers during application, [9] protects the therapeutic agent from potential cross-contamination with adhesive, [10] minimizes patient discomfort during wear, [11] minimizes patient discomfort during peel-off, [12] is disposable without additional precautions, [13] is single-use, [14] offers an effective alternative to needle injection, and [15] provides effective delivery for the therapeutic agent.

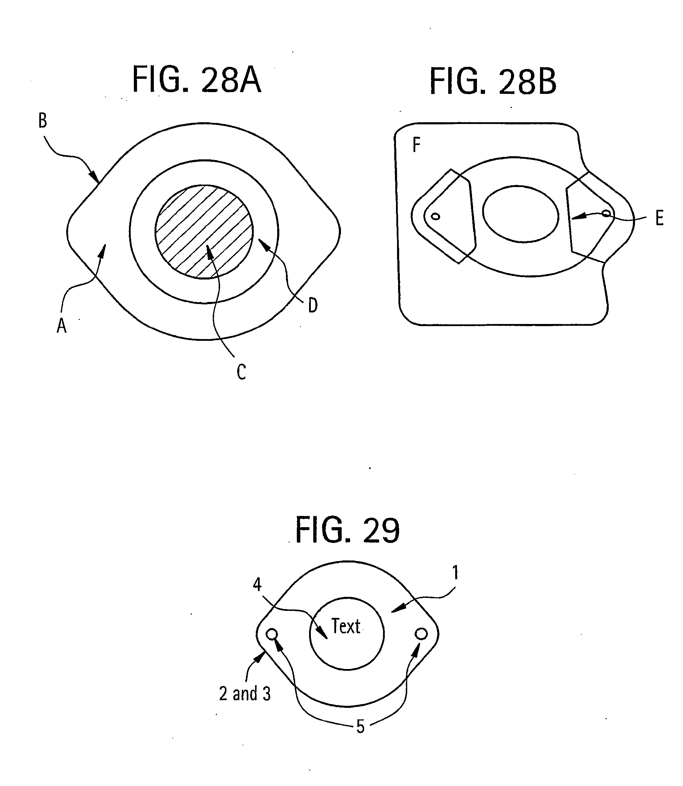

The delivery system typically includes [A] a medical grade adhesive, e.g., acrylic-based, pressure sensitive adhesive, [B] a medical grade overlay, e.g., made of polyurethane, [C] a medical non-woven material, e.g., rayon, containing the therapeutic agent, [D] a medical grade occlusive layer, e.g., coated polyester, [E] graphics on the overlay backing, and [F] release liner, e.g., made from silicone-coated polyester (Figures 28A-28B). The adhesive on the patch may be an aqueous-based adhesive or an acrylic adhesive, or other adhesives known in the art. The acrylic adhesive may be coated onto polyurethane or a similar flexible film. The non-woven material may be, for example a rayon or polyester blend. The occlusive layer may be a polyester with thickness between 0.001" and 0.005" (0.0254 mm and 0.127 mm), e.g., 0.002" (0.0508 mm), and may be coated with a layer that allows the therapeutic agent carrier to be adhered to or heat-sealed to the PET or similar plastic. Additional patches are described herein and are known in the art.

These components are illustrated in an exemplary device in Figures 28A-28B. The components that are adhered to the patient are referred to as a patch. The advantages of this delivery system include (1) effective delivery of therapeutic agent, e.g., vaccine, (2) an alternative to traditional needle injections, and (3) a design that promotes accurate placement. The geometry of the delivery vehicle and the graphics may also promote and reinforce correct user behavior.

As is shown in Figure 28A, one side of the occlusive layer [D] adheres to adhesive [A], and the therapeutic agent is attached on the same side of [D]. Preferably, the diameter of the occlusive layer is greater than the diameter of the therapeutic agent, leaving a buffer zone (no adhesive) between the adhesive and the therapeutic agent to reduce or prevent cross-contamination. Additionally, the occlusive layer may serve as a moisture resistant barrier; this property causes the perspiration and moisture generated from the skin disruption to rehydrate the therapeutic agent, e.g., vaccine formulation to promote better immunization success rates. The strength of adhesive [A] is chosen to meet the requirements for duration of wear and skin properties of the target recipients.

Figure 29 shows the graphical features on the backing of the overlay. The system design offers features that improve user interaction, such as (1) clear overlay, (2) colored outline, (3) geometry, (4) text, and (5) dots.

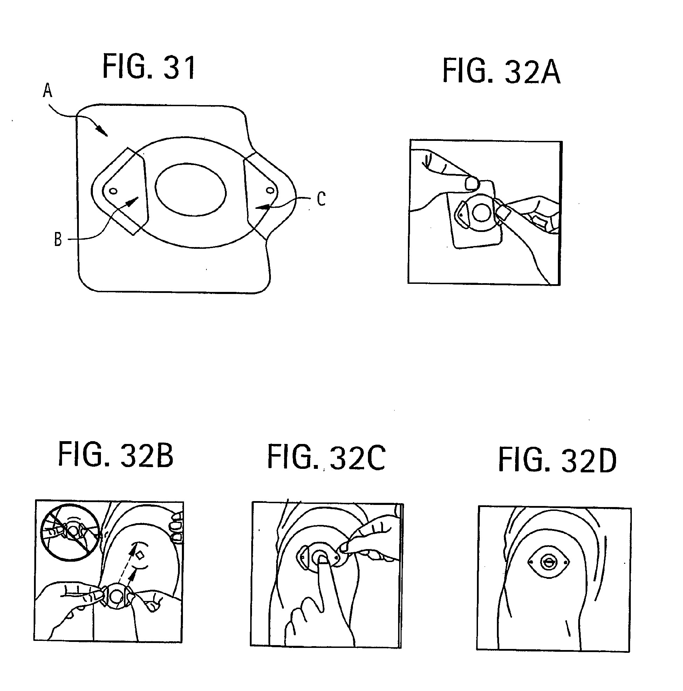

The release liner includes three components: (a) main backing, (b) left tab, (c) right tab. Figure 31 shows these components and how they interact with the patch. The shape of the main backing usually differs from the shape of the patch to assure that the user can easily distinguish between the patch and the release liner. In addition, the delivery vehicle may not be centered on the main backing, to emphasize one tab and encourage the user to use it as the preliminary contact point. For example, the user holds the right tab and peels off the delivery vehicle from the main backing. As a result, the user is left holding the delivery vehicle by one of two release liner tabs. The user holds both tabs with both hands and places the delivery vehicle on the skin disruption site. This method of handling allows the user to apply tension to the vehicle and prevent the vehicle from folding and wrinkling. This is especially beneficial for very flexible materials such as the materials of this patch

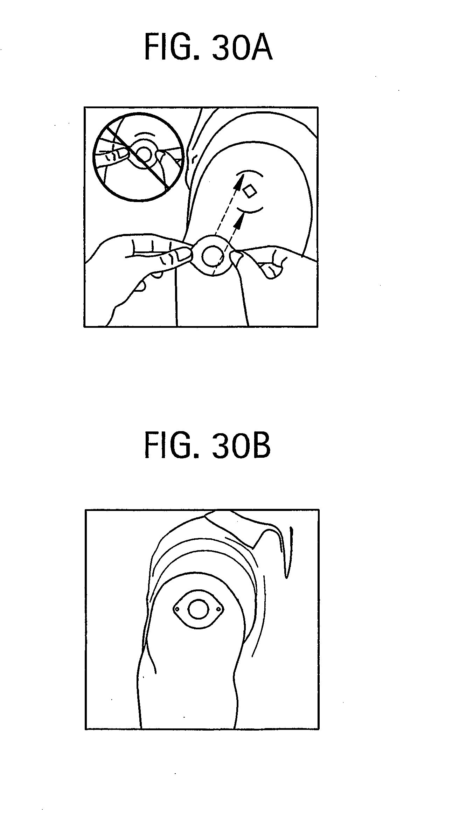

Once the delivery vehicle is placed on the skin, the user pulls both tabs away and smoothes the delivery vehicle on the skin. Figures 30A-30B show the delivery vehicle placement procedure.

The advantages of the release liner design over prior approaches can be categorized into the following benefits: (1) The release liner design provides intuitive interaction. The oversized main backing combined with the two smaller tabs reinforce the order of use. The main backing is removed first, the user holds the delivery vehicle with both hands (e.g., to promote horizontal placement), places it on the skin, and the tabs are then removed. (2) The integrity of the patch is preserved throughout the procedure. The two small tabs do not leave the delivery vehicle until it is placed on the skin, and the tabs are removed. The adhesive of the delivery vehicle is protected at all times since the user does come in direct contact with it, reducing or eliminating damage to the vehicle itself and\or the adhesive performance throughout the wear period.

Figures 32A-32D illustrate how the patch may be placed on the skin. Registration marks on the skin guide the user during patch placement. The user holds the patch by the dots and places it horizontally on the skin. The geometry of the patch, e.g., elliptical-shaped, suggests that the user should hold the patch by the two tapered ends of the patch. This geometry minimizes contact between the fingers and the therapeutic agent. This geometry also promotes horizontal patch placement because of the positioning of the fingers relative to the rest of the patch. Two dots are printed on the further ends of the patch to emphasize the positioning of the fingers during placement, but also to reinforce utilizing both hands during placement, which is more accurate. Additionally, any text on the patch which can include company brand, therapeutic agent, or instructions reinforces the orientation of the patch.

The accuracy of the patch placement may be important, e.g., to the vaccination process. A colored outline on the patch defines the exterior edge and facilitates the alignment of the patch with registration marks on skin. A clear overlay allows the user to see through the patch during placement, aiding the user in aligning the drug delivery vehicle with the pretreatment site. Registration indicators facilitate aligning the patch to skin disruption site.

**Treatment Registration System.** The invention also features a treatment registration system to aid in aligning administration of the therapeutic agent with the site of pretreatment. This system may be employed with any skin disruption method or system, such as those described herein. The system employs two elements: a marking system and an indicator.

The marking system generates shapes in specified locations that are typically in close proximity to, but external to, the target treatment area. The system provides a reference for aligning a single treatment with a site of pretreatment and also for aligning multiple treatments in the same area. In one embodiment, the shapes may define the full or partial outline of a drug delivery vehicle. Preferred indicators are non-toxic and non-irritating, resistant to typical shipping, storage and application conditions, and formulated to minimize flow throughout shelf-life to maintain shape integrity until application to the skin. Exemplary indicators include inks, paints, stains, and adhesive markings, e.g., stickers.

The purpose of the treatment registration system is to mark the skin to [1] provide a method for second and subsequent treatments to precisely be performed at the same location as the first treatment, [2] not interfere or interact with the multi-treatments, [3] minimize the amount of surface area required for treatment, thereby reducing the drug material, cost, and potential patient discomfort, [4] provide guidance during the placement of a vehicle for drug delivery, [5] transfer a defined shape onto the skin, [6] be safe for skin contact, [7] not require additional activation elements, [8] maintain acceptable performance throughout a shelf life of three years, [9] be distinctly visible once applied onto the skin, [10] be non-permanent and easily washable with water (and soap if needed), and [11] be designed to prevent smearing the indicator that could effect the user's ability to accurately place and register the second or subsequent treatment device or vehicle and/or impact the drug delivery vehicle's adhesion to the skin.

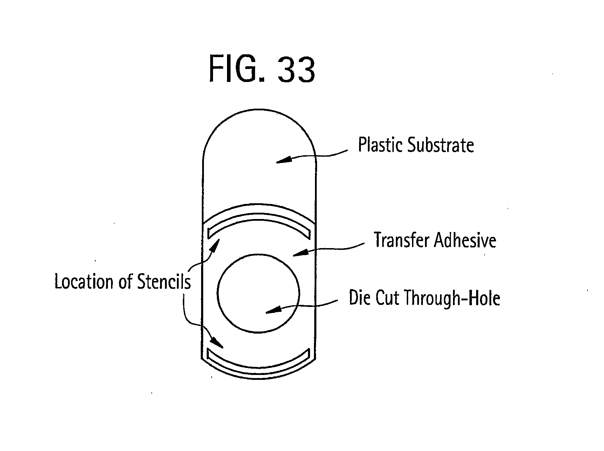

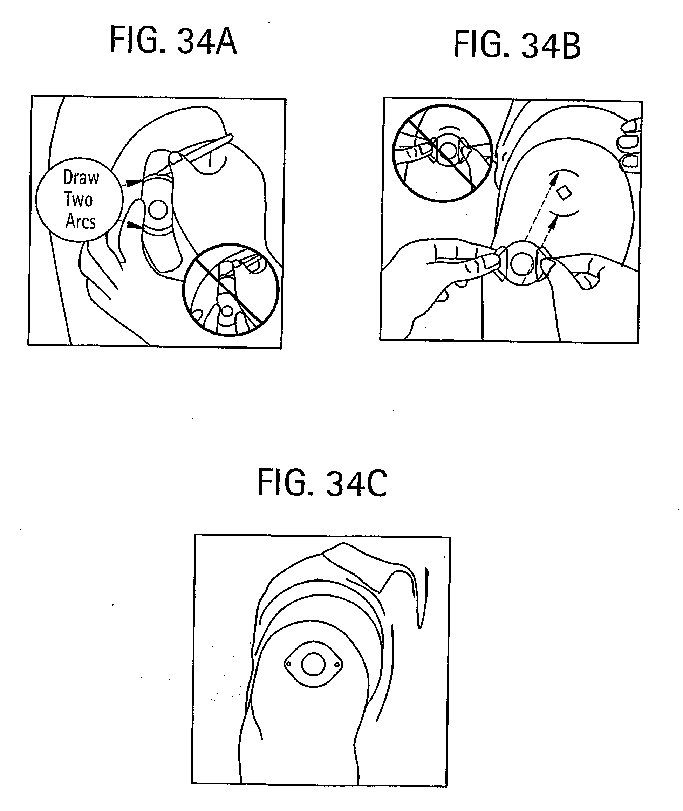

In exemplary registration system, an ink marker is employed with a strip pull system. The strip pull mask has stencils cut through it to define the shape of the ink marks that are to be left on the skin. The marker is used to mark the skin through the stencils. Figure 33 shows the location of the stencils on the mask, and Figures 34A-34C demonstrate how this registration is used for patch placement.

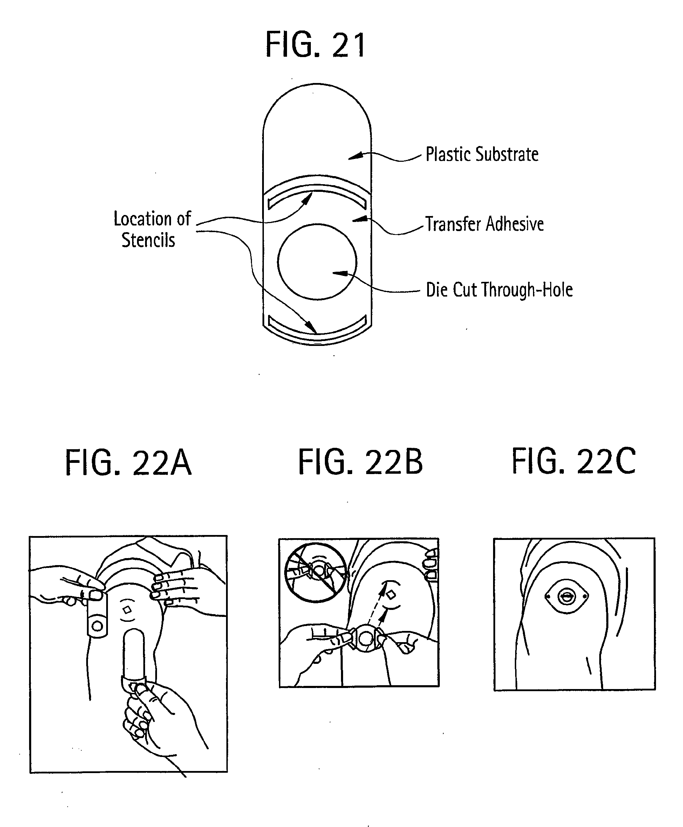

In another system, thin layers of ink are placed between two films, e.g., a plastic substrate and a release liner. The ink is directly printed on the film (e.g., by pad printing, screen printing, or flexographic printing) and is surrounded by adhesive. Figures 21 and 22A-22C illustrate this system. Figure 21 shows the location of the patch registration (ink marks) on the plastic mask, and Figures 22A-22C demonstrate how these marks are used for patch placement.

Markings may also be made around the outside edges of a device of the disclosure to denote the location of skin disruption.

### Methods of Using the Devices

The devices of the present disclosure are applied to the skin of a subject to increase the permeability of the skin and the rate of transcutaneous and/or transdermal delivery of a therapeutic agent or formulation, preferably without pain or irritation to the subject. The devices of the present disclosure may disrupt the outermost layer of the skin without pain to the subject and with minimum irritation to the skin. The devices disrupt the SC and/or the outermost epidermal layer of the skin, disrupting the outer layers of the skin. The devices of the present disclosure may include a disrupting member that does not penetrate or perforate the skin. The devices of the present disclosure improve or enhance transcutaneous and/or transdermal delivery of therapeutic agents and formulations containing therapeutic agents.

Specifically, the devices of the disclosure may be employed to remove at least some of the layers of skin covering the dermis, e.g., for administration of one or more therapeutic agents. For example, the SC may be partially or fully removed, with or without subsequent removal of all or part of the epidermis. Moreover, disruption of the skin by abrasion or by friction with a device of the disclosure may result in an increase in the transepidermal water loss (TEWL) by a factor of at least 2, 5, 8, 10, 15, or 20, compared to undisrupted skin. The disruption may be visualized by confocal microscopy.

In one embodiment, the devices of the present disclosure can be used to apply a substance simultaneously with the disruption of the skin of a subject. A device of the present disclosure may include a container for holding the substance to be applied to the skin. In another embodiment, the device of the present disclosure only disrupts the skin and the formulation is applied to the skin separately. The formulation may be applied to the skin prior to or subsequent to disruption of the skin by the device.

The devices of the present disclosure can be used concurrently with, prior to, or subsequent to treatment of the skin of a subject by other methods for enhancing penetration and/or disruption of the skin including, for example, chemical, physical, and mechanical means, hydration means, or a combination thereof. The skin may be treated with any applying means that enhances penetration and/or disruption of the skin.

The devices of the present disclosure may be applied at various sites on the skin of a subject to improve penetration of a therapeutic agent. The devices may be applied to multiple anatomical sites, for example, the deltoid, the thigh, or the lower back.

The devices of the present disclosure are useful for transcutaneous and/or transdermal delivery of various molecules or compositions, for example, one or more therapeutic agents, and formulations or compositions including one or more therapeutic agents. The devices of the present disclosure may also be used after applying or prior to applying the therapeutic agent or formulation to the skin of a subject.

In one embodiment, the devices of the present disclosure may be used for transdermal delivery of drugs including small molecules. In another embodiment, the devices of the present disclosure may be used with transcutaneous immunization comprising inducing an antigen-specific immune response in a subject.

### Transcutaneous Immunization

The devices of the present disclosure are useful in transcutaneous immunization (TCI). They improve or enhance delivery of therapeutic agents such as vaccines into the skin. The devices of the present disclosure can be used to disrupt the skin to enhance skin penetration allowing for effective TCI. U.S. Patent 5,980,898 discloses TCI.

Generally, TCI induces an immune response (e.g., humoral and/or cellular effector specific for an antigen) in an animal or human. The delivery system provides simple, epicutaneous application of a formulation comprising one or more antigens and/or adjuvants to the skin of a subject. A specific immune response is thereby elicited against the antigen.

For traditional vaccines, their formulations were injected through the skin with needles. Injection of vaccines using needles carries certain drawbacks including the need for sterile needles and syringes, trained medical personnel to administer the vaccine, discomfort from the injection, needle-born diseases, and potential complications brought about by puncturing the skin with the potentially reusable needles. Immunization through the skin without the use of hypodermic needles represents an advance for vaccine delivery by avoiding the hypodermic needles.

Since transcutaneous immunization does not require injection with a hypodermic needle (i.e., penetration to or through the dermis) and the complications and difficulties thereof, the requirements of trained personnel, sterile technique, and sterile equipment are reduced. Furthermore, the barriers to immunization at multiple sites or to multiple immunizations are diminished. The present disclosure also provides for a single application of a therapeutic agent.

Transcutaneous immunization may be achieved using epicutaneous application of a simple formulation of antigen and/or adjuvant in solid form, liquid, or gel, optionally covered by an occlusive dressing, or by using other patch technologies. The immunization could be given by untrained personnel, and is amenable to self-application. Large-scale field immunization could occur given the easy accessibility to immunization. Additionally, a simple immunization procedure would improve access to immunization by pediatric patients and the elderly, and populations in Third World countries.

TCI provides a novel means for immunization through intact skin without perforating the skin. Transcutaneous immunization according to the disclosure provides a method whereby antigens and adjuvant can be delivered to the immune system, especially specialized antigen presentation cells underlying the skin (e.g., Langerhans cells) without requiring a liquid-based delivery system.

Moreover, transcutaneous immunization may be superior to immunization using hypodermic needles as more immune cells would be targeted by the use of several locations targeting large surface areas of skin. A therapeutically-effective amount of antigen sufficient to induce an immune response may be delivered transcutaneously either at a single cutaneous location, or over an area of skin covering multiple draining lymph node fields (e.g., cervical, axillary, inguinal, epitrochelear, popliteal, those of the abdomen and thorax). Such locations close to numerous different lymphatic nodes at locations all over the body will provide a more widespread stimulus to the immune system than when a small amount of antigen is injected at a single location by intradermal subcutaneous or intramuscular injection.

TCI induces an antigen-specific immune response. Antigen passing through or into the skin may encounter antigen presenting cells which process the antigen in a way that induces an immune response. Multiple immunization sites may recruit a greater number of antigen presenting cells and the larger population of antigen presenting cells that were recruited would result in greater induction of the immune response. The skin may deliver antigen to phagocytic cells of the skin such as, for example, dermal dendritic cells, macrophages, and other skin antigen presenting cells; antigen may also be delivered to phagocytic cells of the liver, spleen, and bone marrow that are known to serve as the antigen presenting cells through the blood stream or lymphatic system.

Efficient immunization can be achieved with the disclosure because transcutaneous delivery of antigen may target the Langerhans cell. These cells are found in abundance in the skin and are efficient antigen presenting cells leading to T-cell memory and potent immune responses. Because of the presence of large numbers of Langerhans cells in the skin, the efficiency of transcutaneous delivery may be related to the surface area exposed to antigen and adjuvant. In fact, the reason that transcutaneous immunization is so efficient may be that it targets a larger number of these efficient antigen presenting cells than intramuscular immunization.

Langerhans cells, dendritic cells, and macrophages may be specifically targeted using α2-macroglobulin bound antigen or other agents known to target antigen presenting cells (APCs) conjugated to or recombinantly produced as a protein fusion with adjuvant, antigen or both. Langerhans cells, dendritic cells, and macrophages may be specifically targeted using Fc receptor or the high affinity receptor for IgE (Bieber, 1997) conjugated to or recombinantly produced as a protein fusion with adjuvant; also, adjuvant may be conjugated to or recombinantly produced as a protein fusion with protein A or protein G to target surface immunoglobulin of B cells. The result would be widespread distribution of antigen to antigen presenting cells to a degree that is rarely, if ever achieved, by current immunization practices.

Genetic immunization has been described in U.S. Pat. Nos. 5,589,466, 5,593,972, and 5,703,055. The nucleic acid(s) contained in the formulation may encode the antigen, the adjuvant, or both. The nucleic acid may or may not be capable of replication; it may be non-integrating and non-infectious. For example, the nucleic acid may encode a fusion polypeptide comprising antigen and a ubiquitin domain to direct the immune response to a class I restricted response. The nucleic acid may further comprise a regulatory region operably linked to the sequence encoding the antigen or adjuvant. The nucleic acid may be added with an adjuvant. The nucleic acid may be complexed with an agent that promotes transfection such as cationic lipid, calcium phosphate, DEAE-dextran, polybrene-DMSO, or a combination thereof; also, immune cells can be targeted by conjugation of DNA to Fc receptor or protein A/G, or attaching DNA to an agent linking it to α2-macroglobulin or protein A/G or similar APC targeting material. The nucleic acid may comprise regions derived from viral genomes.

An immune response may comprise humoral (i.e., antigen-specific antibody) and/or cellular (i.e., antigen-specific lymphocytes such as B cells, CD4+T cells, CD8+T cells, CTL, Th1 cells, Th2 cells, and/or T_{DTH} cells) effector arms. Moreover, the immune response may comprise NK cells that mediate antibody-dependent cell-mediated cytotoxicity (ADCC).

The immune response induced by the formulation of the disclosure may include the elicitation of antigen-specific antibodies and/or cytotoxic lymphocytes (CTL, reviewed in Alving and Wassef, 1994). Antibody can be detected by immunoassay techniques, and the detection of various isotypes (e.g., IgM, IgD, IgA1, IgA2, secretory IgA, IgE, IgG1, IgG2, IgG3, or IgG4) may be expected. An immune response can also be detected by a neutralizing assay. Antibodies are protective proteins produced by B lymphocytes. They are highly specific, generally targeting one epitope of an antigen. Often, antibodies play a role in protection against disease by specifically reacting with antigens derived from the pathogens causing the disease. Immunization may induce antibodies specific for the immunizing antigen.

CTLs are particular protective immune cells produced to protect against infection by a pathogen. They are also highly specific. Immunization may induce CTLs specific for the antigen, such as a synthetic oligopeptide based on a malaria protein, in association with self-major histocompatibility antigen. CTLs induced by immunization with the transcutaneous delivery system may kill pathogen infected cells. Immunization may also produce a memory response as indicated by boosting responses in antibodies and CTLs, lymphocyte proliferation by culture of lymphocytes stimulated with the antigen, and delayed type hypersensitivity responses to intradermal skin challenge of the antigen alone.

In a viral neutralization assay, serial dilutions of sera are added to host cells which are then observed for infection after challenge with infectious virus. Alternatively, serial dilutions of sera may be incubated with infectious titers of virus prior to inoculation of an animal, and the inoculated animals are then observed for signs of infection.

The transcutaneous immunization system of the disclosure may be evaluated using challenge models in either animals or humans, which evaluate the ability of immunization with the antigen to protect the subject from disease. Such protection would demonstrate an antigen-specific immune response. In lieu of challenge, as an example, achieving anti-diphtheria antibody titers of 5 IU/ml or greater is generally assumed to indicate optimum protection and serves as a surrogate marker for protection (Plotkin and Mortimer, 1994).

Furthermore, the Plasmodium falciparum challenge model may be used as to induce an antigen-specific immune response in humans. Human volunteers may be immunized using the transcutaneous immunization system containing oligopeptides or proteins (polypeptides) derived from the malaria parasite, and then exposed to malaria experimentally or in the natural setting. The Plasmodium yoelii mouse malaria challenge model may be used to evaluate protection in the mouse against malaria (Wang et al., 1995).

Cholera toxin-specific or LT specific IgG or IgA antibody may provide protection against cholera toxin challenge (Pierce, 1978; Pierce and Reynolds, 1974) and LT specific IgG or IgA is known to protect against ETEC related diarrheal disease.

The devices of the present disclosure and the TCI system may also be used to treat various diseases including cancer, allergies and autoimmune diseases. For example, vaccination has also been used as a treatment for cancer, allergies, and autoimmune disease. Vaccination with a tumor antigen (e.g., prostate specific antigen) may induce an immune response in the form of antibodies, CTLs and lymphocyte proliferation which allows the body's immune system to recognize and kill tumor cells. Tumor antigens useful for vaccination have been described for melanoma (U.S. Pat. Nos. 5,102,663, 5,141,742, and 5,262,177), prostate carcinoma (U.S. Pat. No. 5,538,866), and lymphoma (U.S. Pat. Nos. 4,816,249,5,068,177, and 5,227,159). Vaccination with T-cell receptor oligopeptide may induce an immune response that halts progression of autoimmune disease (U.S. Pat. Nos. 5,612,035 and 5,614,192; Antel et al., 1996; Vandenbark et al., 1996). U.S. Pat. No. 5,552,300 also describes antigens suitable for treating autoimmune disease. An embodiment of the formulation for genetic immunization is polynucleotide (e.g., plasmid) attached to a solid substrate (e.g., a gold particle or other colloidal metal). If such a formulation is ingested by an antigen presenting cell, presentation of the encoded antigen may be facilitated or even enhanced.

Moreover, the devices of the present disclosure and the TCI system may be used to treat diseases caused by enterotoxigenic E. Coli (ETEC). Escherichia coli is a bacterium that normally lives in the intestines of humans and other animals. Most types of E. coli are harmless, but some can cause disease. Disease-causing E. coli are grouped according to the different ways by which they cause illness. Enterotoxigenic Escherichia coli, or ETEC, is the name given to a group of E. coli that produce special toxins which stimulate the lining of the intestines causing them to secrete excessive fluid, thus producing diarrhea. Another name for this group of E. coli is enteropathogenic E. coli (EPEC).

The effect of Escherichia coli infection of mammals is dependent on the particular strain of organism. Since the initial association with diarrheal illness, five categories of diarrheagenic E. coli have been identified: enterotoxigenic (ETEC), enteropathogenic (EPEC), enterohemorrhagic (EHEC), enteroaggregative (EAggEC), and enteroinvasive (EIEC). They are grouped according to characteristic virulence properties, such as elaboration of toxins and colonization factors and/or by specific types of interactions with intestinal epithelial cells. ETEC are the most common of the diarrheagenic E. coli and pose the greatest risk to travelers. Strains which have been cultured from humans include B7A (CS6, LT, STa), H10407 (CFA/I, LT, STa) and E24377A (CS3, CS1, LT, STa). They may be used singly or in combination as whole-cell sources of antigen providing a variety of different toxins and colonization factors. Their toxins may also be used individually or in combination in a mixture, conjugated together or used together by recombinant means.

Moreover, the TCI system may be used to deliver separate formulations, such as for transcutaneous immunostimulation. For example, the first and second formulations may be delivered by different routes. One formulation may be delivered parenterally, such as but not limited to, subcutaneously, intradermally, intravenously, intramuscularly, intraperitoneally, and intramedullary, or by another route, for example, orally, buccally, rectally, or vaginally, while the other formulation may be delivered by transcutaneous immunization. The devices of the present disclosure may be used to treat the skin for transcutaneous delivery of the formulation. As an example, the first formulation may include at least one antigen and the second formulation may include at least one adjuvant. In this example, the present disclosure provides (i) administering parenterally a first formulation comprising an antigen; (ii) treating an area of the skin with a device of the present disclosure; and (iii)applying a second formulation including an adjuvant to the treated area of the skin to induce an immune response. The second formulation may include or consist essentially of an adjuvant. In an alternate example, the formulation including the adjuvant may be applied epicutaneously to the skin of a subject by an immunostimulant patch, and the formulation including the antigen may be administered parenterally to the subject. In TCI, the devices of the present disclosure may be used to treat the skin prior to, concurrently with, or after the application of the formulation including the adjuvant to the skin.

Moreover, the TCI system and devices of the present disclosure may be used to deliver a separate antigen formulation to a subject subsequent to delivery of a first formulation. The first formulation may comprise an antigen and an adjuvant or the first formulation may comprise an antigen alone. The TCI system and the devices of the present disclosure provide various methods of delivering separate formulations to a subject at various sites. In one embodiment, a separate or second formulation may be delivered to the same site or area as the first formulation, such as the same draining lymph node field. For example, studies have shown topical application of immunostimulating patches containing heat-labile enterotoxin of E. coli (LT) as an adjuvant, enhances immune responses to injected vaccines such as the influenza virus. The LT immunostimulating patch is placed on the skin at the site of vaccine injection. (Xabier et al., 2003, J. of Virology, vol. 77, 5218-5225; Xabier et al. 2004, J. of Virology, vol. 78, 7610-7618,2004).

The term "draining lymph node field" as used in the disclosure means an anatomic area over which the lymph collected is filtered through a set of defined lymph nodes (e.g., cervical, axillary, inguinal, epitrochelear, popliteal, those of the abdomen and thorax). The same draining lymph node field may be targeted by immunization (e.g., enteral, mucosal, transcutaneous, transdermal, other parenteral methods of administration) within the few minutes to days required for antigen presenting cells to migrate to the lymph nodes if the sites and times of immunization are appropriately spaced to bring different components of the formulation together (e.g., two closely located patches with either adjuvant or antigen applied at the same time may be effective when neither alone would be successful). For example, a patch delivering adjuvant by the transcutaneous technique may be placed on the same arm as is injected with a conventional vaccine to boost its effectiveness in elderly, pediatric, or other immunologically compromised populations.

### Methods of Treating the Skin

The devices of the present disclosure may be used in conjunction with other methods of treating the skin to enhance skin penetration.

U.S. Patent 6,797,276 discloses treating the skin prior to or concurrently with applying the antigen using the TCI system. The objective of chemical, physical, or mechanical penetration enhancement in conjunction with TCI is to disrupt at least the stratum corneum or deeper epidermal layer without perforating the skin through to the dermal layer. The disruption is preferably accomplished with minor discomfort at most to the human or animal subject and without bleeding at the site.

In TCI, the skin may be treated with skin penetration agent including a hydrating or chemical agent. Thus, in addition to disrupting the skin with the devices of the present disclosure, the skin may be swabbed with an applicator (e.g., adsorbent material on a pad or stick) containing hydration or chemical penetration agents or they may be applied directly to skin. The skin may be hydrated or dried prior to use of the device. For example, aqueous solutions (e.g., water, saline, other buffers), acetone, alcohols (e.g., isopropyl alcohol), detergents (e.g., sodium dodecyl sulfate), depilatory or keratinolytic agents (e.g., calcium hydroxide, salicylic acid, ureas), humectants (e.g., glycerol, other glycols), polymers (e.g., polyethylene or propylene glycol, polyvinyl pyrrolidone), or combinations thereof may be used or incorporated in the formulation.

Hydration of the intact or skin before, during, or immediately after application of the formulation is preferred and may be required in some or many instances. For example, hydration may increase the water content of the topmost layer of skin (e.g. , stratum corneum or superficial epidermis layer exposed by penetration enhancement techniques) above 25%, 50% or 75%.

The skin also may be disrupted using the devices of the present disclosure and other physical or mechanical means. Some examples include disrupting the skin with abrasives like an emery board or paper, sand paper, fibrous pad, and pumice; removing a superficial layer of skin by peeling or stripping with an adhesive tape; microporating the skin using an energy source such as heat, light, sound, electrical, magnetic; a skin disruption device (e.g., gun, microneedle); or combinations thereof to act as a physical penetration enhancer. See WO98/29134 for microporation of skin and U.S. Pat. No. 6,090,790 for microneedles and U.S. Pat. No. 6,168,587 for transdermal guns which might be adapted for use in transcutaneous vaccination.

As an example, iontophoresis techniques are well known but have limitations on effectiveness for drug or vaccine delivery. In one embodiment, a mask and abrade, strip pull, or site and spin device may be used prior to application of an iontophoretic patch. The combination may have a additive or synergistic effect to improve delivery over either technique alone. Similarly, other chemical or physical penetration enhancing techniques may be employed in conjunction with mask/disruption. Laser ablation, microporation by heat, radio frequency, microneedles and similar devices, ultrasound, are examples of penetration enhancing techniques that may also be employed in conjunction with the devices of the present disclosure.

### Formulations

The present disclosure also provides delivery of formulations including one or more therapeutic agents for treating various diseases using the TCI system and the devices of the present disclosure. In one embodiment, the formulation includes an antigen and an adjuvant and a pharmaceutically acceptable carrier. In another embodiment, the formulation includes an antigen or consists essentially of an antigen. The formulation may include more than one antigen and/or adjuvant. In an alternative embodiment, the formulation includes a single molecule that is both an antigen and an adjuvant or the formulation may include an adjuvant or consists essentially of an adjuvant. Moreover, the formulation may include one or more drugs and may in addition to the antigen and/or adjuvant include one or more drugs. The formulation may also include a penetration enhancer, such as a hydrating agent or a chemical agent. However, the formulation need not include a penetration enhancer.

The formulation may be in liquid or dry form. The formulation may be provided as a liquid: cream, emulsion, gel, lotion, ointment, paste, solution, suspension, or other liquid forms. A dry formulation is more easily stored and transported than conventional vaccines, it breaks the cold chain required from the vaccine's place of manufacture to the locale where vaccination occurs. Without being limited to any particular mode of action, another way in which a dry formulation may be an improvement over liquid formulations is that high concentrations of a dry active component of the formulation (e.g., one or more adjuvants and/or antigens) may be achieved by solubilization directly at the site of immunization over a short time span. Moisture from the skin (e.g., perspiration) and an occlusive dressing may hasten this process. In this way, it is possible that a concentration approaching the solubility limit of the active ingredient may be achieved in situ. Alternatively, the dry, active ingredient of the formulation per se may be an improvement by providing a solid particulate form that is taken up and processed by antigen presenting cells. These possible mechanisms are discussed not to limit the scope of the disclosure or its equivalents, but to provide insight into the operation of the disclosure and to guide the use of this formulation in immunization and vaccination.

Dry formulations may be provided in various forms: for example, fine or granulated powders, uniform films, pellets, and tablets. The formulation may be dissolved and then dried in a container or on a flat surface (e.g., skin), or it may simply be dusted on the flat surface. It may be air dried, dried with elevated temperature, freeze or spray dried, coated or sprayed on a solid substrate and then dried, dusted on a solid substrate, quickly frozen and then slowly dried under vacuum, or combinations thereof. If different molecules are active ingredients of the formulation, they may be mixed in solution and then dried, or mixed in dry form only. Compartments or chambers of the patch may be used to separate active ingredients so that only one of the antigens or adjuvants is kept in dry form prior to administration; separating liquid and solid in this manner allows control over the time and rate of the dissolving of at least one dry, active ingredient.

Formulation in liquid or solid form may be applied with one or more adjuvants and/or antigens both at the same or separate sites or simultaneously or in frequent, repeated applications. The patch may include a controlled-release reservoir or a rate-controlling matrix or membrane may be used which allows stepped release of adjuvant and/or antigen. It may contain a single reservoir with adjuvant and/or antigen, or multiple reservoirs to separate individual antigens and adjuvants. The patch may include additional antigens such that application of the patch induces an immune response to multiple antigens. In such a case, antigens may or may not be derived from the same source, but they will have different chemical structures so as to induce an immune response specific for different antigens. Multiple patches may be applied simultaneously; a single patch may contain multiple reservoirs. For effective treatment, multiple patches may be applied at intervals or constantly over a period of time; they may be applied at different times, for overlapping periods, or simultaneously. At least one adjuvant and/or adjuvant may be maintained in dry form prior to administration. Subsequent release of liquid from a reservoir or entry of liquid into a reservoir containing the dry ingredient of the formulation will at least partially dissolve that ingredient.

Solids (e.g., particles of nanometer or micrometer dimensions) may also be incorporated in the formulation. Solid forms (e.g., nanoparticles or microparticles) may aid in dispersion or solubilization of active ingredients; assist in carrying the formulation through superficial layers of the skin; provide a point of attachment for adjuvant, antigen, or both to a substrate that can be opsonized by antigen presenting cells, or combinations thereof. Prolonged release of the formulation from a porous solid formed as a sheet, rod, or bead acts as a depot.

In addition to the therapeutic agent, the formulation may comprise a vehicle. For example, the formulation may comprise an AQUAPHOR, Freund, Ribi, or Syntex emulsion; water-in-oil emulsions (e.g., aqueous creams, ISA-720), oil-in-water emulsions (e.g., oily creams, ISA-51, MF59), microemulsions, anhydrous lipids and oil-in-water emulsions, other types of emulsions; gels, fats, waxes, oil, silicones, and humectants (e.g., glycerol).

At least one ingredient or component of the formulation (i.e., antigen, adjuvant, or drug) may be provided in dry form prior to administration of the formulation and/or as part of a patch. This system may also be used in conjunction with conventional enteral, mucosal, or parenteral immunization techniques.

The formulation may be manufactured under aseptic conditions acceptable to appropriate regulatory agencies (e.g., Food and Drug Administration) for biologicals and vaccines. Optionally, components such as desiccants, excipients, stabilizers, humectants, preservatives, adhesives, patch materials, or combinations thereof may be included in the formulation even though they are immunologically inactive. They may, however, have other desirable properties or characteristics.

Processes for manufacturing a pharmaceutical formulation are well known. The components of the formulation may be combined with a pharmaceutically-acceptable carrier or vehicle, as well as any combination of optional additives (e.g., diluents, binders, excipients, stabilizers, desiccants, preservatives, colorings). The use of solid carriers, and the addition of excipients to assist in solubilization of dry components or stabilizers of antigenic or adjuvant activity, are preferred embodiments. See, generally, Ullmann's Encyclopedia of Industrial Chemistry, 6th Ed. (electronic edition, 2003); Remington's Pharmaceutical Sciences, 22nd (Gennaro, 2005, Mack Publishing); Pharmaceutical Dosage Forms, 2nd Ed. (various editors, 1989-1998, Marcel Dekker); and Pharmaceutical Dosage Forms and Drug Delivery Systems (Ansel et al., 2005, Williams & Wilkins).

Good manufacturing practices are known in the pharmaceutical industry and regulated by government agencies (e.g., Food and Drug Administration). Sterile liquid formulations may be prepared by dissolving an intended component of the formulation in a sufficient amount of an appropriate solvent, followed by sterilization by filtration to remove contaminating microbes. Generally, dispersions are prepared by incorporating the various sterilized components of the formulation into a sterile vehicle which contains the basic dispersion medium. For production of solid forms that are required to be sterile, vacuum drying or freeze drying can be used.

In general, solid dosage forms (e.g., powders, granules, pellets, tablets) can be made from at least one active ingredient or component of the formulation.

Suitable tableting procedures and the production of patches are known. The formulation may also be produced by encapsulating solid forms of at least one active ingredient, or keeping them separate from liquids in compartments or chambers. In one embodiment, the patch may include a pouch containing a vehicle (e.g., a saline solution) which is disrupted by pressure and subsequently solubilizes the dry formulation of the patch. The size of each dose and the interval of dosing to the subject may be used to determine a suitable size and shape of the tablet, capsule, compartment, or chamber.

Formulations will contain an effective amount of the active ingredients (e.g., drug, antigen and adjuvant) together with carrier or suitable amounts of vehicle in order to provide pharmaceutically-acceptable compositions suitable for administration to a human or animal. Formulation that include a vehicle may be in the form of an cream, emulsion, gel, lotion, ointment, paste, solution, suspension, or other liquid forms known in the art; especially those that enhance skin hydration.

The relative amounts of active ingredients within a dose and the dosing schedule may be adjusted appropriately for efficacious administration to a subject (e.g., animal or human). This adjustment may also depend on the subject's particular disease or condition, and whether treatment or prophylaxis is intended. To simplify administration of the formulation to the subject, each unit dose contains the active ingredients in predetermined amounts for a single round of immunization.

There are numerous causes of polypeptide instability or degradation, including hydrolysis and denaturation. In the case of denaturation, the conformation or three-dimensional structure of the protein is disturbed and the protein unfolds from its usual globular structure. Rather than refolding to its natural conformation, hydrophobic interaction may cause clumping of molecules together (i.e., aggregation) or refolding to an unnatural conformation. Either of these results may entail diminution or loss of antigenic or adjuvant activity. Stabilizers may be added to lessen or prevent such problems.

The formulation, or any intermediate in its production, may be pretreated with protective agents (i.e., cryoprotectants and dry stabilizers) and then subjected to cooling rates and final temperatures that minimize ice crystal formation. By proper selection of cryoprotective agents and use of pre-selected drying parameters, almost any formulation might be cryoprepared for a suitable desired end use.

It should be understood in the following discussion of optional additives like excipients, stabilizers, desiccants, and preservatives are described by their function. Thus, a particular chemical may act as some combination of recipient, stabilizer, desiccant, and/or preservative. Such chemical would be immunologically-inactive because it does not directly induce an immune response, but it increases the response by enhancing immunological activity of the antigen or adjuvant: for example, by reducing modification of the antigen or adjuvant, or denaturation during drying and dissolving cycles.

Stabilizers include cyclodextrin and derivatives thereof (see U.S. Pat. No. 5,730,969). Suitable preservatives such as sucrose, mannitol, sorbitol, trehalose, dextran, and glycerin can also be added to stabilize the final formulation (Howell and Miller, 1983). A stabilizer selected from nonionic surfactants, D-glucose, D-galactose, D-xylose, D-glucuronic acid, salts of D-glucuronic acid, trehalose, dextrans, hydroxyethyl starches, and mixtures thereof may be added to the formulation. Addition of an alkali metal salt or magnesium chloride may stabilize a polypeptide, optionally including serum albumin and freeze-drying to further enhance stability. A polypeptide may also be stabilized by contacting it with a saccharide selected from the group consisting of dextran, chondroitin sulfuric acid, starch, glycogen, insulin, dextrin, and alginic acid salt. Other sugars that can be added include monosaccharides, disaccharides, sugar alcohols, and mixtures thereof (e.g., glucose, mannose, galactose, fructose, sucrose, maltose, lactose, mannitol, xylitol). Polyols may stabilize a polypeptide, and are water-miscible or water-soluble. Suitable polyols may be polyhydroxy alcohols, monosaccharides and disaccharides including mannitol, glycerol, ethylene glycol, propylene glycol, trimethyl glycol, vinyl pyrrolidone, glucose, fructose, arabinose, mannose, maltose, sucrose, and polymers thereof. See, for example, Hanson and Roun (1992). Various excipients may also stabilize polypeptides, including serum albumin, amino acids, heparin, fatty acids and phospholipids, surfactants, metals, polyols, reducing agents, metal chelating agents, polyvinyl pyrrolidone, hydrolyzed gelatin, and ammonium sulfate.

As an example, single-dose tetanus vaccine can be stabilized in poly(lactic acid) (PLA) and poly(lactide-co-glycolide) (PLGA) microspheres by suitable choice of excipient or stabilizer (Sanchez et al., 1999). Trehalose may be advantageously used as an additive because it is a non-reducing saccharide, and therefore does not cause aminocarbonyl reactions with substances bearing amino groups such as proteins.

The formulation may be administered directly to the skin of the subject. For example, the skin may be swabbed with an applicator containing the formulation or the formulation may be applied directly to the skin. The skin may be hydrated or dry prior to administration of the formulation. The formulation may also be applied using a patch.

A "patch" refers to a product which includes a solid substrate (e.g., occlusive or non-occlusive surgical dressing) as well as at least one active ingredient. Liquid may be incorporated in a patch (i.e., a wet patch). One or more active components of the formulation may be applied on the substrate, incorporated in the substrate or adhesive of the patch, or combinations thereof. A dry patch may or may not use a liquid reservoir to solubilize the formulation.

The moisture content of the adhesive layer may be more than 0.5%, more than 1%, more than 2%, less than 10%, less than 5%, less than 2%, and intermediate ranges thereof. The patch may be a pliable, planar substrate from about 1 cm² to about 100 cm². An effective amount of the protein is provided by a single patch. For example, the patch may comprise an amount of protein between 1 µg and 1 mg, 5 µg and 500 µg, 10 µg and 100 µg, or intermediate ranges thereof. Depending on the immunologic activity of the protein, the effective amount of a particular protein may vary. The patch may be stored in a moisture-proof package (e.g., blister pack, foil pouch) for at least one or two years at room temperature (e.g., 20°C. to 30°C) with an immunological activity between 85% and 115% of the patch's initial activity.

A formulation that can be administered to the subject in a dry, non-liquid form, may allow storage in conditions that do not require a cold chain. For example, an antigen in solution such as diphtheria toxoid is mixed in solution with an adjuvant such as CT and is placed on a gauze pad with an occlusive backing such as plastic wrap (Saran) and allowed to dry. This patch can then be placed on skin with the gauze side in direct contact with the skin for a period of time and can be held in place covered with a simple occlusive such as plastic wrap (e.g., Saran Wrap) and adhesive tape. Such a patch may have many compositions. The matrix holding the antigen may be cotton gauze, combinations of rayon blends or other synthetic materials and may have occlusive solid backings including polyvinyl chloride, rayons, other plastics, gels, creams, emulsions, waxes, oils, parafilm, rubbers (synthetic or natural), cloths, or membranes. The patch can be held onto the skin and components of the patch can be held together using various adhesives that are well known.

Patches for transdermal delivery of drugs are well known in the pharmaceutical arts as a method for delivering a wide variety of drugs, such as nitroglycerin, estradiol, testosterone, fentanyl, and clonidine. These patches typically contain a carrier (such as a liquid, gel, or solid matrix, or a pressure sensitive adhesive) into which the drug to be delivered is incorporated. For example, U.S. Patent No. 7,097,853 discloses a transdermal drug delivery device containing a macromonomer-containing acrylate or methacrylate copolymer, a softener, and a drug. The mixture of the copolymer and softener also serves as a skin adhesive.

U.S. Patent Application Publication No. 2004/0137004, discloses a protein-in-adhesive patch for transcutaneous immunization. The patch includes at least four different components: (i) backing layer; (ii) pressure-sensitive adhesive adhering to the backing layer; (iii) at least one immunologically-active protein of an immunogenic formulation applied to the pressure-sensitive adhesive layer opposite the backing layer and/or incorporated in the pressure-sensitive adhesive layer so that the protein is in contact with adhesive; and (iv) stabilizer that maintains the immunological activity of the protein under ambient conditions.

A liquid or quasi-liquid formulation may also be applied directly to the skin and allowed to air dry; rubbed into the skin or scalp; placed on the ear, inguinal, or intertriginous regions, especially in animals; placed on the anal/rectal tissues; held in place with a dressing, patch, or absorbent material; immersion; otherwise held by a device such as a stocking, slipper, glove, or shirt; or sprayed onto the skin to maximize contact with the skin. But, this disclosure provides a formulation with at least one active ingredient in solid form. The formulation may be applied in an absorbent dressing or gauze. The formulation may be covered with an occlusive dressing such as, for example, AQUAPHOR (an emulsion of petrolatum, mineral oil, mineral wax, wool wax, panthenol, bisabol, and glycerin from Beiersdorf, Inc.), plastic film, COMFEEL (Coloplast) or vaseline; or a non-occlusive dressing such as, for example, TEGADERM (3M), DUODERM (3M) or OPSITE (Smith & Napheu). An occlusive dressing excludes the passage of water. The formulation may be applied to single or multiple sites, to single or multiple limbs, or to large surface areas of the skin by complete immersion. The formulation may be applied directly to the skin. Other substrates that may be used are pressure-sensitive adhesives such as acrylics, polyisobutylenes, and silicones. The formulation may be incorporated directly into such substrates, perhaps with the adhesive per se instead of adsorption to a porous pad (e.g., gauze) or bilious strip (e.g., filter paper).

A formulation with dry adjuvant may be produced in such a way that it is a universal adjuvant; that is, any vaccine may be added to the basic formulation with dry adjuvant. Such a universal adjuvant may be incorporated into a patch. The added vaccine may be aqueous and subsequently dried before immunization, or may be added in aqueous environment and applied to the vaccine.

Whether or not a patch is used, polymers added to the formulation may act as an excipient, stabilizer, and/or preservative of an active ingredient as well as reducing the concentration of the active ingredient that saturates a solution used to dissolve the dry form of the active ingredient. Such reduction occurs because the polymer reduces the effective volume of the solution by filling the "empty" space. Thus, quantities of antigen/adjuvant can be conserved without reducing the amount of saturated solution. An important thermodynamic consideration is that an active ingredient in the saturated solution will be "driven" into regions of lower concentration (e.g., through the skin). In solution, polymers can also stabilize and/or preserve the antigen/adjuvant-activity of solubilized ingredients of the formulation. Such polymers include ethylene or propylene glycol, vinyl pyrrolidone, and 0-cyclodextrin polymers and copolymers.

A single or unit dose of formulation suitable for administration is provided. The amount of adjuvant or antigen in the unit dose may be anywhere in a broad range from about 0.001 µg to about 10 mg. This range may be from about 0.1 ug to about 1 mg; a narrower range is from about 5 µg to about 500 µg. Other suitable ranges are between about 1 µg and about 10 µg, between about 10 µg and about 50 µg, between about 50 µg and about 200 µg, and between about 1 mg and about 5 mg. A preferred dose for a toxin is about 50 µg or 100 µg or less (*e.g.*, from about 1 µg to about 50 µg or 100 µg). The ratio between antigen and adjuvants may be about 1:1 (*e.g., E. coli* heat-labile enterotoxin when it is both antigen and adjuvant) but higher ratios may be suitable for poor antigens (*e.g.,* about 1:10 or less), or lower ratios of antigen to adjuvant may also be used (*e.g.*, about 10:1 or more). The native ratios between LT and ETEC antigens may be used for whole-cell or lysate formulations.

A formulation comprising an antigen and/or an adjuvant may be applied to skin of a human or animal subject, antigen is presented to immune cells, and an antigen-specific immune response is induced. This may occur before, during, or after infection by pathogen. Only antigen or polynucleotide encoding antigen may be required, but no additional adjuvant, if the immunogenicity of the formulation is sufficient to not require adjuvant activity. The formulation may include an additional antigen such that application of the formulation induces an immune response against multiple antigens (i.e., multivalent). In such a case, antigens may or may not be derived from the same source, but the antigens will have different chemical structures so as to induce immune responses specific for the different antigens. Antigen-specific lymphocytes may participate in the immune response and, in the case of participation by B lymphocytes, antigen-specific antibodies may be part of the immune response. The formulations described above may include desiccants, excipients, humectants, stabilizers, preservatives, adhesives, and patch materials known in the art.

The disclosure is used to treat a subject (e.g., a human or animal in need of treatment such as prevention of disease, protection from effects of infection, reducing or alleviating the symptoms of a disease, such as traveler's diarrhea, or combinations thereof). When the antigen is derived from a pathogen, the treatment may vaccinate the subject against infection by the pathogen or against its pathogenic effects such as those caused by toxin secretion. The disclosure may be used therapeutically to treat existing disease, protectively to prevent disease, to reduce the severity and/or duration of disease, to ameliorate symptoms of disease, or combinations thereof.

The application site may be protected with anti-inflammatory corticosteroids such as hydrocortisone, triamcinolone, and mometazone or non-steroidal anti-inflammatory drugs (NSAID) to reduce possible local skin reaction or modulate the type of immune response. Similarly, anti-inflammatory steroids or NSAID may be included in the patch material, or liquid or solid formulations; and corticosteroids or NSAID may be applied after immunization. IL-10, TNF-α, other immunomodulators may be used instead of the anti-inflammatory agents. Moreover, the formulation may be applied to skin overlying more than one draining lymph node field using either single or multiple applications. The formulation may include additional antigens such that application induces an immune response to multiple antigens. In such a case, the antigens may or may not be derived from the same source, but the antigens will have different chemical structures so as to induce an immune response specific for the different antigens. Multi-chambered patches could allow more effective delivery of multivalent vaccines as each chamber covers different antigen presenting cells. Thus, antigen presenting cells would encounter only one antigen (with or without adjuvant) and thus would eliminate antigenic competition and thereby enhancing the response to each individual antigen in the multivalent vaccine.

The formulation may be epicutaneously applied to skin to prime or boost the immune response in conjunction with penetration techniques or other routes of immunization. Priming by TCI with either single or multiple applications may be followed with enteral, mucosal, parenteral, and/or transdermal techniques for boosting immunization with the same or altered antigens. Priming by enteral, mucosal, parenteral, and/or transdermal immunization with either single or multiple applications may be followed with transcutaneous techniques for boosting immunization with the same or altered antigens. It should be noted that TCI is distinguished from conventional topical techniques like mucosal or transdermal immunization because the former requires a mucous membrane (e.g., lung, mouth, nose, and rectum) not found in the skin and the latter requires perforation of the skin through the dermis. The formulation may include additional antigens such that application to skin induces an immune response to multiple antigens.

The formulations of the present disclosure may not be formulated without any liposomes or transferosomes.

### Therapeutic Agents

The devices of the present disclosure and the TCI system may be used to deliver formulations including therapeutic agents such as drugs, antigens, and adjuvants into or through the skin. The formulation may include a vaccine. The vaccine may include an antigen and an adjuvant or an antigen alone. The formulation may include an adjuvant alone.

In one embodiment, the therapeutic agents are drugs, such as anesthetics, analgesics, anti-inflammatories, steroids, antibiotics, antiarthritics, anorectics, antihistamines, and antineoplastics. For example, lidocaine and nonsteroidal anti-inflammatory drugs (NSAID) may be delivered transdermally with the devices of the present disclosure. In another embodiment, the therapeutic agents are antigens and adjuvants and are delivered via transcutaneously.

In one embodiment, the therapeutic agents are drugs, such as anesthetics, analgesics, anti-inflammatories, steroids, antibiotics, antiarthritics, anorectics, antihistamines, and antineoplastics. In another embodiment, the therapeutic agents are antigens and adjuvants.

**Antigens.** The TCI system delivers an effective amount of one or more agents to specialized cells (e.g., antigen presentation cell, lymphocyte) to produce an immune response. These agents as a class are called antigens. Antigen may be composed of chemicals such as, for example, carbohydrate, glycolipid, glycoprotein, lipid, lipoprotein, phospholipid, polypeptide, conjugates thereof, or any other material known to induce an immune response. Antigen may be provided as a whole organism such as, for example, a bacterium or virion; antigen may be obtained from an extract or lysate, either from whole cells or membrane alone; or antigen may be chemically synthesized or produced by recombinant means. An antigen may also be derived from a pathogen.

Antigen of the disclosure may be expressed by recombinant means, preferably as a fusion with an affinity or epitope tag (Summers and Smith, 1987; Goeddel, 1990; Ausubel et al., 1996); chemical synthesis of an oligopeptide, either free or conjugated to carrier proteins, may be used to obtain antigen of the disclosure (Bodanszky, 1993; Wisdom, 1994). Oligopeptides are considered a type of polypeptide. Oligopeptide lengths of 6 residues to 20 residues are preferred. Polypeptides may also be synthesized as branched structures such as those disclosed in U.S. Patent Nos. 5,229,490 and 5,390,111. Antigenic polypeptides include, for example, synthetic or recombinant B-cell and T-cell epitopes, universal T-cell epitopes, and mixed T-cell epitopes from one organism or disease and B-cell epitopes from another. Antigen obtained through recombinant means or peptide synthesis, as well as antigen of the disclosure obtained from natural sources or extracts, may be purified by means of the antigen's physical and chemical characteristics, preferably by fractionation or chromatography (Janson and Ryden, 1997; Deutscher, 1990; Scopes, 1993). Recombinants may combine B subunits or chimeras of bAREs (Lu et al., 1997). A multivalent antigen formulation may be used to induce an immune response to more than one antigen at the same time. Conjugates may be used to induce an immune response to multiple antigens, to boost the immune response, or both. Additionally, toxins may be boosted by the use of toxoids, or toxoids boosted by the use of toxins. Transcutaneous immunization may be used to boost responses induced initially by other routes of immunization such as by oral, nasal or parenteral routes. Antigen includes, for example, toxins, toxoids, subunits thereof, or combinations thereof (e.g., cholera toxin, tetanus toxoid); additionally, toxins, toxoids, subunits thereof, or combinations thereof may act as both antigen and adjuvant Such oral/transcutaneous or transcutaneous/oral immunization may be especially important to enhance mucosal immunity in diseases where mucosal immunity correlates with protection.

Antigen may be solubilized in a buffer or water or organic solvents such as alcohol or DMSO, or incorporated in gels, emulsion, microemulsions, and creams. Suitable buffers include, but are not limited to, phosphate buffered saline Ca⁺⁺ /Mg⁺⁺ free (PBS), phosphate buffered saline (PBS), normal saline (150 mM NaCl in water), and Tris buffer. Antigen not soluble in neutral buffer can be solubilized in 10 mM acetic acid and then diluted to the desired volume with a neutral buffer such as PBS. In the case of antigen soluble only at acid pH, acetate-PBS at acid pH may be used as a diluent after solubilization in dilute acetic acid. Glycerol may be a suitable nonaqueous buffer for use in the disclosure.

Hydrophobic antigen can be solubilized in a detergent, for example a polypeptide containing a membrane-spanning domain. Furthermore, for formulations containing liposomes, an antigen in a detergent solution (e.g., a cell membrane extract) may be mixed with lipids, and liposomes then may be formed by removal of the detergent by dilution, dialysis, or column chromatography, see, Gregoriadis (1995). Certain antigens such as, for example, those from a virus (e.g., hepatitis A) need not be soluble per se, but can be incorporated directly into a lipid membrane (e.g., a virosome as described by Morein and Simons, 1985), in a suspension of virion alone, or suspensions of microspheres or heat-inactivated bacteria which may be taken up by activate antigen presenting cells (e.g., opsonization). Antigens may also be mixed with a penetration enhancer as described in WO 99/43350.

Plotkin and Mortimer (1994) provide antigens which can be used to vaccinate animals or humans to induce an immune response specific for particular pathogens, as well as methods of preparing antigen, determining a suitable dose of antigen, assaying for induction of an immune response, and treating infection by a pathogen (e.g., bacterium, virus, fungus, or parasite).

Bacteria include, for example: Bacillus anthracis (anthrax), campylobacter, cholera, clostridia including clostridium difficile, diphtheria, enterohemorrhagic E. coli, enterotoxigenic E. coli, giardia, gonococcus, Helicobacter pylori or urease produced by H. pylori (Lee and Chen, 1994), Hemophilus influenza B, Hemophilus influenza non-typable, Legionella, meningococcus, mycobacterium including those organisms responsible for tuberculosis, pertussis, pneumococcus, salmonella, shigella, staphylococcus and their enterotoxins, Group A beta-hemolytic streptococcus, Streptococcus B, tetanus, Vibrio cholerae, Borrelia burgdorfi and Yersinia; and products thereof.

Viruses include, for example: adenovirus, dengue serotypes 1 to 4 (Delenda et al., 1994; Fonseca et al., 1994; Smucny et al., 1995), ebola (Jahrling et al., 1996), enterovirus, hanta virus, hepatitis serotypes A to E (Blum, 1995; Katkov, 1996; Lieberman and Greenberg, 1996; Mast, 1996; Shafara et al., 1995; Smedila et al., 1994; U.S. Pat. Nos. 5,314,808 and 5,436,126), herpes simplex virus 1 or 2, human immunodeficiency virus (Deprez et al., 1996), human papilloma virus, influenza, measles, Norwalk, Japanese equine encephalitis, papilloma virus, parvovirus B19, polio, rabies, respiratory syncytial virus, rotavirus, rubella, rubeola, St. Louis encephalitis, vaccinia, viral expression vectors containing genes coding for other antigens such as malaria antigens, varicella, and yellow fever; and products thereof.

As an example, the influenza virus could be provided in various forms. The influenza virus could be inactivated (killed), attenuated, split, whole, live, purified or a combination thereof, such as attenuated live virus, or purified split virus. There are currently three influenza virus vaccines available in the United States. All are trivalent vaccines, standardized to contain the hemagglutinins, a surface glycoprotein from 2 type A and one type B influenza strains. Two of the influenza vaccines, Fluzone^{®} (Aventis Pasteur) and Fluvirin^{®} (Evans Vaccine Ltd/Chiron), are trivalent inactivated split-virus vaccines (TIV) for intramuscular administration. The term "split-virus" is used interchangeably with "subviron" or "purified antigen preparation." Fluzone^{®} is approved by the Food and Drug Administration (FDA) for use in patients 6 months of age or older. Fluvirin^{®} is approved in children 4 years of age and older.

Parasites include, for example: Entamoeba histolytica (Zhang et al., 1995); Plasmodium (Bathurst et al., 1993; Chang et al., 1989, 1992, 1994; Fries et al., 1992a, 1992b; Herrington et al., 1991; Khusmith et al., 1991; Malik et al., 1991; Migliorini et al., 1993; Pessi et al., 1991; Tam, 1988; Vreden et al., 1991; White etal., 1993; Wiesmueller et al., 1991), Leishmania (Frankenburg et al., 1996), and the Helminthes; Schistosomes; and products thereof.

Fungi including entities responsible for tinea corporis, tinea unguis, sporotrichosis, aspergillosis, candida and other pathogenic fungi.

Antigen may be derived from any pathogen that infects a human or animal subject (e.g., bacterium, virus, fungus, or protozoan). For example, a pathogen may produce toxins that are useful as antigens. As an example, Bacillus anthracis produces a toxin composed of three distinct proteins. These are termed protective antigen (PA), oedema factor, and lethal factor. Protective antigen is secreted in a precursor form which can heptamerize and form a channel in membranes, which allows the other two factors to enter the target cell. Oedema factor is an adenylate cyclase which leads to an impairment of host defenses. Lethal factor is a protease causing the lysis of macrophages.

PA of anthrax toxin is the major component of human anthrax vaccine. The PA may be the recombinant and/or purified form. Singh et al. (Infect. Immun., 1998, 66(7):3447-3448) evaluated the efficacy of the purified mutant PA protein alone or in combination with the lethal factor and edema factor components of anthrax toxin to protect against anthrax. Singh et al. reported that both mutant and native PA preparations elicited high anti-PA titers in Hartley guinea pigs. Mutant PA alone and in combination with lethal factor and edema factor completely protected the guinea pigs from B. anthracis spore challenge. The mutant PA protein may be used to develop an effective recombinant vaccine against anthrax.

The chemical structure of the antigen may be described as one or more of carbohydrate, fatty acid, and protein (e.g., glycolipid, glycoprotein, and lipoprotein). An antigen used in the present disclosure may or may not include a polynucleotide.

In one embodiment, the antigen is a proteinaceous antigen. The molecular weight of the antigen may be greater than 500 daltons, 800 daltons, 1000 daltons, 10 kilodaltons, 100 kilodaltons, or 1000 kilodaltons. Chemical, mechanical, or physical penetration enhancement may be used with for macromolecular structures like cells, viral particles, and molecules of greater than one megadalton (e.g., CS6 antigen), but techniques like hydration and swabbing with a solvent may be sufficient to induce immunization into the skin. Antigen may be obtained by recombinant techniques, chemical synthesis, or at least partial purification from a natural source. It may be a chemical or recombinant conjugates: for example, linkage between chemically reactive groups or protein fusion. Antigen may be provided as a live cell or virus, an attenuated live cell or virus, a killed cell, or an inactivated virus. Alternatively, antigen may be at least partially purified in cell-free form (e.g., cell or viral lysate, membrane or other subcellular fraction). Because most adjuvants would also have immunogenic activity and would be considered antigens, adjuvants would also be expected to have the aforementioned properties and characteristics of antigens.

**Adjuvant.** The formulation may also contain an adjuvant, although a single molecule may contain both adjuvant and antigen properties (e.g., cholera toxin) (Elson and Dertzbaugh, 1994). Adjuvants are substances that are used to specifically or non-specifically potentiate an antigen-specific immune response. Usually, the adjuvant and the formulation are mixed prior to presentation of the antigen but, alternatively, they may be separately presented within a short interval of time. Adjuvants include, for example, an oil emulsion (e.g., complete or incomplete Freund's adjuvant), a chemokine (e.g., defensins 1 or 2, RANTES, MIP1-α, MIP-2, interleukin-8, or a cytokine (e.g., interleukin-1β, -2, -6, -10 or -12; interferon-γ; tumor necrosis factor-α; or granulocyte-monocyte-colony stimulating factor) (reviewed in Nohria and Rubin, 1994), a muramyl dipeptide derivative (e.g., murabutide, threonyl-MDP or muramyl tripeptide), a heat shock protein or a derivative, a derivative of Leishmania major LeIF (Skeiky et al., 1995), cholera toxin or cholera toxin B, bacterial ADP-ribosylating exotoxins and their subunits, or recombinants utilizing the bacterial ADP-ribosylating exotoxins or their subunits, a lipopolysaccharide (LPS) derivative (e.g., lipid A or monophosphoryl lipid A or lipid A analogs), superantigen (Saloga et al., 1996b), mutant bacterial ADP-ribosylating exotoxins including mutants at the trypsin cleavage site (Dickenson and Clements, 1995) and affecting ADP-ribosylation (Douce et al., 1997), QS21, Quill A or alum. Also, see Richards et al. (1995) for other adjuvants useful in immunization.

An adjuvant may be chosen to preferentially induce antibody or cellular effectors, specific antibody isotypes (e.g., IgM, IgD, IgA1, IgA2, secretory IgA, IgE, IgG1, IgG2, IgG3, and/or IgG4), or specific T-cell subsets (e.g., CTL, Th1, Th2 and/or T_{DTH}) (see, for example, Munoz et al., 1990; Glenn et al., 1995).

Unmethylated CpG dinucleotides or motifs are known to activate B cells and macrophages (Kreig et al., 1995; Stacey et al., 1996). Other forms of bacterial DNA can be used as adjuvants. Bacterial DNAs are among a class of structures which have patterns allowing the immune system to recognize their pathogenic origins to stimulate the innate immune response leading to adaptive immune responses (Medzhitov and Janeway, 1997). These structures are called pathogen-associated molecular patterns (PAMPs) and include lipopolysaccharides, teichoic acids, unmethylated CpG motifs, double-stranded RNA, and mannins. PAMPs induce endogenous signals that can mediate the inflammatory response, act as co-stimulators of T-cell function and control the effector function. The ability of PAMPs to induce these responses play a role in their potential as adjuvants and their targets are APCs such as macrophages and dendritic cells. The antigen presenting cells of the skin could likewise be stimulated by PAMPs transmitted through the skin. For example, Langerhans cells, a type of dendritic cell, could be activated by a PAMP in solution on the skin with a transcutaneously poorly immunogenic molecule and be induced to migrate and present this poorly immunogenic molecule to T-cells in the lymph node, inducing an antibody response to the poorly immunogenic molecule. PAMPs could also be used in conjunction with other skin adjuvants such as cholera toxin to induce different co-stimulatory molecules and control different effector functions to guide the immune response, for example from a Th2 to a Th1 response.

For example, cholera toxin is a bacterial exotoxin from the family of ADP-ribsoylating exotoxins (referred to as bAREs). Most bAREs are organized as A:B dimer with a binding B subunit and an A subunit containing the ADP-ribosyltransferase. Such toxins include diphtheria, Pseudomonas exotoxin A, cholera toxin (CT), E. coli heat-labile enterotoxin (LT), pertussis toxin, C. botulinum toxin C2, C. botulinum toxin C3, C. limosum exoenzyme, B. cereus exoenzyme, Pseudomonas exotoxin S, Staphylococcus aureus EDIN, and B. sphaericus toxin.

Cholera toxin is an example of a bARE that is organized with A and B subunits. The B subunit is the binding subunit and is non-covalently bound to the A subunit. The B subunit pentamer is arranged in a symmetrical doughnut-shaped structure that binds to GM1-ganglioside on the target cell. The A subunit serves to ADP ribosylate the alpha subunit of a subset of the heterotrimeric GTP proteins (G proteins) including the Gs protein which results in the elevated intracellular levels of cyclic AMP. This stimulates release of ions and fluid from intestinal cells in the case of cholera. However, cholera toxin (CT) and its B subunit (CTB) have adjuvant properties when used as either an intramuscular or oral immunogen (Elson and Dertzbaugh, 1994; Trach et al., 1997). Heat-labile enterotoxin from E. coli (LT) is 75-77% homologous at the amino acid level with CT and possesses similar binding properties; it also appears to bind the GM1-ganglioside receptor in the gut and has similar ADP-ribosylating exotoxin activities. Pseudomonas exotoxin A (ETA) binds to the a2-macroglobulin receptor-low density lipoprotein receptor-related protein (Kounnas et al., 1992). bAREs are reviewed by Krueger and Barbieri (1995). CT, CTB, LT, ETA and PT, despite having different cellular binding sites, are potent adjuvants for transcutaneous immunization, inducing IgG antibodies but not IgE antibodies. CTB without CT can also induce IgG antibodies. Thus, both bAREs and a derivative thereof can effectively immunize when epicutaneously applied to the skin in a simple solution.

When an adjuvant such as CT is mixed with BSA, a protein not usually immunogenic when applied to the skin, anti-BSA antibodies are induced. An immune response to diphtheria toxoid was induced using pertussis toxin as adjuvant, but not with diphtheria toxoid alone. Native LT as an adjuvant and antigen, however, is clearly not as potent as native CT. But activated bAREs can act as adjuvants for non-immunogenic proteins in an transcutaneous immunization system. Thus, therapeutic immunization with an antigen for the organism such as HIV, HPV or leishmania could be used separately or in conjunction with immunostimulation of the infected APCs to induce a therapeutic immunization.

Protection against the life-threatening infections diphtheria, pertussis, and tetanus (DPT) can be achieved by inducing high levels of circulating anti-toxin antibodies. Pertussis may be an exception in that some investigators feel that antibodies directed to other portions of the invading organism are necessary for protection, although this is controversial (see Schneerson et al., 1996) and most new generation acellular pertussis vaccines have PT or genetically detoxified PT as a component of the vaccine (Krueger and Barbieri, 1995). The pathologies in the diseases caused by DPT are directly related to the effects of their toxins and anti-toxin antibodies most certainly play a role in protection (Schneerson et al., 1996).

In general, toxins can be chemically inactivated to form toxoids which are less toxic but remain immunogenic. The TCI system using toxin-based immunogens and adjuvants can achieve anti-toxin levels adequate for protection against these diseases. The anti-toxin antibodies may be induced through immunization with the toxins, or genetically-detoxified toxoids themselves, or with toxoids and adjuvants such as CT. Genetically toxoided toxins which have altered ADP-ribosylating exotoxin activity or trypsin cleavage site, but not binding activity, are envisioned to be especially useful as non-toxic activators of antigen presenting cells used in transcutaneous immunization and may reduce concerns over toxin use.

CT can also act as an adjuvant to induce antigen-specific CTLs through transcutaneous immunization. The bARE adjuvant may be chemically conjugated to other antigens including, for example, carbohydrates, polypeptides, glycolipids, and glycoprotein antigens. Chemical conjugation with toxins, their subunits, or toxoids with these antigens would be expected to enhance the immune response to these antigens when applied epicutaneously. To overcome the problem of the toxicity of the toxins, (e.g., diphtheria toxin is known to be so toxic that one molecule can kill a cell) and to overcome the difficulty of working with such potent toxins as tetanus, several workers have taken a recombinant approach to producing genetically produced toxoids. This is based on inactivating the catalytic activity of the ADP-ribosyl transferase by genetic deletion. These toxins retain the binding capabilities, but lack the toxicity, of the natural toxins. This approach is described by Burnette et al. (1994), Rappuoli et al. (1995), and Rappuoli et al. (1996). Such genetically toxoided exotoxins would be expected to induce a transcutaneous immune response and to act as adjuvants (Douce et al., 1997). They may provide an advantage in a transcutaneous immunization system in that they would not create a safety concern as the toxoids would not be considered toxic. Activation through a technique such as trypsin cleavage, however, would be expected to enhance the adjuvant qualities of LT through the skin which is lacking inherent trypsin enzymes. Additionally, several techniques exist to chemically toxoid toxins which can address the same problem (Schneerson et al., 1996). These techniques could be important for certain applications, especially pediatric applications, in which ingested toxins (e.g., diphtheria toxin) might possibly create adverse reactions.

Optionally, an activator of Langerhans cells may be used as an adjuvant. Examples of such activators include: an inducer of heat shock protein; contact sensitizer (e.g., trinitrochlorobenzene, dinitrofluorobenzene, nitrogen mustard, pentadecylcatechol); toxin (e.g., Shiga toxin, Staph enterotoxin B); lipopolysaccharide, lipid A, or derivatives thereof; bacterial DNA (Stacey et al., 1996); cytokine (e.g., tumor necrosis factor-α, interleukin-1β,-10, -12); a member of the TGF-β superfamily, calcium ions in solution, calcium ionophore, and a chemokine (e.g., defensins 1 or 2, RANTES, MIP-1β, MIP-2, interleukin-8).

In addition, formulations containing a small amount of cholera toxin along with at least one of IL-1β, IL-1β, TNF-α, CTB and LPS are envisioned. The molar ratio of the two parts of the formulation may be between about 0.001 and about 0.20.

If an immunizing antigen has sufficient Langerhans cell activating capabilities then a separate adjuvant may not be required, as in the case of CT which is both antigen and adjuvant. Alternatively, such antigens can be considered not to require an adjuvant to induce an immune response because they are sufficiently immunogenic. It is envisioned that whole cell preparations, live viruses, attenuated viruses, DNA plasmids, and bacterial DNA could be sufficient to immunize transcutaneously. It may also be possible to use low concentrations of contact sensitizers or other activators of Langerhans cells to induce an immune response without inducing skin lesions.

The choice of adjuvant may allow potentiation or modulation of the immune response. Moreover, selection of a suitable adjuvant may result in the preferential induction of a humoral or cellular immune response, specific antibody isotypes (e.g., IgM, IgD, IgA1, IgA2, IgE, IgG1, IgG2, IgG3, and/or IgG4), and/or specific T-cell subsets (e.g., CTL, Th1, Th2 and/or T_{DTH}). The adjuvant is preferably a chemically activated (e.g., proteolytically digested) or genetically activated (e.g., fusions, deletion or point mutants) ADP-ribosylating exotoxin or B subunit thereof. Adjuvant, antigen, or both may optionally be provided in the formulation with a polynucleotide (e.g., DNA, RNA, cDNA, cRNA) encoding the adjuvant or antigen as appropriate. Covalently closed, circular DNA such as plasmids are preferred forms of the polynucleotide; however, linear forms may also be used. The polynucleotide may include a region such as an origin of replication, centromere, telomere, promoter, enhancer, silencer, transcriptional initiation or termination signal, splice acceptor or donor site, ribosome binding site, translational initiation or termination signal, polyadenylation signal, cellular localization signal, protease cleavage site, polylinker site, or combinations thereof as are found in expression vectors.

Undesirable properties or harmful side effects of antigen or adjuvant (e.g., allergic or hypersensitive reaction; atopy, contact dermatitis, or eczema; systemic toxicity) may be reduced by modification without destroying its effectiveness in TCI. Modification may involve, for example, removal of a reversible chemical modification (e.g., proteolysis) or encapsulation in a coating which reversibly isolates one or more components of the formulation from the immune system. For example, one or more components of the formulation may be encapsulated in a particle for delivery (e.g., microspheres, nanoparticles) although we have shown that encapsulation in lipid vesicles is not required for TCI and appears to have a negative effect.

### EXAMPLES

The guinea pig is a relevant model for vaccine delivery to the skin. The stratum corneum and epidermis are similar in thickness to humans, unlike murine skin. Examples for the use of the invention are presented first using the guinea pig model and then using human subjects.

### Example 1: Studies of SPS Using Animals

*General Animal Study Methods:* Four SPS surfaces were evaluated in these studies. 1) SPS™-SP is a strip-pull device with a strip of 180 grit sandpaper (7.5 cm length and 2.5 cm width). The strip-pull was equipped with a 700 gram snap dome, which was used to control the force applied during the treatment. 2) Chemical etching (CE) is a process used to precisely modify metal surfaces. In this application, thin stainless steel strips were produced with ridges (edges) on the surface. The surfaces were designed to have one or two rows made up of 6 or 9 ridges per row. CE strips with a single row of ridges were 3.5 cm in length and 3.0 cm wide. CE strips with two rows of ridges were 7.5 cm in length and 3.0 cm wide (SPS™-CE 6/9 and SPS-CE 2x6). Ridge height was also investigated over the range of 100 µm to 250 µm. The CE strips were mounted on strip-pull devices equipped with 700 gram or 1100 gram snap dome. 3) SPS™-ME³ (micro-edge) is a third design using microblades mounted in a plastic block. In the studies described here, 7 microblades protruded 150 µm from the surface of the block (5.0 cm length and 2.0 cm width). A 400 gram snap dome was used to control the force applied with treatment. 4) ECG prep pads (Marquette Medical Systems #9386-001) are small, single use pads used to prepare skin prior to application electrocardiogram electrodes.

*Animals:* Female Hartley guinea pigs were 350-400 grams and >7 weeks old prior to the start of the study and they were judged to be in good health by a staff veternarian. Animals were ear tagged and housed in individual cages. Housing, feed, water, environmental controls, animal observations, and blood draws were performed. Immunizations and serologic evaluations were also performed.

*Influenza vaccine and LT:* Inactivated monovalent influenza vaccine included A/Wyoming/3/2003 H3N2, A/New Caledonia/20/90 H1N1, and B/Jiangsu/10/2003. Trivalent influenza vaccine (TIV) was made by blending equal amounts of hemagglutinin (HA) from each strain.

*Immunization method:* Before immunization and shaving, animals were sedated by IM injection with ketamine and xylazine using standard procedures (Genelogic and Biocon SOPs). In the infleuza model, animals were primed by intramuscular (IM) injection with 0.5 µg hemagglutinin (that is, 0.167 µg HA from each influenza strain) on study day 1, unless otherwise indicate. This was done to reflect the priming of the general population prior to receipt of the influenaz vaccine. On study day 22 to 28, groups receiving patches were shaved on the abdomen and the shaved area was treated with the indicated SPS™. Dry formulated patches (described below) containing 15 µg HA (5 µg each strain) were applied to the treated area. A control group (N = 6/group) was primed with 0.5 µg HA on study day 1 and received a bolus IM injection with 15 µg HA on study day 22.

*Patch construction and formulations:* Two dry formulated influenza/LT patch configurations were used in these studies. In protocols 1134-04221 and 5235-054, 15 µg of trivalent influenza vaccine (5 µg hemagglutinin per strain) was formulated alone or blended with LT (5 µg) and dried onto a single 1 cm² rayon/cellulose matrix. This is referred to as a "blended patch". In study protocols 5235-052, 5235-057, 5235-058, 5235-059 and 5235-068, influenza vaccine and LT were formulated and dried onto separate 1 cm² rayon/cellulose matrices. This is referred to as a "layered patch." The influenza patch was formulated with 15 µg HA and the adjuvant patch was formulated with different amounts of LT (0.5 µg to 12.5 µg), or with the vehicle buffer (no LT). TIV and LT were formulated in a carbohydrate stabilizer formulation. The TIV and/or LT formulations were applied to a rayon blend matrix and dried at 45°C for 1 hour.

*Patch application:* Patches were applied to the shaved skin immediately following pretreatement. This step is done to remove hair. Multiple studies indicate that this is not an effective pretreatment method. It is also noted that in human studies, no shaving step preceeded the use of the SPS™. In the case of layered patches, the influenza vaccine patch was applied directly to the treated skin and the LT (or no LT) patch was applied over the flu patch. In all cases, Tegaderm was applied over the patch and the abdominal area was wrapped with an adhesive tape. Patches were removed the next day (18-20 hours) and the area rinsed with water.

*Serology:* Blood samples were collected from the orbital plexus prior to immunization and two weeks after the second immunization (study day 36 or 43). Serum was collected following centrifugation and stored in labeled tubes at -20°C until assayed.

*ELISA Method:* Antibodies to influenza antigens were determined by an ELISA method. Briefly, monovalent influenza stock solutions were prepared (21 µg/ml) and 5100 µl was added to 96-well plates. The plates were sealed with plastic wrap and stored overnight at 4°C. The plates were washed four times with 300 µl/well using 0.05% Tween-20/PBS (wash buffer). The wells were blocked with 200 µl of blocking buffer (0.5% casein, 1% Tween-20 in PBS) for 2 two hours at room temperature. The plates were washed four times with washing buffer, and 5100 µl of casein diluent buffer was added to each well. Serum samples were diluted with casein buffer and 5100 µl of diluted serum was added to the appropriate well. Serum samples were 2-fold serially diluted. Plates were sealed and incubated at 4°C overnight. Plates were thoroughly rinsed with washing buffer and 5100 µl of peroxidase conjugated anti-guinea pig IgG (Heavy chain and Light chain) (diluted 1:1,000) was added to each well. After 2 two hours at room temperature, the plates were washed 4-four times with washing buffer. One hundred micro-liters of 2,2'-azinobis(3-ethylbenzthiazoline-6-sulfonate (ABTS) substrate was added to each well and the color was allowed to develop for 30 minutes at room temperature. The reaction was stopped by adding 50 µl of 2% sodium dodecyl sulfate to the wells. Titers are represented as the reciprocal value of the highest dilution that results in an O.D. 405 nm reading of 1.0 (ELISA Units or EU).Serum antibody titers are reported as ELISA. Units (EU), which is the serum dilution equal to 1 OD unit at 405 nm. The geometric mean titers (GMT) were determined for each immunization group. A T-test was used to compare the post-immunization serum antibody titers between paired groups.

### Results and Discussion:

Serum was collected prior to immunization (pre-immune) and assessed for antibody titers to each of the influenza strains. Pre-immune serum antibody titers to all strains were quantified and found to be very low and therefore considered to be at the background level for the ELISA method. The geometric mean titer (GMT) range for A/Wyoming strain was 27-169, for the A/New Caledonia strain the GMT range was 44-197 and for the B/Jiangsu strain the GMT range was 29-102. The following studies.were performed.

*Protocol Number 1134-04221.026:* This study was conducted to compare the immune response elicited by influenza vaccine, which was administered on intact and abraded skin. In this study, guinea pigs were primed by IM injection with a low dose of TIV (0.5 µg HA). Three weeks later these animals were shaved on the abdomen and the shaved area was hydrated with 10% glycerol/saline. Half of the animals received no skin treatment and half of the animals were treated with an ECG prep pad (15 strokes) to disrupt the SC. A dry patch formulated with 15 µg HA (5 µg per strain) alone or with 5 µg LT-adjuvant was applied to the shaved area as described in Methods. Two weeks after removal of the patches, serum was collected and antibody titers to each of the influenza strains was determine by the ELISA method. The results are depicted in Figure 5. The group receiving TIV alone patches (without adjuvant) applied to treated skin produced antibody titers to each of the flu strains that were 4.8- to 20-fold greater than the group receiving the same patch applied to untreated skin. Furthermore, the group receiving patches containing TIV and LT-adjuvant applied to treated skin generated antibody titers to each of the flu strains that were 26- to 91-fold greater than the group receiving the same patch applied to untreated skin.

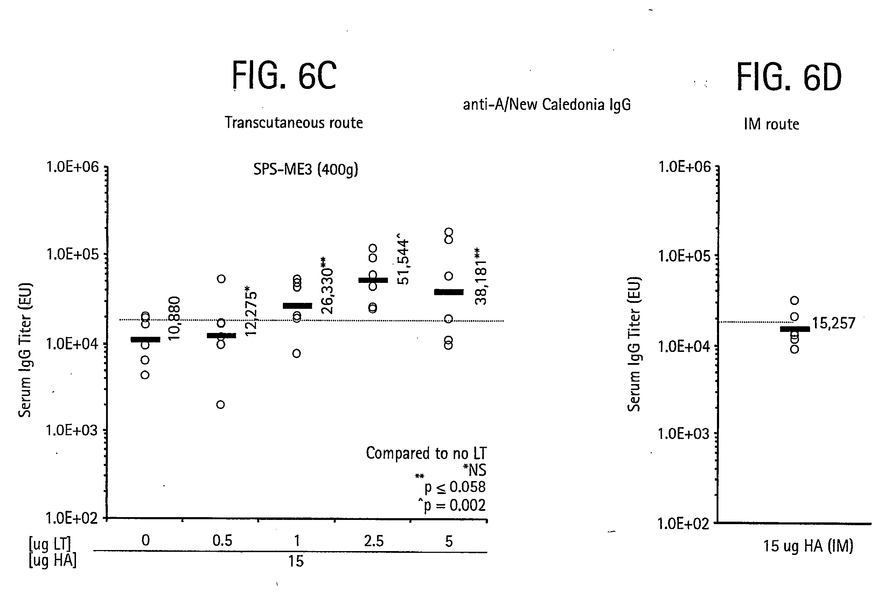

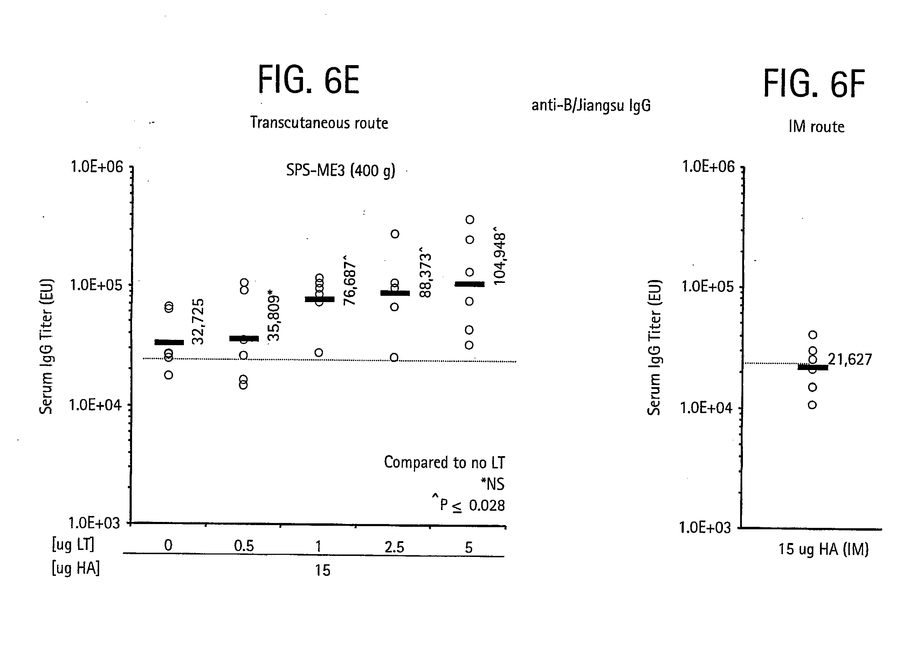

*Protocol Number 5235-052:* In this study, the effectiveness of SPS™-ME³ as a skin pretreatment method was examined. Groups of 6 animals received a layered patch with influenza (15 µg HA) sandwiched with a patch containing different amounts of LT (0.5 - 5 µg) or a placebo patch (no LT). The results depicted in Figure 6 show animals receiving the influenza patch, without the adjuvant (no LT), generated serum antibody titers to all strains that were equivalent to IM injection with the same HA dose. Furthermore, animals receiving an influenza vaccine patch sandwiched with an LT containing patch generated antibody titers that were greater than IM injected animals. The enhanced immune response was LT dose dependant and significant (p ≤ 0.036) at the 2.5 and 5 µg LT dose levels for all three strains. The immune response elicited by transcutaneous delivery of multivalent flu vaccine and LT-adjuvant is significantly improved using simple skin treatment, such as mild abrasion.

Following patch removal, local reactogenicity was evaluated using the four point scoring procedure described in Methods. The groups receiving patches containing LT-adjuvant developed mild to moderate erythema and edema that was limited to the site of patch application. In general, local inflammation was observed 1-2 days following patch removal and was self resolving within 8-13 days. In addition, reactogenicity was LT dose related with groups receiving low dose LT (0.5-1.0 µg) developed slight responses (Draise score ≤1.5), while groups receiving higher LT doses (2.5-5 µg) developed moderate local inflammation (Draise score = 2.5-3.5). TIV alone (no adjuvant) was not reactogenic in the guinea pig.

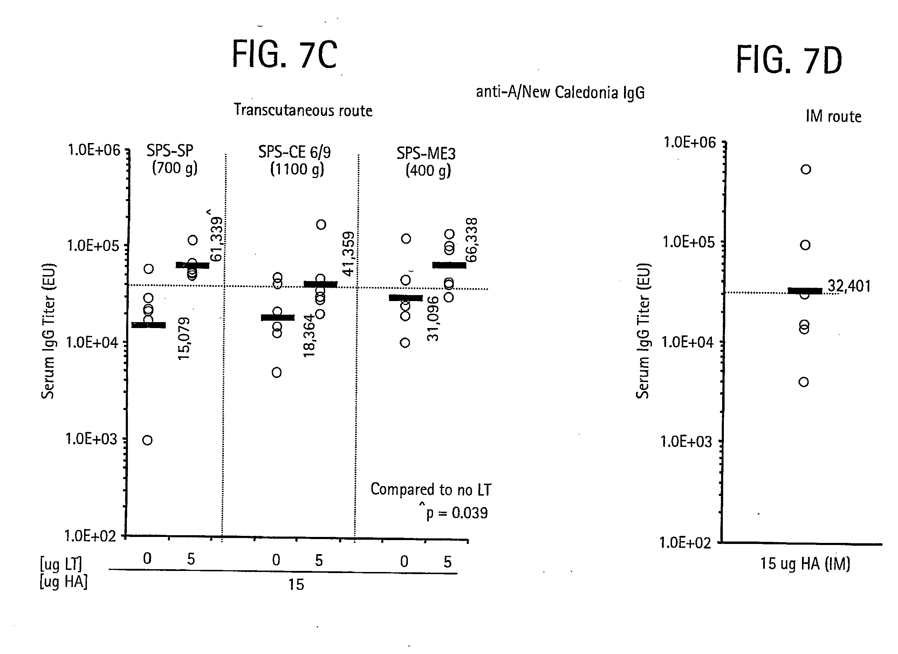

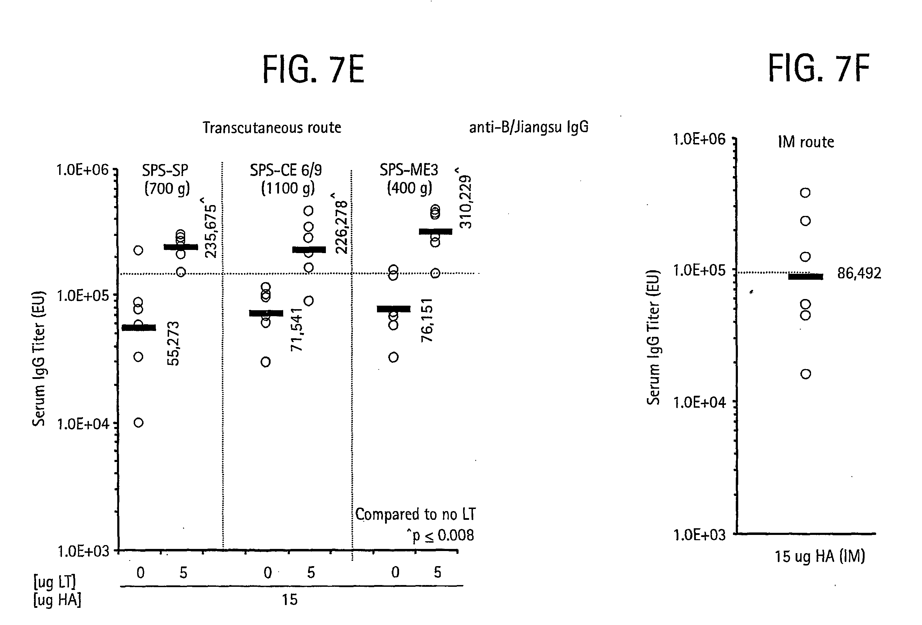

*Protocol Numbed 5235-054:* The next study compared the immune response elicited by influenza-LT blended patches applied to skin treated with three different designs of SPS™. In this study, groups of 6 guinea pigs previously primed with low dose flu vaccine (0.5 µg HA) were pretreated using the SPS™-SP device equipped with a 700 gram snap dome, the SPS™-CE 6/9 (250 µm ridges) equipped with a 1100 gram snap dome, or the SPS™-ME³ device equipped with a 400 gram snap dome. A patch containing 15 µg HA alone (no LT) or mixed with 5 µg LT was applied to the treated skin. A separate group was immunized by intramuscular injection with 0.5 µg HA (study day 1) and boosting with 15 µg HA by IM injection on study day 22. As seen in Figure 7, all animals receiving the LT-adjuvanted flu patch generated very high titer antibodies to the A/Wyoming (Figure 7A), A/New Caledonia (Figure 7C) and B/Jiangsu (Figure 7E) strains, regardless of the skin treatment used. The geometric mean titers were equal to or greater than the antibody titers produced by animals receiving the influenza vaccine administered by the IM route (Figure 7B, 7D, 7F).

Groups receiving influenza vaccine patches without LT also produced antibodies to each of the flu strains. In this case, however, the groups treated with the SPS™-SP and SPS™-CE 6/9 devices developed antibody titers that were lower than the IM immunized group. The group treated with the SPS™-ME³ device (1 stroke) and immunized with the non-adjuvanted flu patch, developed antibody titers to the two A-strains that were comparable to the IM immunized group. These results indicate that the three SPS™ were effective methods for treating skin prior to patch application. The immune responses to all flu strains were equal to or greater than the immune response elicited by the IM route, when 5 µg of LT was co-administered with the flu vaccine. Without the adjuvant, however, the immune responses were slightly lower than IM injection when the SPS™-SP and SPS™-CE 6/9 devices were used to treat the skin.

Local reactogenicity was compared between groups pretreated with SPS™-SP, SPS™-CE 6/9 and SPS™-ME³ devices and immunized with patches containing TIV alone or with 5 µg LT. The groups immunized with the TIV/LT patches and pretreated with the SPS™-SP device had slightly greater local edema (Draise score = 3.2) compared to the other treatment groups (results not shown). The groups pretreated with the SPS™-CE 6/9 and SPS™-ME³ devices developed mild to moderate edema and erythema (Draise score = 2.5-3). The inflammation peaked at 4-5 days after patch removal and resolved by about 8 days. The group receiving TIV without LT-adjuvant did not exhibit any local erythema or edema.

*Protocol Number 5235-058:* The design of the SPS™-CE was further refined and evaluated in this study. The treatment surface consisted of ridges that were 200 µm in height and arranged in two rows of 6 (SPS™-CE 2x6). The metal strip was mounted on a strip pull device that was equipped with a 700 gram snap dome. The effectiveness of the SPS™-CE 2x6 design was compared to skin treatment with the SPS™-ME³ device. Layered patches were used in this study. As depicted in Figure 7, all animals receiving the flu patch (no LT) generated high titer antibodies to A/Wyoming, A/New Caledonia and B/Jiangsu strains with both treatment devices. The geometric mean titers were equal to the antibody titers produced by animals receiving the influenza vaccine by the IM route. Groups receiving influenza patches layered with a LT patch also generated very high titers to all three strains (2-5 fold higher than the IM levels). The group treated with the SPS™-CE 2x6 device and received TIV+LT patches generated statistically higher titers than TIV alone. This was true for all strains. Local reactogenicity was observed only in the groups that received the LT-adjuvant patch. The magnitude and duration of the erythema and edema was similar between the two pretreatment groups (results not shown).

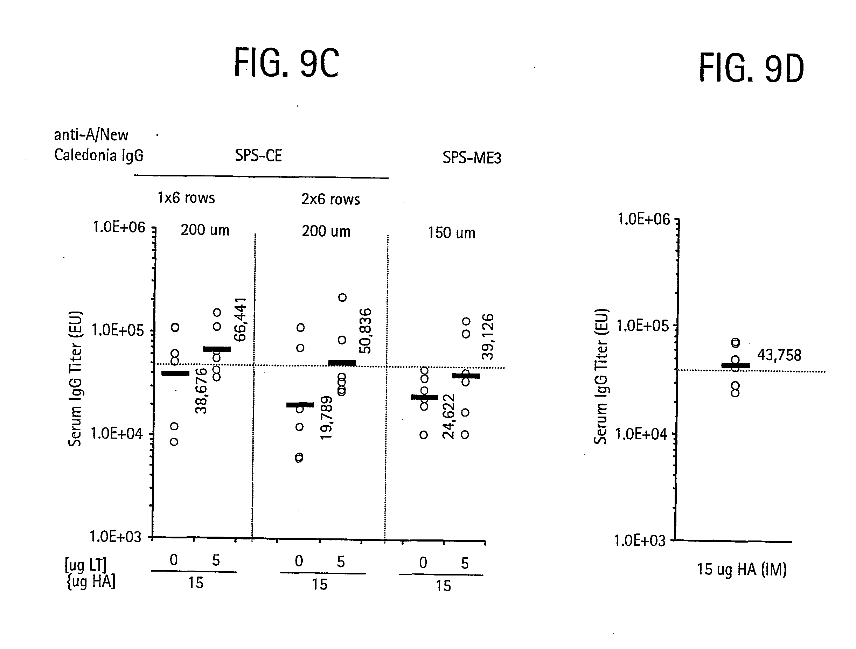

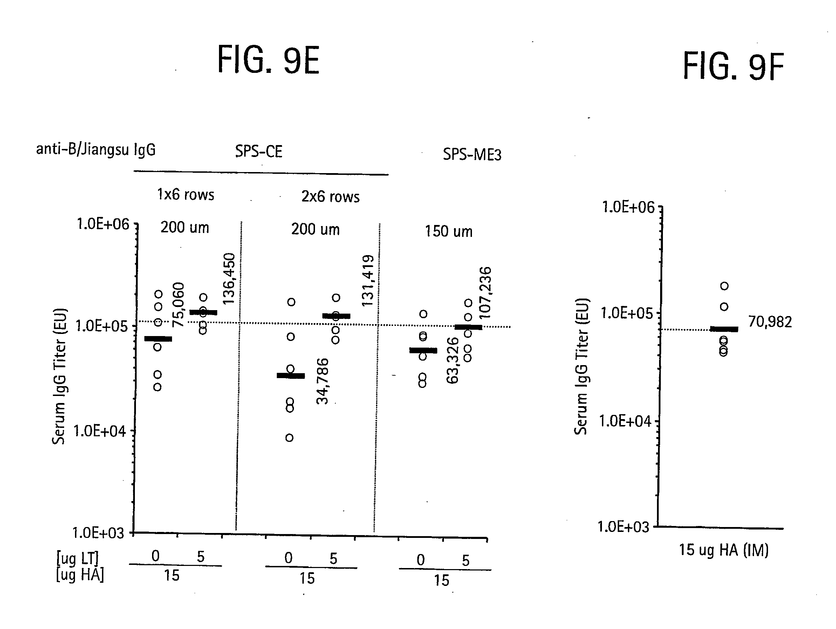

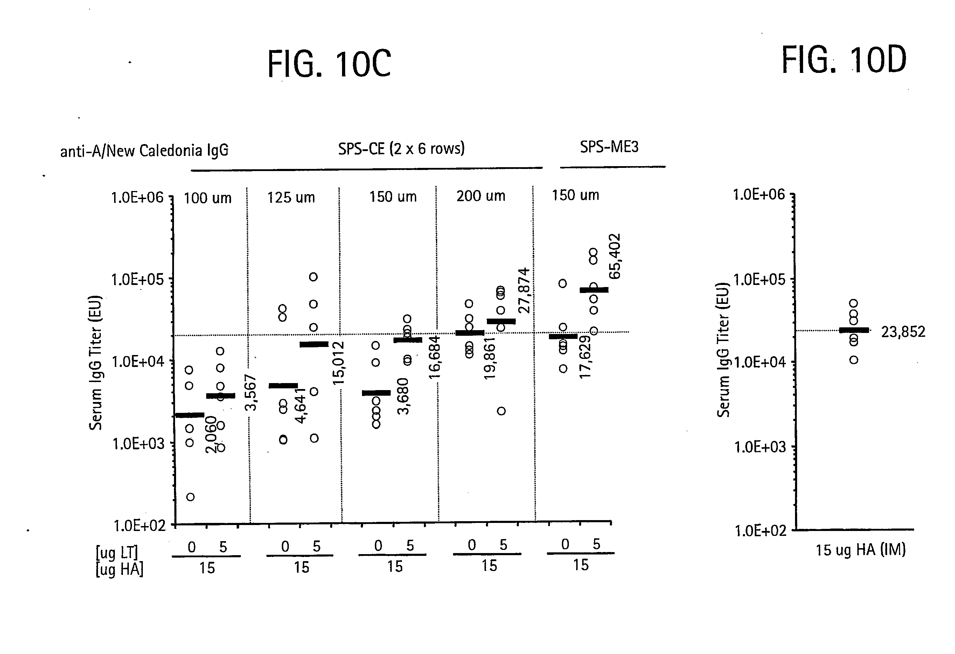

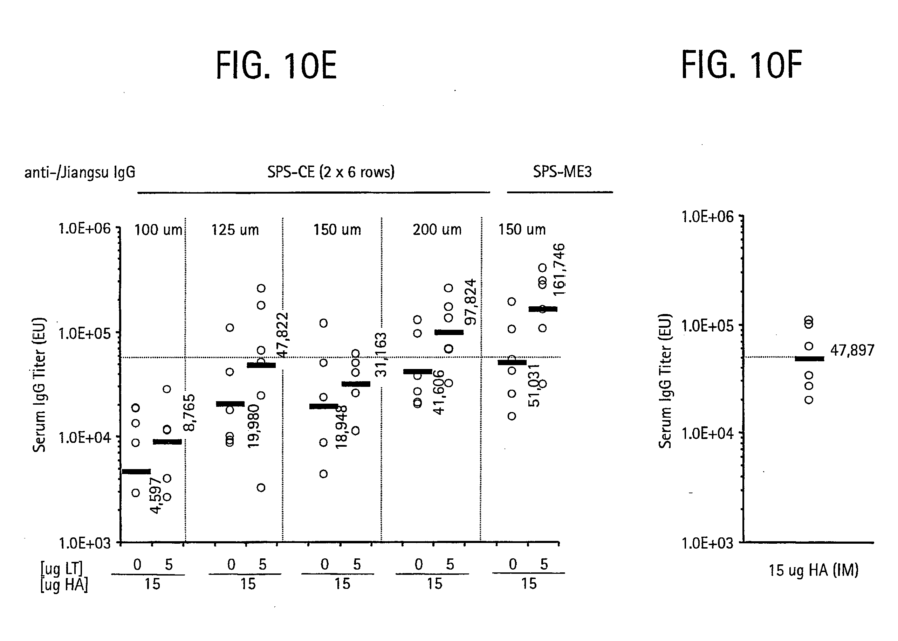

*Protocol Number 5235-057:* In this study, chemical etched surfaces were prepared with a single row of 6 ridges (SPS™-CE 1x6) and two rows of 6 ridges (SPS™-CE 2x6). The ridge heights were 200 µm. As seen in Figure 9, animals pretreated with SPS™-CE 1x6 produced serum antibody titers to each of the three influenza strains that were comparable to animals that had been pretreated with the SPS™-CE 2x6 design, indicating that these two designs were equally effective as skin pretreatments for delivery of flu vaccine. Consistent with other studies, the magnitude of the immune response was equal to IM injection when LT was co-administered with the vaccine. As described for other studies, mild to moderate local reactogenicity (Draise score = 2.0-3.0) was associated with application of the LT patch and absent from the group receiving the TIV patch without the adjuvant. The magnitude and course of the reactogenicity was similar between all pretreatment groups (results not shown). *Protocol Number 5235-068:* This study was conducted to investigate the effect of CE ridge height on the delivery and immune response to topically administered flu vaccine. In this study, chemical etched strips were produced with two rows of six ridges (SPS™-CE 2x6). Ridge heights were varied and included 100 µm, 125 µm, 150 µm and 200 µm height. A separate group was treated with SPS™-ME³ (150 µm edge). As described previously, a layered patch containing 15 µg HA alone or with a layered 5 µg LT patch was applied immediately following the skin treatment. Serum antibody titers to each of the flu strains were determined using serum collected two weeks after the removal of the patch. The results in Figure 10 show that ridge height is a significant factor in obtaining optimal delivery and immune response to flu vaccine. Animals pretreated with 100 µm, 120 µm, and 150 µm SPS™-CE 2x6 devices and receiving flu vaccine patches (without adjuvant) generated relatively low titer antibodies to A/Wyoming (GMT = 10,000 - 20,000), A/New Caledonia (GMT = 2,000 to 4,500) and B/Jiangsu (GMT = 5,000 - 19,000). In contrast, the group pretreated with the 200 µm SPS™-CE 2x6 device and receiving flu vaccine patches (without adjuvant) produced antibody titers that were 2.2 to 10-fold higher (A/Wyoming (GMT = 84,000), A/New Caledonia (GMT = 20,000) and B/Jiangsu (GMT = 42,000)). These results indicate that the height of the chemical etched ridge is an important factor in delivery of flu vaccine. Furthermore, animals pretreated with the 200 µm SPS™-CE 2x6 or the SPS™-ME³ device produced antibody titers that were equal to or greater than the IM injected group.

Consistent with the other studies reported here, local reactogenicity was observed in the groups immunized with the LT-adjuvant patch. The group pretreated with SPS™-CE 2x6 with 100 µm ridge height had slightly reduced local edema (Draise score = 2.5-3.0) compared to the groups pretreated with SPS™-CE 2x6 with 125 µm, 150 µm, and 200 µm ridge heights (Draise score = 3.0). The duration of erythema and edema were the same in all pretreated groups receiving the LT-adjuvant patch (results not shown).

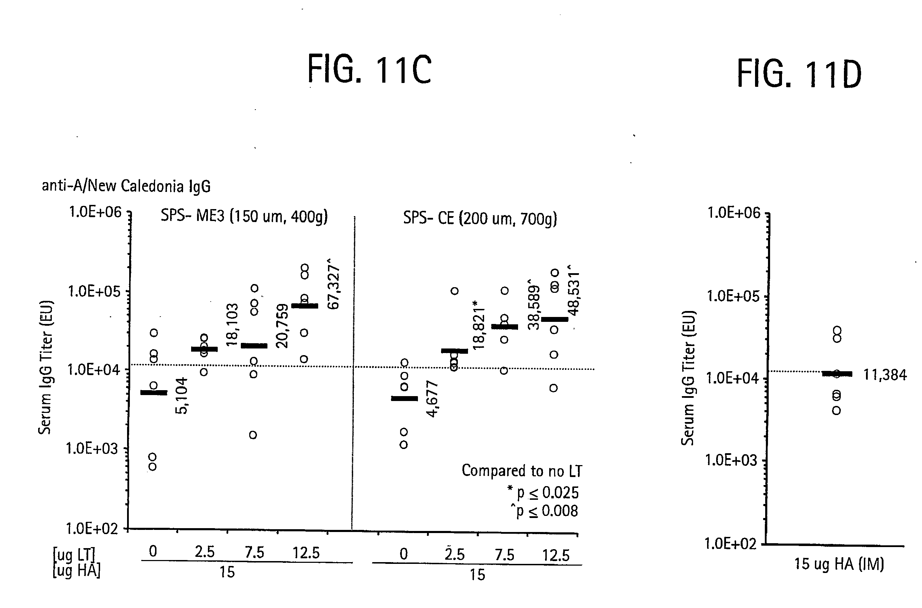

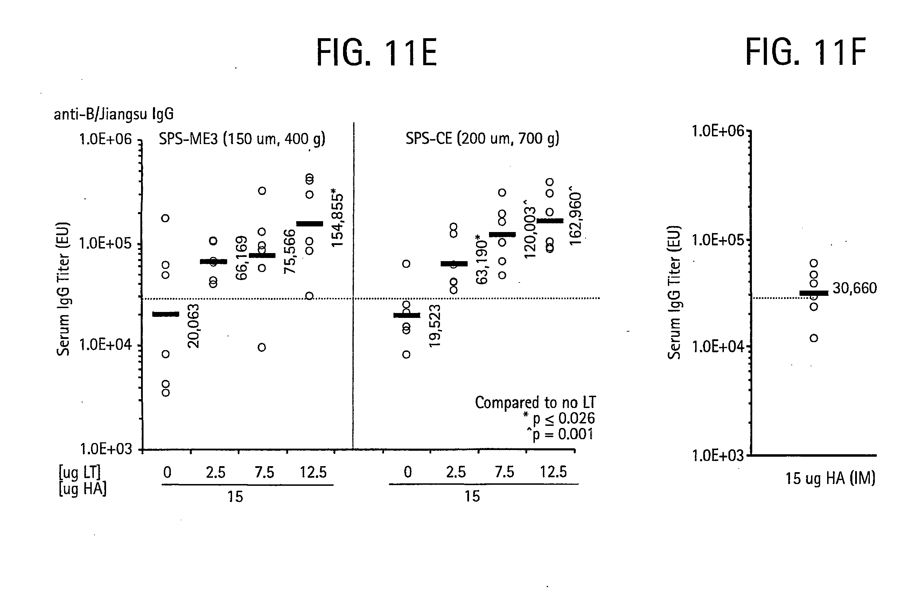

*Protocol Number 5235-059:* This study was conducted to evaluate the effect of increasing the LT-adjuvant dose upon the immune response to co-administered flu vaccine. In this study, groups of guinea pigs were treated on the shaved abdomen with either the SPS™-ME³ (150 µm) or SPS™-CE 2x6 (200 µm) devices. A 15 µg flu vaccine patch was applied without LT-adjuvant or with a layered adjuvant patch containing 2.5 µg, 7.5 µg, or 12.5 µg LT. Patches were applied and removed as already described. As seen in Figure 11, animals pretreated with SPS™-ME³ or SPS™-CE 2x6 and receiving 15 µg HA flu patches with 12.5 µg LT generated antibody titers that were superior to IM injection with the same dose of flu vaccine. The anti-A/Wyoming IgG titers were 2.4 to 3.5-fold greater in the topically immunized group compared to the IM injected group (Figure 11A and 11B). The anti-A/New Caledonia IgG titers were 4.3 to 6-fold greater in the topically immunized group compared to the IM injected group (Figure 11C and 11D) and the anti-B/Jiangsu IgG titers were 5-fold greater in the topically immunized group compared to the IM injected group (Figure 11E and 11F). Groups receiving lower doses of LT-adjuvant (2.5 to 7.5 µg) generated antibody titers to each strain that were equivalent to IM injection. The group immunized with flu vaccine alone patch (no LT) produced antibody titers that were lower than the IM injected group. These results indicate that the SPS™-CE 2x6 (200 µm) and SPS™-ME³ (150 µm) devices were effective treatments for in vivo delivery of inactivated TIV. Co-administering LT in a separate patch significantly augmented the immune response to each of the flu strains resulting in the generation of antibody titers that were significantly greater than IM injection with the same dose of flu vaccine. As observed in the other studies, local reactogenicity was observed only in the groups receiving LT-adjuvant. The groups receiving low dose LT (2.5 µg) had reduced local edema and erythema compared to the groups receiving higher amounts of LT (7.5 and 12.5 µg). The group receiving low dose LT and pretreated with SPS™-ME³ device exhibited little reactogenicity compared to the group pretreated with SPS™-CE device at the same dose. The duration of the reactogenicity was similar (8-11 days) regardless of the LT dose or pretreatment used.

### Conclusions

Previous studies have demonstrated that the stratum corneum (SC) is an effective barrier to transcutaneous delivery of flu antigens. In the studies reported here, the guinea pig was developed as a model to investigate new methods for disrupting the SC. The objective of these studies was to test the delivery of flu vaccine from a dry patch applied to skin which had been treated with different SPS™. The SPS™ devices were designed to provide uniform disruption of the SC, to be simple to administer, for single use, and to be disposable. Two designs were investigated in the model. The SPS™-ME3 design was a plastic block with microblades protruding 150 µm from the surface. The second design was a "strip-pull" device equipped with an abrasive (SPS™-SP, 180 grit sandpaper) or various CE metal surfaces (SPS™-CE). The CE surfaces were designed with different number and different heights (100 µm to 250 µm) of ridges on the treatment surface. Serum antibodies to the influenza strains was used to judge *in vivo* delivery of flu. These studies demonstrate that SPS™-ME3, SPS™-SP and SPS™-CE were all effective methods for pretreating skin. In general, antibody titers to influenza antigens were comparable or superior to IM injection when skin was sufficiently pretreated. With regard to SPS™-CE pretreatment, it was observed that the height of the chemically etched ridges was an important factor for delivery of flu vaccine. Animals pretreated with SPS™-CE with ridge heights 100 µm to 150 µm generated lower titer antibodies to flu compared to animals pretreated with SPS™-CE with a ridge heights of 200 µm. In addition, co-administration of LT-adjuvant with TIV potentiated the immune responses to flu antigens and the antibody titers were typically greater than (2.4 to 6-fold) IM injection. Mild to moderate local reactogenicity was observed only in groups that were immunized with the LT-adjuvant, whether delivered in a blended formulation or co-administered with a layered patch. The magnitude of the the inflammatory response did appear to be LT dose dependant. Typically, local inflammation was observed 1-2 days following patch removal and was self resolving within 8-13 days. The reactogenicity profiles were similar between the different skin pretreatment devices evaluated in these studies.

### Example 2. Strip Pull Device

In this example, an area of guinea pig skin was shaved and various methods of disruption were administered - hydration, tape stripping, and abrasion with strip pull. The strip had dimensions of 1 inch (25.4 mm) in width by 2.25 inches (57.1 mm) in length. For the strip pull device, the abrasive material was 3M 431Q Tri-M-Ite sandpaper, and grit ranging from 120-180 was employed. The abrasive was pulled across the guinea pig skin with at least 700 grams of pressure constantly applied. Patches containing different amounts of LT adjuvant (0.15 µg and 15 µg, respectively) were applied to different treatment groups, and serum IgG anti-LT titer levels were observed after 21 days using ELISA. The data in Table 1 show that the geometric mean titer levels were significantly greater using the strip pull device than the titer levels for hydration or tape stripping at equivalent LT doses.

| **Table 1- Strip Pull LT Geometric Mean Titer** | | | | | | |
|---|---|---|---|---|---|---|
| | Hydration only | | Tape stripping | | Strip Pull | |
| LT amount | 0.15 | 15 | 0.15 | 15 | 0.15 | 15 |
| LT titer | 128 | 1,701 | 590 | 21,864 | 13,922 | 154,532 |

### Example 3. Site and Spin Device

In this example, guinea pigs were primed with HA trivalent flu antigen, then after 21 days, an area of guinea pig skin was shaved and barrier disruption methods including hydration, abrasion with GE Medical ECG non-woven abrasive pad, and abrasion with a site and spin device of the invention were employed. The abrasive employed in the site and spin device was 3M 431 Q Tri-M-Ite 180 grit sandpaper, and it was rotated 9 times on the guinea pig skin in less than 1 second. Operators were instructed to apply firm pressure of approximately 500 grams; force was not mechanically controlled in this example, although it could be controlled in other embodiments of the invention. A patch containing LT adjuvant (0 or 5 µg ) and trivalent flu antigen (HA, 15 µg) was then applied overnight, and serum IgG titer levels observed 21 days later using ELISA. The data in Table 2 show that the geometric mean titer of serum IgG against all three flu strains was greater using the site and spin device than when using hydration alone. The barrier disruption was also accomplished much more rapidly using the site and spin device than the ECG pad, which required 15 repeated strokes. The site and spin device also increased the TEWL compared to hydration and ECG.

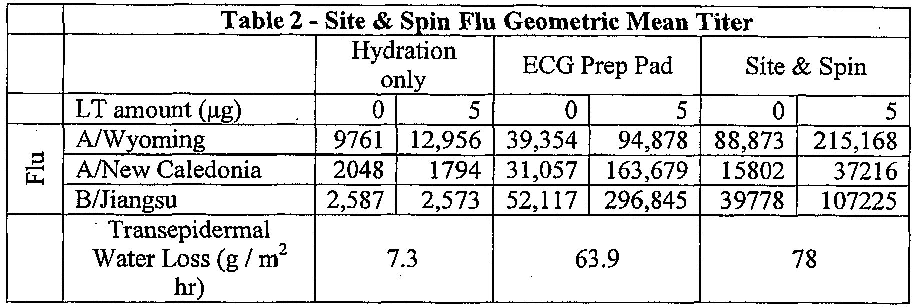

### Example 4. Mask & Abrade Device

In this example, an area of guinea pig skin was shaved, and hydration and the mask & abrade invention were used for skin barrier disruption. The device utilized snap dome feedback to ensure a force of at least 700 grams of pressure was constantly applied. The abrasive material was 3M 431 Q Tri-M-Ite sandpaper, with grit ranging from 150-180. Transepidermal water loss (TEWL (g/m²hr)) was measured to determine the degree of skin barrier disruption. Results in Table 3 show that the mask & abrade invention was effective in disrupting the skin when compared to hydration.

| **Table 3 - Mask & Abrade TEWL data** | | | | | |
|---|---|---|---|---|---|
| | **Basal** | **2x** | **4x** | **5x** | **6x** |
| **Hydration** | 7.8 | - | - | - | - |
| **180 grit** | 7.6 | 24.0 | 36.6 | 55.7 | 62.4 |
| **150 grit** | 5.7 | 22.5 | 51.3 | 52.2 | 59.0 |

### Example 5. Use of In-vivo Confocal Microscopy to Evaluate Effects of Skin Disruption

In this example, an in-vivo confocal reflectance microscope (Lucid Vivascope 1500) was used to characterize the skin disruption created by various photochemically etched surfaces mounted onto the strip pull device (SPS-CE, 700g snap dome). Chemically etched surfaces consisted of parallel microprotrusions arranged in two rows of 6 protrusions. The protrusions were of uniform height within each lot, and varied across lots from a height of 202 µm to a height of 272 µm. The skin disruption profile was characterized by visualizing the parallel grooves created in the skin and measuring the average depth and width of the grooves using the confocal microscope (see Table below). Skin disruption was measured in 5 human subjects using this method, and the average groove depths and widths are displayed. This technique allows the user to differentiate between the effectiveness of various treatments, including the skin disruption created by the SPS-CE.

| | 252 µm | 272 µm | 252 µm | 202 µm |
|---|---|---|---|---|
| Depth (µm) | 32.9 | 24.7 | 25.0 | 15.0 |
| Width (µm) | 12.2 | 15.8 | 10.4 | 8.7 |

### Example 6. Ink Marking for Patch/Vehicle Registration

Treatment of the skin to for vaccine or drug delivery may be performed on a circumscribed site on the skin. In order to place the active drug substance over the disrupted skin (registration), a system of skin marking may be employed. In one embodiment, an SPS™ system containing an inking system can be employed. As shown in the example below, the ink can be incorporated into the masking system, so that application of the mask leaves an ink mark over which the patch is placed.

Many systems may be employed for marking the skin.

### Example 7. Transdermal Drug Delivery.

A device of the invention, either a mask and abrade, strip pull, or site and spin can also be employed to disrupt layers of the skin prior to, during, or after application of a drug to the skin. The drug is then delivered transdermally.

Topical delivery of lidocaine currently is achieved using a cream over a long period of time. Other complex devices such as lasers, ultrasound or microporation have been employed. In an embodiment of the current invention, a device of the present invention, such as the mask and abrade system, can be employed prior to placement of lidocaine in a pharmaceutically acceptable carrier on the skin. The improved delivery can be quantified by known techniques for detecting anesthesia of a given area.

In an alternative embodiment of the invention, NSAIDs have proven to be very difficult to deliver topically. Here a solution or formulated patch can be placed over the site of skin treated with a device of the invention, such as the mask and abrade system. Enhanced delivery can be measured in a pK study, sampling sera to show enhanced delivery using skin disruption.

Enhanced delivery using the current invention may lead to self-administration at various anatomical sites on the body. For example, a strip pull with a formulated NSAID patch may be placed by the patient. The strip pull motion may result in skin disruption and patch placement in one motion.

### Example 8. Use of the Mask and Abrade with the Immune Stimulant Patch

In this example (Protocol Number: B5235-061), the mask and abrade device is used with the immune stimulant patch. The mask and abrade device is used to enhance skin penetration.

*Immunization technique:* Female Dunkin Hartley guinea pigs, weighing 350-400 g, were shaved at the abdomen or thigh muscle, pretreated and immunized with A/New Caledonia (A/NC) injected intradermally (ID) or intramuscularly (IM). The skin was pretreated five times using 180 grit sandpaper (Tegaderm overlay 3M) mounted on a force controlled, hand-held buffer (Mask and Abrade (M&A) vSLA 105,2 cm² mask 700g force). Following skin pretreatment 1 µg or 5 µg A/New Caledonia were injected ID and IM respectively. 1 cm² dry patches containing 1, 5, or 10 µg LT were placed over the injection site for approximately 24 hours. Immunizations were performed on day 1 and 22. The prime and boost were done on alternate sites. Guinea pigs received patch either at first immunization or at the second immunization or at both immunizations. Serum was collected two weeks after the second immunization and hemagglutination inhibition titers (HAI) and IgG titers were determined by ELISA and HAI assay.

*Antibody Assays:* Serum antibody titers to A/New Caledonia were determined by an ELISA method, as previously described. Serum antibody titers are reported as the serum dilution equal to 1 OD at 405 nm. The geometric mean titer for each group is indicated. Statistical analysis generated by two tailed T-test on the titers in log10 and considered significant at p=0.05.

### Results:

The results are shown in the Tables 3-5 below.

**Table 3. Serum IgG to A/New Caledonia titers following one immunization**

| **Gps** | **First Immunization** | | **Second Immunization** | | **Pretreatment only with patch** | **Geo Mean (EU)** | **P value** |
|---|---|---|---|---|---|---|---|
| | **A/NC (ug)** | **LT-IS patch** | **A/NC (ug)** | **LT-IS patch** | | | vs shave only |
| 1 | **1 ug ID** | none | **1 ug ID** | none | Shave only | **700** | |
| 2 | **1 ug ID** | none | **1 ug ID** | 1 ug LT | 5x M&A | **1302** | 0.1997 |
| 3 | **1 ug ID** | 1 ug LT | **1 ug ID** | none | 5x M&A | **16993** | 0.0001 |
| 4 | **1 ug ID** | 1 ug LT | **1 ug ID** | 1 ug LT | 5x M&A | **8076** | 0.0001 |
| 5 | **1 ug ID** | none | **1 ug ID** | 5ug LT | 5x M&A | **1042** | 0.2937 |
| 6 | **1 ug ID** | 5ug LT | **1 ug ID** | none | 5x M&A | **10235** | 0 |
| 7 | **1 ug ID** | 5ug LT | **1 ug ID** | 5ug LT | 5x M&A | **8147** | 0 |
| 8 | **1 ug ID** | none | **1 ug ID** | 10 ug LT | 5x M&A | **1022** | 0.3281 |
| 9 | **1 ug ID** | 10ug LT | **1 ug ID** | none | 5x M&A | **9094** | 0.0002 |
| 10 | **1 ug ID** | 10ug LT | **1 ug ID** | 10ug LT | 5x M&A | **12293** | 0 |
| 11 | **5 ug IM** | none | **5 ug IM** | none | Shave only | **3774** | |
| 12 | **5 ug IM** | none | **5 ug IM** | 1 ug LT | 5x M&A | **3223** | 0.7649 |
| 13 | **5 ug IM** | 1 ug LT | **5 ug IM** | none | 5x M&A | **4254** | 0.8338 |
| 14 | **5 ug IM** | 1 ug LT | **5 ug IM** | 1 ug LT | 5x M&A | **7605** | 0.2852 |
| 15 | **5 ug IM** | none | **5 ug IM** | 5 ug LT | 5x M&A | **1767** | 0.1671 |
| 16 | **5 ug IM** | 5ug LT | **5 ug IM** | none | 5x M&A | **15347** | 0.0361 |
| 17 | **5 ug IM** | 5ug LT | **5 ug IM** | 5ug LT | 5x M&A | **8856** | 0.0066 |
| 18 | **5 ug IM** | none | **5 ug IM** | 10 ug LT | 5x M&A | **3026** | 0.726 |
| 19 | **5 ug IM** | 10ug LT | **5 ug IM** | none | 5x M&A | **12423** | 0.0215 |
| 20 | **5 ug IM** | 10ug LT | **5 ug IM** | 10ug LT | 5x M&A | **26081** | 0.0023 |

**Table 4. Serum IgG titers to A/New Caledonia following two immunizations with ID injection and IS patch**

| **Gps** | **First Immunization** | | **Second Immunization** | | **Pretreatment only with patch** | **Geo Mean** | **P value** |
|---|---|---|---|---|---|---|---|
| | **A/NC (ug)** | **LT-IS patch** | **A/NC (ug)** | **LT-IS patch** | | **(EU)** | vs shave only |
| 1 | **1 ug ID** | none | **1 ug ID** | none | Shave only | **13755** | |
| 2 | **1 ug ID** | none | **1 ug ID** | 1 ug LT | 5x M&A | **98377** | 0.0018 |
| 3 | **1 ug ID** | 1ug LT | **1 ug ID** | none | 5x M&A | **256922** | 0.0004 |
| 4 | **1 ug ID** | 1ugLT | **1 ug ID** | 1ug LT | 5x M&A | **297479** | 0.0001 |
| 5 | **1 ug ID** | none | **1 ug ID** | 5ug LT | 5x M&A | **168127** | 0.0007 |
| 6 | **1 ug ID** | 5ug LT | **1 ug ID** | none | 5x M&A | **207994** | 0.0003 |
| 7 | **1 ug ID** | 5ug LT | **1 ug ID** | 5ug LT | 5x M&A | **514504** | 0.0083 |
| 8 | **1 ug ID** | none | **1 ug ID** | 10 ug LT | 5x M&A | **189483** | 0.0013 |
| 9 | **1 ug ID** | 10ug LT | **1 ug ID** | none | 5x M&A | **91627** | 0.0042 |
| 10 | **1 ug ID** | 10ug LT | **1 ug ID** | 10ug LT | 5x M&A | **289846** | 0.0001 |
| 11 | **5 ug IM** | none | **5 ug IM** | none | Shave only | **111726** | |
| 12 | **5 ug IM** | none | **5 ug IM** | 1 ug LT | 5x M&A | **158908** | 0.3155 |
| 13 | **5 ug IM** | 1 ug LT | **5 ug IM** | none | 5x M&A | **166448** | 0.5489 |
| 14 | **5 ug IM** | 1 ug LT | **5 ug IM** | 1 ug LT | 5x M&A | **385046** | 0.0069 |
| 15 | **5 ug IM** | none | **5 ug IM** | 5ug LT | 5x M&A | **200284** | 0.1624 |
| 16 | **5 ug IM** | 5ug LT | **5 ug IM** | none | 5x M&A | **398611** | 0.0067 |
| 17 | **5 ug IM** | 5ug LT | **5 ug IM** | 5ug LT | 5x M&A | **1234754** | 0.0014 |
| 18 | **5 ug IM** | none | **5 ug IM** | 10ug LT | 5x M&A | **517207** | 0.0039 |
| 19 | **5 ug IM** | 10ug LT | **5 ug IM** | none | 5x M&A | **307290** | 0.0203 |
| 20 | **5 ug IM** | 10ug LT | **5 ug IM** | 10ug LT | 5x M&A | **670532** | 0.0001 |

**Table 5. Enhanced HAI titers in guinea pigs immunized with ID injection and IS-patch**

| | **1st Immunization** | | **2nd Immunization** | | | **Geomean Titer of NT Ab 2 wks post** |
|---|---|---|---|---|---|---|
| **Gr** | **ID inject. HA (µg)** | **IS patch LT(µg)** | **ID inject. HA (µg)** | **IS patch LT(µg)** | **Pretreat** | |
| 1 | 1ug | none | 1µg | none | Shave only | < 15 |
| 2 | 1µg | none | 1µg | 1µg | 5x M & A | < 24 |
| 3 | 1µg | 1µg | 1µg | none | 5x M & A | 50 |
| 4 | 1µg | 1µg | 1µg | 1µg | 5x M & A | 86 |
| 5 | 1µg | none | 1µg | 5µg | 5x M & A | 46 |
| 6 | 1µg | 5µg | 1µg | none | 5x M & A | 48 |
| 7 | 1µg | 5µg | 1µg | 5µg | 5x M & A | 121 |
| 8 | 1µg | none | 1µg | 10µg | 5x M & A | 41 |
| 9 | 1µg | 10µg | 1µg | none | 5x M & A | 48 |
| 10 | 1µg | 10µg | 1µg | 10µg | 5x M & A | 254 |

**Table 6. Enhanced HAI titers in guinea pigs immunized with IM injection and IS-patch**

| | **1st Immunization** | | **2nd Immunization** | | | **Geomean Titer of NT Ab 2 wks post** |
|---|---|---|---|---|---|---|
| **Gr** | **IM inject. HA (µg)** | **IS patch LT(µg)** | **IM inject. HA (µg)** | **IS patch LT(µg)** | **Pretreat** | |
| 11 | 5 µg | none | 5 µg | none | Shave only | 63 |
| 12 | 5 µg | none | 5 µg | 1 µg | 5x M&A | 86 |
| 13 | 5 µg | 1 µg | 5 µg | none | 5x M&A | 45 |
| 14 | 5 µg | 1 µg | 5 µg | 1 µg | 5x M&A | 121 |
| 15 | 5 µg | none | 5 µg | 5 µg | 5x M&A | 101 |
| 16 | 5 µg | 5 µg | 5 µg | none | 5x M&A | 220 |
| 17 | 5 µg | 5 µg | 5 µg | 5 µg | 5x M&A | 297 |
| 18 | 5 µg | none | 5 µg | 10 µg | 5x M&A | 231 |
| 19 | 5 µg | 10 µg | 5 µg | none | 5x M&A | 90 |
| 20 | 5 µg | 10 µg | 5 µg | 10 µg | 5x M&A | 806 |

### Conclusions:

The LT immunostimulant patch (IS patch) applied with the mask and abrade device was effective at delivering LT resulting in significantly enhanced responses to the injected influenza vaccine. Measurement of the immune response by ELISA (IgG) and HAI (functional) indicates the adjuvant effect. The enhancement can be seen in a single injection in conjunction with an IS patch or with two immunizations. Table 5 shows enhanced HAI titers in guinea pigs immunized with ID injection and IS-patch. Table 6 shows enhanced HAI titers in guinea pigs immunized with IM injection and IS-patch.

### Example 9. Use of Various Pretreatment Surfaces with Transcutaneous Immunization

### Materials:

- 78 female Hartley guinea pigs 350-400g, >7 weeks old at start of study from Charles River, received on 14 March, 2006, IM primed with 0.5 ug TIV on day 0
- Trivalent Influenza Vaccine (TIV): Solvay, A/Wyoming/03/2003 (H3N2), 284 µgHA/ml; A/New Caledonia/20/99 (H1N1), 237ngHA/ml; B/Jingsu/10/2003, 542ugHA/ml. TIV concentrated at 3.8mgHA/ml
- LT (iLT)
- 1 cm² rayon patch, formulated in dried sucrose containing 15 µg HA (5 µg per strain)
- 1 cm² rayon patch, formulated in dried sucrose containing 5ug iLT
- Strip Pull v4 device: 2" 180g (grit) sandpaper
- SPS™-ME³ 006" and 004" 400g snap dome
- Chemical Etch Strip Pull:
- Tegaderm 1624W: 3M, 6cm x 7cm size
- Adhesive tape: 3M

*Immunizations:* Before immunization and shaving, guinea pigs are sedated with ketamine and xylazine by standard procedure.

*Primping immunization:* All animals were primed on study day 1 with 0.5 µg trivalent influenza vaccine (0.167 µg HA each strain) by bolus intramuscular injection into the thigh muscle.

### Booster immunization,:

*Pretreatment:* Guinea pigs were shaved on the abdomen immediately before patch application on study day 22. The immunization site were marked with a permanent marker and the shaven skin were pretreated as follows: Groups 1-12: were pretreated as shown in the table of study design; no additional hydration. TEWL: measured for Groups 1-12 right after pretreatment

### Dosing:

1cm² dry sucrose formulated layered patches were applied to immediately following treatment. Groups were dosed as follows:
Groups 1, 3, 5, 7, 9, 11 received patches loaded with 15 µg HA and overlaid with a 1cm² dry placebo patch placed directly over top the 1^{st} patch.
Groups 2, 4, 6, 8, 10, 12 received patches loaded with 15 µg HA and overlaid with a 1 cm² dry 5ug iLT patch placed directly over top the 1^{st} patch.
Group 13 was immunized by intramuscular (IM) injection with 15 µg HA on study day 22.

*Patch application:* To insure proper patch adherence, patches were covered with a modified Tegaderm overlay. Animals were wrapped with adhesive tape. Patches were applied for 18-24 hr, removed, and the skin rinsed with warm water.

### Results:

The results are shown in Tables 7A and 7B below.

**Table 7A. Serum anti-FluA/W and FluB/JS IgG titers in guinea pigs**

| **Immunization dates (0, 3 wk): 1** **Serum collection date:** 1) (preb) 2) (wk3) 3) (wk5) | | | | | | | | | |
|---|---|---|---|---|---|---|---|---|---|
| **Gp** | **IM prime (ugHA)** | **TCI boost (1cm2 dry layered rayon patch)** | | | | **Test** | **Geometric Mean (EU)** | **T-Test P, log** | **T-Test P, log** |
| | | **Pretrx method** | **Materials** | **HA (ug)** | **iLT (ug)** | | | | |
| 1 | 0.5 | SPS™-ME³, 1x | 0.004", 400g SD* | 15 | 0 | **Flu A/W** | **42901** | 0.712 | |
| 2 | 0.5 | SPS™-ME³, 1x | 0.004", 400g SD | 15 | 5 | **Flu A/W** | **52409** | 0.961 | 0.661 |
| 3 | 0.5 | SPS™-ME³, 1x | 0.006", 400g SD | 15 | 0 | **Flu A/W** | **28435** | 0.283 | |
| 4 | 0.5 | SPS™-ME³, 1x | 0.006", 400g SD | 15 | 5 | **Flu A/W** | **170875** | 0.091 | **0.002** |
| 5 | 0.5 | SPS™-ME³, 1x | 0.006", 400g SD | 15 | 0 | **Flu A/W** | **69797** | 0.643 | |
| 6 | 0.5 | SPS™-ME³, 1x | 0.006", 400g SD | 15 | 5 | **Flu A/W** | **160850** | 0.133 | 0.097 |
| 7 | 0.5 | SPS™-SP | 2" 180grit, 700g | 15 | 0 | **Flu A/W** | **57825** | 0.911 | |
| 8 | 0.5 | SPS™-SP | 2" 180grit, 700g | 15 | 5 | **Flu A/W** | **109177** | 0.267 | 0.180 |
| 9 | 0.5 | SPS™-CE (200um 6/6) | 700g SD | 15 | 0 | **Flu A/W** | **50663** | 0.915 | |
| 10 | 0.5 | SPS™-CE (200um 6/6) | 700g SD | 15 | 5 | **Flu A/W** | **179889** | 0.051 | **0.003** |
| 11** | 0.5 | Arc 200um 3rpts, CURVED | | 15 | 0 | **Flu A/W** | **44245** | 0.731 | |
| 13 | 0.5 | IM injection | None | 15 | -- | **Flu A/W** | **53991** | # | |
| | | | | | | | | | |
| 1 | 0.5 | SPS™-ME³, 1x | 0.004", 400g SD | 15 | 0 | **Flu B/J** | **6885** | 0.114 | |
| 2 | 0.5 | SPS™-ME³, 1x | 0.004", 400g SD | 15 | 5 | **Flu B/J** | **30123** | 0.913 | 0.138 |
| 3 | 0.5 | SPS™-ME³, 1x | 0.006", 400g SD | 15 | 0 | **Flu B/J** | **18768** | 0.376 | |
| 4 | 0.5 | SPS™-ME³, 1x | 0.006", 400g SD | 15 | 5 | **Flu B/J** | **85702** | 0.057 | **0.004** |
| 5 | 0.5 | SPS™-ME³, 1x | 0.006", 400g SD | 15 | 0 | **Flu B/J** | **37688** | 0.607 | |
| 6 | 0.5 | SPS™-ME³, 1x | 0.006", 400g SD | 15 | 5 | **Flu B/J** | **85733** | 0.114 | 0.251 |
| 7 | 0.5 | SPS™-SP | 2" 180grit, 700g SD | 15 | 0 | **Flu B/J** | **44508** | 0.382 | |
| 8 | 0.5 | SPS™-SP | 2" 180grit, 700g SD | 15 | 5 | **Flu B/J** | **61395** | 0.110 | 0.462 |
| 9 | 0.5 | SPS™-CE (200um 6/6) | 700g SD | 15 | 0 | **Flu B/J** | **54035** | 0.180 | |
| 10 | 0.5 | SPS™-CE (200um 6/6) | 700g SD | 15 | 5 | **Flu B/J** | **156084** | 0.002 | **0.008** |
| 11** | 0.5 | Arc 200um 3rpts, CURVED | | 15 | 0 | **Flu B/J** | **19887** | 0.438 | |
| 13 | 0.5 | IM injection | None | 15 | -- | **Flu B/J** | **27907** | # | |

| | | | | | | | | | |
|---|---|---|---|---|---|---|---|---|---|
| *SD: Snap dome **Group 11 used a chemically etched surface of 200 µm nominal height, mounted on a puck. The bottom of the puck was curved, and the chemically etched surface was adhered to the puck so that the entire surface had a slight curve longitudinally along it. This curve caused the edges of the microprotrusions to contact the skin first, rather than the tops of the chemically etched microprotrusions. The force applied was approximately 700g, but was operator controlled, not controlled by a snap dome. | | | | | | | | | |

**Table 7B. Serum anti-FluA/NC and LT IgG titers in guinea pigs**

| Immunization dates (0, 3 wk): 1 Serum collection date: 1) (preb) 2) (wk3) 3) (wk5) | | | | | | | | | |
|---|---|---|---|---|---|---|---|---|---|
| **Gp** | **IM prime (ug HA)** | **TCI boost (1cm2 dry layered rayon patch)** | | | | **Test** | **Geometric Mean (EU)** | **T-Test P, log** | **T-Test P, log** |
| | | **Pretrx method** | **Materials** | **HA (ug)** | **iLT (ug)** | | | | |
| 1 | 0.5 | SPS™-ME³, 1x | 0.004", 400g SD | 15 | 0 | **Flu A/NC** | **2946** | 0.170 | |
| 2 | 0.5 | SPS™-ME³, 1x | 0.004", 400g SD | 15 | 5 | **Flu A/NC** | **5676** | 0.374 | 0.505 |
| 3 | 0.5 | SPS™-ME³, 1x | 0.006", 400g SD | 15 | 0 | **Flu A/NC** | **3760** | 0.161 | |
| 4 | 0.5 | SPS™-ME³, 1x | 0.006", 400g SD | 15 | 5 | **Flu A/NC** | **21102** | 0.653 | **0.039** |
| 5 | 0.5 | SPS™-ME³, 1x | 0.006", 400g SD | 15 | 0 | **Flu A/NC** | **15149** | 0.899 | |
| 6 | 0.5 | SPS™-ME³, 1x | 0.006", 400g SD | 15 | 5 | **Flu A/NC** | **27506** | 0.460 | 0.338 |
| 7 | 0.5 | SPS™-SP | 2" 180grit, 700g | 15 | 0 | **Flu A/NC** | **12290** | 0.898 | |
| 8 | 0.5 | SPS™-SP | 2" 180grit, 700g | 15 | 5 | **Flu A/NC** | **16946** | 0.803 | 0.564 |
| 9 | 0.5 | SPS™-CE (200um 6/6) | Arc **SP-BLT2E,** 700g SD | 15 | 0 | **Flu A/NC** | **17202** | 0.794 | |
| 10 | 0.5 | SPS™-CE (200um 6/6) | Arc **SP-BLT2E,** 700g SD | 15 | 5 | **Flu A/NC** | **56123** | 0.101 | **0.044** |
| 11 | 0.5 | Arc 200um 3rpts, CURVED | **CPP-BLT2D** | 15 | 0 | **Flu A/NC** | **6731** | 0.404 | |
| 13 | 0.5 | IM injection | None | 15 | | **Flu A/NC** | **13688** | # | |
| | | | | | | | | | |
| 1 | 0.5 | SPS™-ME³, 1x | 0.004", 400g SD | 15 | 0 | **LT** | **38** | | |
| 2 | 0.5 | SPS™-ME³, 1x | 0.004", 400g SD | 15 | 5 | **LT** | **314** | | |
| 3 | 0.5 | SPS™-ME³, 1x | 0.006", 400g SD | | 0 | **LT** | **33** | | |
| 4 | 0.5 | SPS™-ME³, 1x | 0.006", 400g SD | 15 | 5 | **LT** | **1968** | | |
| 5 | 0.5 | SPS™-ME³, 1x | 0.006", 400g SD | 15 | 0 | **LT** | **49** | | |
| 6 | 0.5 | SPS™-ME³, 1x | 0.006", 400g SD | 15 | 5 | **LT** | **1359** | | |
| 7 | 0.5 | SPS™-SP | 2" 180grit, 700g | 15 | 0 | **LT** | **75** | | |
| 8 | 0.5 | SPS™-SP | 2" 180grit, 700g | 15 | 5 | **LT** | **4784** | | |
| 9 | 0.5 | SPS™-CE (200um 6/6) | Arc **SP-BLT2E,** 700g SD | 15 | 0 | **LT** | **63** | | |
| 10 | 0.5 | SPS™-CE (200um 6/6) | Arc **SP-BLT2E,** 700g SD | 15 | 5 | **LT** | **5622** | | |
| 11 | 0.5 | Arc 200um 3rpts, CURVED | **CPP-BLT2D** | 15 | 0 | **LT** | **100** | | |
| 13 | 0.5 | IM injection | None | 15 | -- | **LT** | **106** | | |

### Conclusions:

In this Example, three different skin disruption methods were employed. The micro-edge cubes provide a simple method for creating a groove in the skin to enhance delivery. In this embodiment, a block with sharp edges protruding at sufficient height to create a groove that extends through the stratum corneum and epidermis is used. Similarly, chemically etched surfaces designed to be loaded and pulled through the strip pull were used. Finally, an abrasive strip designed to be loaded and pulled through the strip pull was employed and all pretreatment and patch immunization regimens were compared to IM injection of the same influenza vaccine.

Immunization with influenza vaccine is ideal when subjects respond to all 3 strains included in the vaccine. As shown in Tables 7A and B above, immune responses using the patch indicate that delivery and immunity is induced for all 3 strains. For two of the strains, the use of the adjuvant enhanced the responses. However, responses to the antigen alone were strong and in two of the 3 strains no different than responses to the same dose given by the IM route. For unmet vaccine need requiring immune responses superior to response seen by the IM route use of the said invention with adjuvant and antigen may be employed.

### Example 10. Use of SPS™-ME³ System with Cross-Hatched Grooves

In this example, an area of guinea pig skin was shaved, and vaccination administered via the methods described in Example 1. The SPS™-ME³ system was administered using either 200g or 400g of force, and was administered using either a single stroke (1X) or two strokes (2X), which were applied in a cross-hatch manner, with the second stroke applied in a direction perpendicular to the original stroke. As can be seen from the Table 8, using both a single stroke and using two perpendicular strokes produced antibody titers to all three flu strains that approached or surpassed the titers resulting from an IM injection in the model. This example demonstrates the effectiveness of perpendicular grooves or furrows created using an SPS™ with a surface enabled to create grooves.

**Table 8. Serum anti-FluA/W and LT IgG titers in guinea pigs**

| Objective: Determining serum anti-FluA/W and LT IgG titers in guinea pigs | | | | | | | |
|---|---|---|---|---|---|---|---|
| Immunization dates (0 and 4wk): | | | | | | | |
| Serum collection date: 1) (preb) 2) (wk3) 3) (wk5) | | | | | | | |
| | IM prime | boost (Separate 1 cm2 dry rayon patch) | | | | Geometric | T-Test |
| Gp | (ug HA) | Pretrx | TIV (ug HA) | iLT (ug) | Test | Mean (EU) | P, log |
| 1 | 0.5 | SPS™-ME³ (1X, 200g, 0.006") | 15 | 0 | Flu A/W | 44164 | 0.02 |
| 2 | 0.5 | SPS™-ME³ (1X, 200g, 0.006") | 15 | 5 | Flu A/W | 104965 | 0.74 |
| 3 | 0.5 | SPS™-ME³ (1X, 400g, 0.006") | 15 | 0 | Flu A/W | 110845 | 0.70 |
| 4 | 0.5 | SPS™-ME³ (1X, 400g, 0.006") | 15 | 5 | Flu A/W | 172765 | 0.13 |
| 5 | 0.5 | SPS™-ME³ (2x 200g, 0.006") | 15 | 0 | Flu A/W | 62540 | 0.37 |
| 6 | 0.5 | SPS™-ME³ (2x 200g, 0.006") | 15 | 5 | Flu A/W | 209991 | 0.05 |
| 7 | 0.5 | SPS™-ME³ (2x 400g, 0.006") | 15 | 0 | Flu A/W | 118763 | 0.51 |
| 8 | 0.5 | SPS™-ME³ (2x 400g, 0.006") | 15 | 5 | Flu A/W | 341761 | 0.01 |
| 11 | 0.5 | 15 ug HA (IM) | | | Flu A/W | 95730 | # |
| | | | | | | | |
| 1 | 0.5 | SPS™-ME³ (1X, 200g, 0.006") | 15 | 0 | Flu A/NC | 8211 | 0.02 |
| 2 | 0.5 | SPS™-ME³ (1X, 200g, 0.006") | 15 | 5 | Flu A/NC | 21903 | 0.61 |
| 3 | 0.5 | SPS™-ME³ (1X, 400g, 0.006") | 15 | 0 | Flu A/NC | 27563 | 0.98 |
| 4 | 0.5 | SPS™-ME³ (1X, 400g, 0.006") | 15 | 5 | Flu A/NC | 42206 | 0.36 |
| 5 | 0.5 | SPS™-ME³ (2X, 200g, 0.006") | 15 | 0 | Flu A/NC | 22945 | 0.74 |
| 6 | 0.5 | SPS™-ME³ (2X, 200g, 0.006") | 15 | 5 | Flu A/NC | 61954 | 0.16 |
| 7 | 0.5 | SPS™-ME³ (2X, 400g, 0.006") | 15 | 0 | Flu A/NC | 43805 | 0.30 |
| 8 | 0.5 | SPS™-ME³ (2X, 400g, 0.006") | 15 | 5 | Flu A/NC | 94560 | 0.02 |
| 11 | 0.5 | 15 ug HA (IM) | | | Flu A/NC | 27316 | # |
| | | | | | | | |
| 1 | 0.5 | SPS™-ME³ (1X, 200g, 0.006") | 15 | 0 | Flu B/J | 14981 | 0.03 |
| 2 | 0.5 | SPS™-ME³ (1X, 200g, 0.006") | 15 | 5 | Flu B/J | 73099 | 0.40 |
| 3 | 0.5 | SPS™-ME³ (1X, 400g, 0.006") | 15 | 0 | Flu B/J | 80375 | 0.34 |
| 4 | 0.5 | SPS™-ME³ (1X, 400g, 0.006") | 15 | 5 | Flu B/J | 128472 | 0.06 |
| 5 | 0.3 | SPS™-ME³ (2X, 200g, 0.006") | 15 | 0 | Flu B/J | 64364 | 0.61 |
| 6 | 0.5 | SPS™-ME³ (2X, 200g, 0.006") | 15 | 5 | Flu B/J | 147281 | 0.03 |
| 7 | 0.5 | SPS™-ME³ (2X, 400g, 0.006") | 15 | 0 | Flu B/J | 88992 | 0.17 |
| 8 | 0.5 | SPS™-ME³ (2X, 400g, 0.006") | 15 | 5 | Flu B/J | 216315 | 0.01 |
| 11 | 0.5 | 15 ug HA (IM) | | | Flu B/J | 49545 | # |
| | | | | | | | |
| 1 | 0.5 | SPS™-ME³ (1X, 200g, 0.006") | 15 | 0 | LT | 104 | |
| 2 | 0.5 | SPS™-ME³ (1X, 200g, 0.006") | 15 | 5 | LT | 567 | |
| 3 | 0.5 | SPS™-ME³ (1X, 400g, 0.006") | 15 | 0 | LT | 75 | |
| 4 | 0.5 | SPS™-ME³ (1X, 400g, 0.006") | 15 | 5 | LT | 516 | |
| 5 | 0.5 | SPS™-ME³ (2X, 200g, 0.006") | 15 | 0 | LT | 58 | |
| 6 | 0.5 | SPS™-ME³ (2X, 200g, 0.006") | 15 | 5 | LT | 1214 | |
| 7 | 0.5 | SPS™-ME³ (2X, 400g, 0.006") | 15 | 0 | LT | 72 | |
| 8 | 0.5 | SPS™-ME³ (2X, 400g, 0.006") | 15 | 5 | LT | 6053 | |
| 11 | 0.5 | 15 ug HA (IM) | | | LT | 53 | |

### Example 11. Use of the SPS™-Mask and Abrade in Humans

This example evaluates the effect of various pretreatment methods and patch formulations on the immunogenicity of heat-labile enterotoxin of *Escherichia coli* (LT).

A total of 160 subjects were randomized to treatment (20 subjects in each of 8 treatment groups). All randomized subjects received the first designated treatment on Day 0 and 157/160 (98%) received the second designated treatment on Day 21 (although in the case of Group 8, the designated treatment was no treatment on Day 21). All subjects completed the Day 42 visit. With the exception of Group 8, subjects in all treatment groups were to receive 2 vaccinations, 21 days apart, using alternate arms. The duration of patch application was 6 hours for each vaccination. At the time of this report, both vaccinations had been conducted, and the Day 42 visit had been completed for all subjects. Treatment for each Group is summarized in Table 9 below.

**Table 9. Treatment Groups (Humans SLA 105)**

| **Group** | **No. of Subjects** | **Vaccination 1 (Day 0) Pretreatment** | **Vaccination 2 (Day 21) Pretreatment** | **Patch Type and Size** |
|---|---|---|---|---|
| 1 | 20 | Mask & Abrade Emery Paper 5 Strokes | Mask & Abrade Emery Paper 5 Strokes | Wet 3cm² |
| 2 | 20 | Mask & Abrade Emery Paper 2 Strokes | Mask & Abrade Emery Paper 2 Strokes | Wet 3cm² |
| 3 | 20 | Mask & Abrade Emery Paper 5 Strokes | Mask & Abrade Emery Paper 5 Strokes | Dry 3cm² |
| 4 | 20 | Mask & Abrade Emery Paper 2 Strokes | Mask & Abrade Emery Paper 2 Strokes | Dry 3cm² |
| 5 | 20 | No pretreatment Vaccination only | Mask & Abrade Emery Paper 5 Strokes | Dry 3cm² |
| 6 | 20 | Mask & Abrade Emery Paper 2 Strokes | Mask & Abrade Emery Paper 5 Strokes | Dry 3cm² |
| 7 | 20 | D-Squame® Adhesive Tape X 20 and Hydration with 10%Glycerol/Saline | D-Squame® Adhesive Tape X 20 and Hydration with 10%Glycerol/Saline | Wet 5cm² |
| 8 | 20 | Mask & Abrade Emery Paper 5 Strokes | No pretreatment No vaccination | Dry 3cm² |

Subjects were treated with the Mask & Abrade pretreatment method. This procedure uses a pretreatment abrader, comprising two acrylonitrile butadiene styrene (ABS) molded components and an internal mechanism that provides audible feedback to the user when the correct downward force is applied. An abrasive is attached to the 'foot' of the pretreatment abrader. This component was used in conjunction with a 'mask' (adhesive-backed polyester tape) that minimizes the sensation of the abrasive contacting the skin and ensures that a defined skin area is targeted for pretreatment. Subjects were pretreated using 2 or 5 strokes with the Mask & Abrade device.

### Human LT ELISA Description

Anti-LT IgG and IgA were quantified in an ELISA assay. Eight 2-fold serial-dilutions of subject's sera are added to microtiter wells coated with 1 µg/ml of the LT antigen. After an overnight incubation, the wells were washed and a peroxidase conjugated anti-human IgG (or IgA) secondary antibody is added to the wells to detect the presence of antigen-specific antibodies. Following a second incubation, the microtiter wells were again washed and the peroxidase substrate ABTS {2,2'-azino-di-(3-ethylbenzthiazoline-6-sulfonate)}was added. Cleavage of the ABTS substrate by the peroxidase enzyme results in the development of a blue-green reactant that was measured at 405 nm. The reaction was stopped by the addition of a 1% SDS solution and the optical density (OD) measured at a wavelength of 405 nm on a microplate reader. Titers were represented as the reciprocal value of the highest dilution that results in an OD 405 nm reading of 1.0. Seroconversion was defined as ≥ 2-fold rise in LT IgG titer at any time point with respect to baseline.

### Results:

Comparisons of the various pretreatment methods suggest that the 5-stroke Mask & Abrade pretreatment method, combined with the 3cm² dry patch (Group 3), produced consistently higher anti-LT IgG titers and fold ratio increases than any of the other treatments at all studied time points. The incidence of LT IgG seroconversions at Day 42 was 90% or higher for all treatment groups involving 2 vaccinations with pretreatment (Groups 1, 2, 3, 4, and 6) except the reference treatment (Group 7, D-Squame^{®} adhesive tape (20 applications) and hydration with 10% glycerol/saline).

Factorial analyses using baseline titer, type of patch (wet or dry) and number of strokes at vaccination 1 and 2 were conducted for LT IgG titers and fold ratios. Factorial analysis using LT IgG titers and fold ratios across the eight groups indicated that the dry and the wet patch produced similar results after two vaccinations, with the dry patch producing higher titers and fold ratios from Day 14 through Day 42; and statistically significantly higher titers on Day 14 [p=0.0151, generalized linear model (GLM)] and Day 21 (p=0.0135, GLM).

As shown in Table 10, the employment of the SPS™ for patch application resulted in high levels of serconversion. Anti-LT immune responses can be used for example against the enterotoxigenic E. coli that causes travelers and infant diarrhea. The ability to induce high levels of serocoversion should improve the vaccine efficacy rates. The level of serconversion with a single patch with the SPS™ in naive subjects is remarkable making this invention an important advance for vaccines.

**Table 10: Mask and Abrade with Human Subjects**

| Geometric Mean Titers | | | | | | | |
|---|---|---|---|---|---|---|---|
| | GM D0 | GM D7 | GM D14 | GM D21 | GM D28 | GM D35 | GM D42 |
| Group 1 | 802 | 804 | 2594 | 7271 | 12405 | 22832 | 22188 |
| Group 2 | 762 | 738 | 1694 | 5090 | 8618 | 12948 | 16800 |
| Group 3 | 645 | 682 | 6092 | 16734 | 22067 | 29587 | 29480 |
| Group 4 | 496 | 547 | 1745 | 7004 | 10805 | 16992 | 18304 |
| Group 5 | 659 | 740 | 715 | 714 | 926 | 5844 | 19736 |
| Group 6 | 915 | 966 | 3002 | 11163 | 17056 | 27091 | 27676 |
| Group 7 | 537 | 538 | 649 | 868 | 1049 | 1120 | 1620 |
| Group 8 | 717 | 764 | 4261 | 10247 | 13530 | 12993 | 12685 |
| | | | | | | | |

| Fold Rise | | | | | | | |
|---|---|---|---|---|---|---|---|
| | | | | | | | |

| | GM FR7 | GM FR14 | GM FR21 | GM FR28 | GM FR35 | GM FR42 | |
|---|---|---|---|---|---|---|---|
| Group 1 | 1.03 | 3.27 | 9.28 | 15.83 | 29.13 | 28.31 | |
| Group 2 | 0.97 | 2.22 | 6.68 | 11.30 | 16.98 | 23.58 | |
| Group 3 | 1.06 | 9.44 | 27.00 | 35.60 | 45.87 | 45.70 | |
| Group 4 | 1.10 | 3.52 | 14.13 | 21.80 | 34.28 | 36.93 | |
| Group 5 | 1.12 | 1.07 | 1.02 | 1.32 | 8.35 | 28.21 | |
| Group 6 | 1.06 | 3.28 | 12.20 | 18.64 | 29.60 | 30.24 | |
| Group 7 | 1.00 | 1.21 | 1.67 | 2.04 | 2.18 | 3.16 | |
| Group 8 | 1.07 | 5.94 | 14.29 | 20.10 | 19.30 | 17.32 | |
| | | | | | | | |

| Seroconversion | | | | | | | |
|---|---|---|---|---|---|---|---|
| | | | | | | | |
| | SC D7 | SC D14 | SC D21 | SC D28 | SC D35 | SC D42 | |
| Group 1 | 0% | 44% | 84% | 95% | 100% | 100% | |
| Group 2 | 0% | 30% | 80% | 90% | 95% | 100% | |
| Group 3 | 0% | 80% | 89% | 95% | 100% | 100% | |
| Group 4 | 5% | 55% | 90% | 100% | 100% | 100% | |
| Group 5 | 0% | 5% | 5% | 21% | 84% | 100% | |
| Group 6 | 0% | 50% | 95% | 95% | 100% | 100% | |
| Group 7 | 0% | 5% | 26% | 39% | 56% | 56% | |
| Group 8 | 5% | 70% | 90% | 95% | 95% | 94% | |

### Example 12. Skin Pretreatment Systems: NWAP v. Mask and Abrade

In this Example, the SPS™-mask and abrade was evaluated. The utility of an SPS™ is improved if the system is well tolerated by subjects. Several studies have shown the SPS™ to be well tolerated. In prior studies using TCI, methods for pretreatment were employed including the use of a non-woven abrasive pad (NWAP). In this Example, a direct comparison of the tolerability of the NWAP and SPS™-Mask and Abrade was performed.

**Table 11. Masking Effect**

| | **Average TEWL** | **Average Tolerability** |
|---|---|---|
| **NWAP - 15X** | 26.1 | 3.3 |
| **M&A - 5X**, **150g** | 35.5 | 1.7 |

*Description of methods:* Human volunteers received pretreatments on the deltoid with either using a non-woven abrasive pad (NWAP) or the Mask and Abrade (M&A). The arm was stroked 15 times in a downward motion with NWAP saturated with water. The second site was pretreated using the mask & abrade system with 150g sandpaper, and stroked 5 times in a downward motion. Pretreatment effectiveness was evaluated by trans-epidermal water loss (TEWL) - measured using standard methods. The patients were acclimatized for 30 minutes after pretreatment. A patient questionnaire was filled out where subjects assessed tolerability - subjects rated treatment tolerability on a 1-5 scale, where 1 means 'no problem' and 5 means 'never again'.

### Results and Conclusions:

This Example demonstrated the masking effect of the said invention. Fifteen strokes with a non-woven abrasive pad was at the limit of patient tolerability whereas use of the Mask and Abrade was well tolerated. Additionally, the Mask and Abrade generated a higher average TEWL. The TEWL achieved indicates the degree of stratum corneum disruption and correlates with delivery in both human and animal vaccine skin delivery studies.

### Example 13. Self Administration of an Active Material from a Patch

Self-administration of a vaccine or drug would add access to health care, could increase compliance, aid mass immunization campaigns and improve convenience for travelers. In this Example, pretreatment of the skin was administered by both clinicians and by patients. The present example pertains to a traveler's vaccine patch but would be expected to apply to delivery of any vaccines or drugs.

*Pretreatment:* The subjects in Groups 1-4 were pretreated as summarized below:
Group 1: Mask & Abrade, 2X, using 180 grit (g) in the deltoid.
Group 2: Mask & Abrade, 2X, using 180 grit (g) in the anterior thigh.
Group 3: Mask & Abrade, 2X, using 180 grit (g) in the lower back.
Group 4: Mask & Abrade, 2X, using 180 grit (g) in the anterior thigh (self administered).

*Vaccination:* Following clinician-administered skin pretreatment, each subject in Groups 1-4 (n=20 per group) were vaccinated by a clinician with a 50µg LT dry patch to the deltoid (Group 1), anterior thigh (Group 2),or the buttocks (Group 3), on Day 0 and Day 21 ±1 day, to the same site (opposite limb) randomized at Day 0. Following self-administered skin pretreatment, each subject in Group 4 self-vaccinated by administering a 50µg LT dry patch to the anterior thigh on Day 0 and Day 21 ±1 day, to the same site (opposite limb) randomized at Day 0.

Administration of the vaccine was achieved by applying the mask, applying two strokes of the Mask and Abrade, removing the mask and applying the LT patch. For the self-administration arm, patients were provided with written self-administration instructions. Sera were drawn at time points indicated and anti-LT IgG was evaluated as previously described below in the general methods (see also Glenn et al, Nature Medicine, 2000).

Previous studies have utilized various abrasives commonly used for electrocardiogram (EKG) electrode site preparation. In this Example, subjects in Groups 1-4 (180 grit abrasive) were pretreated either by self-administration or clinician administration, using the Mask & Abrade pretreatment method. This procedure uses a pretreatment abrader, comprising two Acrylonitrile Butadiene Styrene (ABS) molded components and an internal mechanism that provides audible feedback to the user when the correct downward force is applied. An abrasive is attached to the 'foot' of the pretreatment abrader. This component will be used in conjunction with a 'mask' (adhesive-backed polyester tape) that minimizes the sensation of the abrasive contacting the skin and ensures that a defined skin area is targeted for pretreatment. Subjects were pretreated (or pretreated themselves) using mild strokes with the Mask & Abrade.

*Administration of the vaccine Patch:* Following completion of screening procedures to confirm subject eligibility, each subject was randomized into a specific study group. Each subject had a non-hairy area on the designated randomized site demarcated with an indelible ink pen (Sharpie™).

After the designated area had been marked, the marked area was pretreated (clinician- or self-administered) with the Mask & Abrade (using the designated grit abrasive) pretreatment method.

For subjects in Groups 1 a 50µg LT dry patch with overlay was placed on the deltoid. For subjects in Group 2, a 50µg LT dry patch with overlay was placed on the anterior thigh. For subjects in Group 3, a 50µg LT dry patch with overlay was placed on the lower back. For Group 4, subjects pretreated themselves and placed the 50µg LT dry patch and overlay on the anterior thigh.

### Results:

The results are shown in Table 12.

**Table 12. Geometric mean anti-LT IgG titers, seroconversion and fold rise**

| Geometric Mean Anti-LT IgG titers | | | | |
|---|---|---|---|---|
| Day | 0 | 21 | 28 | 42 |
| Group 1 | 769 | 19663 | 23639 | 26348 |
| Group 2 | 548 | 5833 | 8285 | 13035 |
| Group 3 | 527 | 28026 | 35652 | 28598 |
| Group 4 | 975 | 8038 | 11042 | 21607 |
| | | | | |

| Seroconversion | | | | |
|---|---|---|---|---|
| day | 0 | 21 | 28 | 42 |
| Group 1 | 0.00% | 100% | 100% | 100% |
| Group 2 | 0.00% | 82% | 94% | 95% |
| Group 3 | 0.00% | 100% | 100% | 100% |
| Group 4 | 0.00% | 84% | 88% | 94% |
| | | | | |

| Fold Rise IgG | | | | |
|---|---|---|---|---|
| | 0 | 21 | 28 | 42 |
| | 1 | 25.58 | 31.46 | 35.06 |
| | 1 | 11.09 | 15.27 | 24.57 |
| | 1 | 53.87 | 71.30 | 52.74 |
| | 1 | 9.72 | 13.68 | 26.76 |

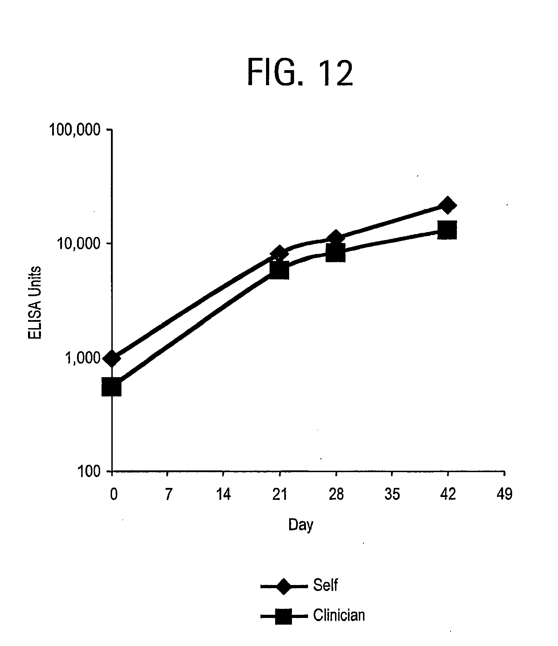

In Table 12 above, the geometric mean titers and fold rise for Groups 1-4 are indicated. In figure 12 the anti-LT IgG antibody response is shown for the comparable groups on the thigh for which there are no statistical differences at any time point. This data indicates that the SPS™ and patch can be self-administered.

In Table 12, the immune responses to each group indicate that the SPS™ is flexible and allows immunization to be performed at different body sites. The high level of serconversion for all sites indicates that this procedure is effective at inducing high levels and high frequency of immune responses. The immune responses to patch placement on the back (upper buttocks) gave a very high immune response consistent with targeting non-sun exposed skin.

In Table 12 above, the geometric mean titers and fold rise for Groups 1-4 are indicated. In figure 12 the anti-LT IgG antibody response is shown for the comparable groups on the thigh for which there are no statistical differences at any time point. These data indicate that the SPS™ and patch can be self-administered.

In Table 12, the immune responses to each group indicate that the SPS™ is flexible and allows immunization to be performed at different body sites. The high level of serconversion for all sites indicates that this procedure is effective at inducing high levels and high frequency of immune responses. The immune responses to patch placement on the back (upper buttocks) gave a very high immune response consistent with targeting non-sun exposed skin.

### Example 14: Use of the SPS™-SP with TCI

In this Example, the SPS™-SP system was used to pretreat the human subject. The human subject was vaccinated with an antigen, such as LT using a patch. The anti-LT IgG or IgA were quantified, for example in an ELISA.

*Skin Preparation:* The subjects had a non-hairy area on the deltoid demarcated with an indelible ink pen (Sharpie™). A photograph was taken, by the clinician, of the vaccination site (for all groups) prior to the vaccination. Each photo included the protocol number, subject number, initials, date, visit number, group number, and indicated whether it is the left or right side. Measures were taken to preserve the anonymity of the subject during photography.

The penetration of the SC improves the efficiency of an immune response with TCI. Previous studies have utilized various abrasives commonly used for electrocardiogram (EKG) electrode site preparation. Subjects in Groups 1,2, and 3 were pretreated by a clinician with an SPS™- Buffer composed of two assemblies; the mask assembly and the buffer strip. The mask assembly consisting of a medical-grade ABS bridge mounted die-cut medical tape defined the area of treatment. A snap dome mounted on the top of the mask assembly providing the user with audible and tactile feedback to ensure the proper force was applied as the buffer strip pull moves through the assembly. Three different buffer strips were examined for this study. The strips contained abrasive buffers similar to the abrasives that were used in Group 4.

For comparison, the site on the subjects in Group 4 was prepped using the Mask & Abrade skin preparation method (320 grit abrasive x 2 swipes). This procedure used an abrader, comprising two ABS molded components and an internal mechanism that provided audible feedback to the user when the correct downward force was applied. An abrasive was attached to the 'foot' of the skin prep abrader. This component was used in conjunction with a 'mask' (adhesive-backed polyester tape) that minimized the sensation of the abrasive contacting the skin and ensured that a defined skin area was targeted for preparation. Subjects were prepped using two (2) strokes with the Mask & Abrade.

### Administration of Investigational Vaccine:

Vaccination - Day 0 (all subjects): The vaccination site on the upper arm was demarcated with an indelible ink pen (e.g. Sharpie™). A photograph was taken of the vaccination site prior to the vaccination. Photos included the protocol number, subject number, initials, date, visit number, and indicate whether it is the left or right arm (the right arm is only to be used if the left arm cannot be). Measures were taken to preserve the anonymity of the subject during photography.

Following vaccination site preparation, subjects were vaccinated via TCI with patches. For all subjects the TIV and LT (or placebo) patches were placed on the arm by the vaccine administrator, with an adhesive overlay placed over the patch on the arm. The patches were layered, with the TIV patch placed directly in contact with the skin, the LT patch with an occlusive backing that was layered on top of that, and an adhesive overlay was placed on top that held them in place.

After 30 minutes, the arm was assessed for patch adhesion and adverse effects (AEs). Study subjects were provided with diary cards and instructed on completion of the diary cards, and then the subjects were dismissed from the clinic. Subjects were instructed to return the next day for patch removal and follow-up procedures.

Patch Removal and Follow-up: Subjects wore the patch overnight, for approximately 24 hours (range of 18-24 hours). Subjects returned to the clinic the next day following patch administration for patch removal.

*Quantification of Anti*-*LT Antibodies:* Human anti-LT IgGs were quantified by ELISA as described in Example 11.

### Results:

The results are shown in Table 13 below.

**Table 13: Quantification of Anti-LT IgG**

| **GROUP** | **Subject number** | | **Day 0** | **Day 21** | **Day 42** | **d42 Fold Rise** | **% Seroconversion** |
|---|---|---|---|---|---|---|---|
| SPS™-SP | | 106 | 1767 | 7053 | 1697 | 1 | |
| 1/2" | 12.7 mm | 127 | 369 | 363 | 2152 | 6 | |
| 1/2" | | 107 | 272 | 2682 | 2188 | 8 | |
| 1/2" | | 116 | 494 | 809 | 2985 | 6 | |
| 1/2" | | 117 | 330 | 358 | 3645 | 11 | |
| 1/2" | | 119 | 224 | 2902 | 6063 | 27 | |
| 1/2" | | 140 | 473 | 4817 | 12500 | 26 | |
| 1/2" | | 132 | 751 | 31183 | 15060 | 20 | |
| 1/2" | | 136 | 343 | 7725 | 17511 | 51 | |
| 1/2" | | 113 | 383 | 18223 | 17923 | 47 | |
| **Geomean** | | | **445** | **3226** | **5615** | **13** | 90 |
| | | | | | | | |
| SPS™- SP | | 128 | 924 | 897 | 1220 | 1 | |
| 1" | 25.4 mm | 109 | 410 | 1936 | 2422 | 6 | |
| 1" | | 125 | 126 | 122 | 3806 | 30 | |
| 1" | | 124 | 309 | 317 | 5841 | 19 | |
| 1" | | 133 | 164 | 4654 | 6088 | 37 | |
| 1" | | 118 | 452 | 7357 | 8456 | 19 | |
| 1" | | 103 | 205 | 1810 | 10540 | 51 | |
| 1" | | 114 | 553 | 24730 | 26684 | 48 | |
| 1" | | 137 | 382 | 9713 | 31599 | 83 | |
| 1" | | 101 | 478 | 73370 | 96910 | 203 | |
| **Geomean** | | | **343** | **3066** | **8839** | **26** | 90 |
| | | | | | | | |
| SPS™-SP | | 104 | 334 | 365 | Not available | | |
| 1 ½" | 38.1 mm 131 | | 1109 | 6055 | 6536 | 6 | |
| 1 ½" | | 112 | 432 | 2705 | 7589 | 18 | |
| 1 ½" | | 122 | 1219 | 1992 | 14387 | 12 | |
| 1 ½" | | 139 | 297 | 16083 | 15795 | 53 | |
| 1 ½" | | 105 | 112 | 14899 | 17960 | 160 | |
| 1 ½" | | 110 | 753 | 29489 | 24430 | 32 | |
| 1 ½" | | 135 | 693 | 6118 | 54210 | 78 | |
| 1 ½" | | 121 | 172 | 184 | 62080 | 361 | |
| 1 ½" | | 126 | 377 | 128040 | 112660 | 299 | |
| **Geomean** | | | **431** | **5110** | **23103** | **52** | 100 |
| | | | | | | | |
| M&A 2x | | 108 | 184 | 195 | 3029 | 16 | |
| M&A 2x | | 130 | 143 | 113 | 6961 | 49 | |
| M&A 2x | | 129 | 723 | 7723 | 7575 | 10 | |
| M&A 2x | | 123 | 458 | 11208 | 12385 | 27 | |
| M&A 2x | | 138 | 290 | 5063 | 13538 | 47 | |
| M&A 2x | | 111 | 934 | 29271 | 14720 | 16 | |
| M&A 2x | | 120 | 1397 | 17394 | 16043 | 11 | |
| M&A 2x | | 115 | 193 | 2045 | 16282 | 84 | |
| M&A 2x | | 134 | 416 | 41416 | 27400 | 66 | |
| M&A 2x | | 102 | 1731 | 34530 | 49880 | 29 | |
| **Geomean** | | | **464** | **5197** | **13027** | **28** | 100 |

### Conclusion:

This example demonstrates that the immune response can be increased by changing the length of the abrasive strip of the SPS™-SP. In this trial, increasing strip length from 1/2" to 1" to 1.5" produced geometric mean titer responses at Day 42 of 5,615, 8,839 and 23,103, respectively. This demonstrates the flexibility of the device to produce varying degrees of skin disruption and delivery of the therapeutic agent.

### Example 15: Use of the SPS™-ME³ and SPS™- CE

The penetration of the SC improves the efficiency of an immune response with TCI. This study evaluated two novel pretreatment methods (SPS™) to prepare the skin for vaccination. In this Example, the SPS™-ME³ and SPS™-CE systems were used to pretreat the human subject. The human subject was vaccinated with an antigen, such as LT using a patch. The anti-LT IgG and IgA were quantified, for example in an ELISA.

*Skin Preparation System: Microedge Cube (SPS*™-*ME3):* The SPS™-ME³ is an ABS component containing microedge protrusions (0.006" ± 0.0025") designed to disrupt the SC. The SPS™-ME³ assembly also contains an internal mechanism that provides feedback to the user when the correct downward force is applied. The SPS™-ME³ skin preparation was administered using one pass with the assembly in a downward motion within a template-marked area at the vaccination site. The SPS™-ME³ assemblies were single-use, and were placed biohazard bags and returned to the Sponsor after use.

*Skin Preparation System: Microedge Chemical Etch (SPS*™-*CE)*: The SPS™-CE system consists of two components: 1) a strip assembly with 316L stainless steel containing microedge protrusions (≤200µm) designed to disrupt the SC; and 2) an adhesive-backed glide assembly that contains an internal mechanism that provides feedback to the user when the correct downward force is applied for the strip to be pulled through. The SPS™-CE skin preparation was administered using one pass of the strip assembly pulled in a downward motion through the glide assembly, which was adhered to a pre-marked vaccination site area. The SPS™-CE assemblies were single-use, and were placed in biohazard bags and returned to the Sponsor after use.

### Administration of Investigational Vaccine:

Vaccination - Day 0 (all subjects): The vaccination site on the upper arm was demarcated with an indelible ink pen (e.g. Sharpie™). A photograph was taken of the vaccination site prior to the vaccination. Photos included the protocol number, subject number, initials, date, visit number, and indicate whether it is the left or right arm (the right arm is only to be used if the left arm cannot be). Measures were taken to preserve the anonymity of the subject during photography.

Following vaccination site preparation, subjects were vaccinated via TCI with patches. For all subjects the TIV and LT (or placebo) patches were placed on the arm by the vaccine administrator, with an adhesive overlay placed over the patch on the arm. The patches were layered, with the TIV patch placed directly in contact with the skin, the LT patch with an occlusive backing that was layered on top of that, and an adhesive overlay was placed on top that held them in place.

After 30 minutes, the arm was assessed for patch adhesion and AEs. Study subjects were provided with diary cards and instructed on completion of the diary cards, and then the subjects were dismissed from the clinic. Subjects were instructed to return the next day for patch removal and follow-up procedures.

Patch Removal and Follow-up: Subjects wore the patch overnight, for approximately 24 hours (range of 18-24 hours). Subjects returned to the clinic the next day following patch administration for patch removal.

*Quantification of Anti-LT Antibodies:* Human anti-LT IgGs were quantified by ELISA as described in Example 11.

### Results:

The results are shown in Tables 14A and 14B

**Table 14A: LT Delivery Using SPS™-ME³**

| | anti-LT IgG | | anti-LT IgA | |
|---|---|---|---|---|
| SUBJECT | Day 0 | Day 21 | Day 0 | Day 21 |
| A | 452 | 6,238 | 127 | 434 |
| B | 108 | 14,334 | 25 | 2,038 |
| C | 259 | 7,342 | 19 | 1,037 |
| D | 223 | 6,272 | 28 | 434 |

**Table 14B: LT Delivery Using SPS™-CE**

| SUBJECT | anti-LT IgG | |
|---|---|---|
| 1 | 259 | 7342 |

### Conclusion:

Seroconversion of these subjects (< 2-fold anti-LT IgG rise) after a single dose demonstrates the ability of the SPS™-ME³ and SPS™-CE to aid in delivery of therapeutic agents to humans.

### Example 16: Exemplary SPS™-ME³ and SPS™-CE

In this example, the SPS™-CE is a single use, disposable system composed of three assemblies: the mask assembly, snap dome, and the chemically etched pull strip.

The mask assembly consists of a medical-grade acrylonitrile butadiene styrene (ABS) bridge adhered to 3M 9952 medical tape which adheres to the skin during the skin preparation process and also defines the area of treatment. A snap dome mounted on the top of the mask assembly provides the user with audible and tactile feedback to ensure the proper force is applied to the skin as the pull strip moves through the assembly.

The pull strip consists of a strip of 316L stainless steel with parallel linear microprotrusions of a defined height and geometry. These microprotrusions are created using a chemical photoetching process then cleaned and passivated according to ASTM A967. The pull strip is mounted on a backing of 3M 9952 medical tape, and inserted into the system. The SPS™-CE provides a simple and intuitive mechanism of use, reliable audible and tactile feedback to ensure proper treatment force, and a clearly defined area of pretreatment that corresponds to patch size. Assembly of the SPS™-CE systems is as follows. The snap dome is adhered to the top of the ABS bridge, which is in turn adhered to the 9952 medical tape mask. These steps create the bridge assembly. The strip assembly is created by laminating the thin, CE microprotrusions to the adhesive part of the 9952 medical tape strip. The strip assembly is then inserted into the bridge assembly.

In this example, the SPS™-ME³ is a single use, disposable system consisting of a single assembly of multiple carbon steel #10 surgical blades mounted between medical-grade acrylonitrile butadiene styrene (ABS) blocks. In this system, the surgical blades form the parallel linear microprotrusions, and their height is manually set against the blocks using a shim. Like the SPS™-CE, a snap dome is mounted on the top of the system to provide the users with audible and tactile feedback confirming the amount of force applied. Assembly of the SPS™-ME³ is achieved by inserting two-1" (25.4 mm) 18-8 stainless steel Button Head Socket Cap screws through the holes in the ABS end block. One Havel #10 blade is then threaded over the back screw, followed by an ABS spacer block. This process is repeated 10 times. The microprotrusion height is then set by placing the front and back ends of the ME³ on 0.006" (0.1524 mm) shims and ensuring the that blade edges are touching the countertop surface. Finally, this configuration is locked by tightening the screws.

The components of each SPS™ and the source of materials are summarized in Table 15.

**Table 15. SPS™ Component Material and Source**

| **Component Name** | **Material** | **Source** |
|---|---|---|
| **SPS™-CE** | | |
| 700g snap dome | Stainless steel | Snaptron |
| Bridge | ABS | Solid Concepts |
| Mask, Base w/ Aperture | 3M 9952 medical tape | Fralock |
| Mask, Rails | 3M 9952 medical tape | Fralock |
| Strip | 3M 9952 medical tape | Fralock |
| Chemically Etched Surface | 316L stainless steel | Italix |
| Bridge-to-Mask adhesive | Permacel PO-2 | Permacel |

| **SPS™**-**ME³** | | |
|---|---|---|
| 400g snap dome | Stainless steel | Snaptron |
| End plates | ABS | Solid Concepts |
| Front Plates | ABS | Solid Concepts |
| Spacer plates | ABS | Solid |
| Screws | - | McMaster-Carr |
| Havel's #10 blades (Sterile) | Carbon steel | Havel's |

The nominal specifications for microprotrusion heights are 0.006" (0.1524 mm) for the SPS^{™} ME³ and 0.008" (0.2032 mm) for the SPS^{™}-CE. The nominal microprotrusion height is different between the two systems because of the difference in the geometry of the microprotrusions themselves.

The 0.006" (0.1524 mm) SPS^{™}-ME³ has shown optimal delivery of influenza antigens in nonclinical models and excellent tolerability in human studies. For reference, the non woven abrasive pads used in previous nonclinical and clinical studies were rated by study participants to have a tolerability of 3.3 on the scale described above. By contrast, the SPS^{™}-ME³ had a tolerability rating of 1.0. The height of 0.008" (0.2032 mm) for the SPS^{™}-CE was specified because it gave similar channel geometry in both human and guinea pig skin.

We have adopted a tolerance range of +/- 0.0015" (0.0381 mm) from nominal height for the miroprotrusions, which is within the generally accepted industry standards for etch down heights of +/- ∼ 10% of metal thickness. This is controlled in the SPS^{™}-ME³ by a manual assembly process using a shim to control and align the height of every blade.

### REFERENCES

Alving and Wassef (1994) AIDS Res. Hum. Retro., 10 (suppl. 2):S91-S94.
Antel et al. (1996) Nature Medicine, 2:1074-1075.
Ausubel et al. (1996) Current Protocols in Molecular Biology. Wiley, New York.
Bathurst et al. (1993) Vaccine, 11:449-456.
Bieber (1997) Intl. Arch. Allergy Immunol., 113:30-34.
Blum (1995) Digestion, 56:85-95.
Bodanszky (1993) Peptide Chemistry. Springer-Verlag, New York.
Burnette et al. (1994) In: Bioprocess Technology, (Burnette et al.), pp. 185-203.
Chang et al. (1989) Proc. Natl. Acad. Sci. USA, 86:6343-6347.
Chang et al. (1992) J. Imnunol., 139:548-555.
Chang et al. (1994) J. Immunol., 152:3483-3490.
Delenda et al. (1994) J. Gen. Virol., 75:1569-1578.
Deprez et al. (1996) Vaccine, 14:375-382.
Deutscher (1990) Guide to Protein Purification. Academic Press, San Diego.
Dickenson and Clements (1995) Infect. Immun., 63:1617-1623.
Douce et al. (1997) Infect. Immun., 65:28221-282218.
Elson and Dertzbaugh (1994) In: Handbook of Mucosal Immunology. Academic Press, San Diego, p. 391.
Fonseca et al. (1994) Vaccine, 12:279-285.
Frankenburg et al. (1996) Vaccine, 14:923-929.
Fries et al. (1992a) Proc. Natl. Acad. Sci. USA, 89:358-362.
Fries et al. (1992b) Infect. Inmuun., 60:1834-1839..
Glenn et al. (1995) Immunol. Lett., 47:73-78.
Glenn et al. (2000) Nature Medicine, 6(12):1403-1406.
Goeddel (1990) Gene Expression Technology. Academic Press, San Diego.
Gregoriadis (1995) Trends Biotechnol., 13(12):527-537.
Hanson and Roun (1992) In: Stability of Protein Pharmaceuticals, Vol. 3, Plenum Press, New York, pp. 209-233.
Herrington et al. (1991) Am. J. Trop. Med. Hyg., 45:695-701.
Howell and Miller (1983) J. Clin. Microbiol., 18:658-662.
Jahrling et al. (1996) Arch. Virol. Suppl., 11:135-140.
Janson and Ryden (1997) Protein Purification, 2nd Ed. Wiley, New York.
Katkov (1996) Med. Clin. North Am., 80:189-200.
Khusmith et al. (1991) Science, 252:715-718.
Kounnas et al. (1992) J. Biol. Chem., 267:12420-12423.
Kreig et al. (1995) Nature, 374:546.
Kriegler (1 990) Gene Transfer and Expression. Stockton Press, New York.
Krueger and Barbieri (1995) Clin. Microbiol. Rev., 8:34-47.
Lee and Chen (1994) Infect. Immun., 62:3594-3597.
Lieberman and Greenberg (1996) Adv. Pediatr. Infect. Dis., 11:333-363.
Lu et al. (1997) Vaccine Res., 6:1-13.
Malik et al. (1991) Proc. Natl. Acad. Sci. USA, 88:3300-3304.
Mast and Krawczynski (1996) Annu. Rev. Med., 47:257-266.
Medzhitov and Janeway (1997) Curr. Opin. Immunol., 9:4-9.
Migliorini et al. (1993) Eur. J. Immunol., 23:582-585.
Morein and Simons (1985) Vaccine, 3:83-93.
Munoz et al (1990) J. Exp. Med., 172:95-103.
Murray (1991) Gene Transfer and Expression Protocols. Humana Press, Clifton, N.J.
Nohria and Rubin (1994) Biotherapy, 7:261-269.
Pessi et al. (1991) Eur. J. Immunol., 24:2273-2276.
Pierce and Reynolds (1974) J. Immunol., 113:1017-11023.
Pierce et al. (1978) Infect. Immun., 21:185-193.
Plotkin and Mortimer (1994) Vaccines, 2nd Ed. W. B. Saunders, Philadelphia.
Rappuoli et al. (1995) Intl. Archiv. Allergy Immunol., 108:327-333.
Rappuoli et al. (1996) Adv. Exp. Med. Biol., 397:55-60.
Richards et al. (1995) In: Vaccine Design, Plenum, New York.
Saloga et al. (1996b) Exp. Dermatol., 5:65-71.
Sanchez et al. (1999) Intl. J. Pharm., 185:255-266.
Schneerson et al. (1996) Lancet, 348:1289-1292.
Scopes (1993) Protein Purification. Springer-Verlag, New York.
Shafara et al. (1995) Ann. Intern. Med., 125:658-668.
Skeiky et al. (1995) J. Exp. Med., 181:1527-1537.
Smedile et al. (1994) Prog. Liver Dis., 12:157-175.
Smucny et al. (1995) Am. J. Trop. Med. Hyg., 53:432-437.
Stacey et al. (1996) J. Immunol., 157:2116-2122.
Summers and Smith (1987) A manual of methods for baculovirus vectors and insect cell culture procedure. Texas Agricultural Experiment Station Bulletin, No. 1555.
Tam (1988) Proc. Natl. Acad. Sci. USA, 85:5409-5413.
Trach et al. (1997) Lancet, 349:231-235.
Vandenbark et al. (1996) Nature Medicine, 2:1109-1115.
Vreden et al. (1991) Am. J. Trop. Med. Hyg., 45:533-538.
Wang et al. (1995) J. Immunol., 154:2784-2793.
White et al. (1993) Vaccine, 11I:1341-1346.
Wiesmueller et al. (1991) Immunology, 72:109-113.
Wisdom (1994) Peptide Antigens. IRL Press, Oxford.
Zhang et al. (1995) Infect. Immun., 63:1349-1355.

## Claims

1. A system comprising a device for disrupting skin, said device comprising a mask and a disrupting member mechanically coupled to said mask, wherein said mask has an opening, which defines the location and the size of the skin to be disrupted, and comprises a fastener to secure said mask to skin and **characterized in that** said disrupting member further comprises:
(a) a snap dome mounted on the top of the mask assembly, and
(b) a disrupting strip,
wherein said snap dome is adapted to provide a sufficient amount of pressure, when being depressed, to disrupt the skin as the strip is pulled over said opening in said mask.

2. The system of claim 1, **characterized in that** said device further comprises:
(a) a bridge bonded onto the mask, and
(b) a button assembled so that said snap dome is positioned between the bridge and the button.

3. The system of claim 1 or 2, **characterized in that** said device further comprises an interlocking mechanism, comprising a clip bonded to the strip, wherein:
(a) when no force is applied to the snap dome or button, the strip is captured inside the bridge; and
(b) when the prescribed force is applied to the snap dome or button, the button depresses and releases the strip to be pulled out by a user.

4. The system of any of claims 1 to 3, further comprising a reservoir comprising a formulation.

5. The system of claim 4, wherein said reservoir is a patch.

6. The system of claim 4 or 5, wherein the formulation comprises a therapeutic agent.

7. The system of claim 4 or 5, wherein said formulation comprises an antigen and a pharmaceutically acceptable carrier, wherein said antigen is capable of inducing an immune response when applied transcutaneously on the area of the subject's skin treated with said system; and wherein treating the area of the skin with said system enhances the immune response.

8. The system of claim 7, wherein said formulation further comprises an adjuvant in an amount effective to enhance the immune response to the antigen.

9. The system of claim 8, wherein the adjuvant and the antigen is a single molecule having both adjuvant and antigen properties.

10. The system of claim 4 or 5, wherein said formulation consists of an adjuvant and a pharmaceutically acceptable carrier and is capable of enhancing an antigen-specific immune response when applied transcutaneously on the area of the subject's skin treated with said system, and wherein the antigen is not administered by a transcutaneous route.

11. The system of any of claims 1 to 10, wherein said disrupting strip comprises a metal or ceramic abrasive.

12. The system of any of claims 1 to 11, wherein said fastener comprises an adhesive.

13. The system of any of claims 1 to 12, wherein said mask further comprises an opening for marking the skin while said mask is adhered to the skin, wherein a marking on the skin made through said opening defines the location of disruption.

14. The system of any of claims 1 to 12, wherein said mask further comprises an indicator disposed to transfer to the skin when the mask is fastened thereto, wherein said indicator denotes the location of skin disruption.

15. The system of any of claims 5 to 10, wherein said patch further comprises an adhesive for adhering to skin and a release liner disposed over said adhesive.

## Patentansprüche

1. System das eine Vorrichtung zur Hautdurchtrennung umfasst, wobei die Vorrichtung eine Maske und ein Durchtrennungsstück, das mechanisch an die Maske gebunden ist, umfasst, wobei die Maske eine Öffnung hat, die die Lage und Größe der zu durchtrennenden Haut definiert, und ein Befestigungsmittel umfasst, um die Maske an der Haut zu sichern, und **dadurch gekennzeichnet, dass** das Durchtrennungsstück zusätzlich
(a) eine Schnappscheibe, die auf der Oberseite des Maskenaufbaus angebracht ist, und
(b) einen Durchtrennungsstreifen umfasst,
wobei die Schnappscheibe angepasst ist eine ausreichende Druckmenge bereit zu stellen, um, wenn gedrückt, die Haut zu durchtrennen während der Streifen über die Öffnung in der Maske gezogen wird.

2. System nach Anspruch 1, **dadurch gekennzeichnet, dass** die Vorrichtung zusätzlich
(a) einen Steg, der an die Maske gebunden ist, und
(b) einen Knopf, der so angebracht ist, dass die Schnappscheibe zwischen dem Steg und dem Knopf positioniert ist,
umfasst.

3. System nach Anspruch 1 oder 2, **dadurch gekennzeichnet, dass** das System zusätzlich einen Verriegelungsmechanismus umfasst, umfassend eine Lasche die an den Streifen gebunden ist, wobei
(a) wenn keine Kraft auf die Schnappscheibe oder den Knopf angewendet wird, der Streifen innerhalb des Stegs gefangen ist; und
(b) wenn die vorgeschriebene Kraft auf die Schnappscheibe oder den Knopf angewendet wird, der Knopf niederdrückt und den durch den Verwender zu ziehenden Streifen freilässt.

4. System nach einem der Ansprüche 1 bis 3, zusätzlich umfassend ein Reservoir umfassend eine Formulierung.

5. System nach Anspruch 4, wobei das Reservoir ein Füllstück ist.

6. System nach Anspruch 4 oder 5, wobei die Formulierung ein therapeutisches Mittel umfasst.

7. System nach Anspruch 4 oder 5, wobei die Formulierung ein Antigen und ein pharmazeutisch verträgliches Trägermittel umfasst, wobei das Antigen in der Lage ist eine Immunantwort hervorzurufen wenn es transkutan in dem Bereich der Haut des Patienten, der mit dem System behandelt wird, appliziert wird; und wobei Behandlung des Bereichs der Haut mit dem System die Immunantwort verstärkt.

8. System nach Anspruch 7, wobei die Formulierung zusätzlich einen Hilfsstoff in einer Menge umfasst, die effektiv ist die Immunantwort auf das Antigen zu verstärken.

9. System nach Anspruch 8, wobei der Hilfsstoff und das Antigen ein einzelnes Molekül sind, das sowohl Hilfsstoff- als auch Antigeneigenschaften hat.

10. System nach Anspruch 4 oder 5, wobei die Formulierung aus einem Hilfsstoff und einem pharmazeutisch verträglichen Trägermittel besteht und in der Lage ist eine antigen-spezifische Immunantwort zu verstärken, wenn sie transkutan in dem Bereich der Haut des Patienten, der mit dem System behandelt wird, appliziert wird, und wobei das Antigen nicht über einen transkutanen Weg verabreicht wird.

11. System nach einem der Ansprüche 1 bis 10, wobei der Durchtrennungsstreifen ein metallisches oder keramisches Schleifmittel umfasst.

12. System nach einem der Ansprüche 1 bis 11, wobei das Befestigungsmittel ein Haftmittel umfasst.

13. System nach einem der Ansprüche 1 bis 12, wobei die Maske zusätzlich eine Öffnung zur Markierung der Haut umfasst, während die Maske an der Haut befestigt ist, wobei eine Markierung der Haut, die durch die Öffnung gemacht wird, die Lage der Durchtrennung anzeigt.

14. System nach einem der Ansprüche 1 bis 12, wobei die Maske zusätzlich eine Anzeigevorrichtung umfasst, die angewendet wird um sich auf die Haut zu übertragen wenn die Maske daran befestigt wird, wobei die Anzeigevorrichtung die Lage der Hautdurchtrennung anzeigt.

15. System nach einem der Ansprüche 5 bis 10, wobei das Füllstück zusätzlich ein Haftmittel zur Haftung an Haut und eine abziehbare Abdichtung, die über dem Haftmittel angebracht ist, umfasst.

## Revendications

1. Système comprenant un dispositif pour séparer la peau, ledit dispositif comprenant un masque et un élément de séparation couplé mécaniquement audit masque, ledit masque comportant une ouverture qui définit la position et la dimension de la peau à séparer, et comprenant un dispositif de fixation servant à fixer ledit masque à la peau et **caractérisé en ce que** ledit élément de séparation comprend en outre :
(a) une calotte à pression montée au sommet de l'ensemble masque, et
(b) une bande de séparation,
dans lequel ladite calotte à pression est conçue pour fournir une quantité suffisante de pression, lorsqu'elle est enfoncée, pour séparer la peau lorsque la bande est tirée au-dessus de ladite ouverture réalisée dans ledit masque.

2. Système selon la revendication 1, **caractérisé en ce que** ledit dispositif comprend en outre :
(a) une arcade collée sur le masque, et
(b) un bouton assemblé de sorte que ladite calotte à pression est disposée entre l'arcade et le bouton.

3. Système selon la revendication 1 ou 2, **caractérisé en ce que** ledit dispositif comprend en outre un mécanisme de verrouillage mutuel, comprenant une attache liée à la bande, dans lequel :
(a) la bande est capturée à l'intérieur de l'arcade lorsque qu'aucune force n'est appliquée à la calotte à pression ou au bouton ; et
(b) le bouton enfonce et libère la bande devant être retirée par un utilisateur lorsque la force prescrite est appliquée à la calotte à pression ou au bouton.

4. Système selon l'une quelconque des revendications 1 à 3, comprenant en outre un réservoir comprenant une formulation.

5. Système selon la revendication 4, dans lequel ledit réservoir est un patch.

6. Système selon la revendication 4 ou 5, dans lequel la formulation comprend un agent thérapeutique.

7. Système selon la revendication 4 ou 5, dans lequel ladite formulation comprend un antigène et un véhicule pharmaceutiquement acceptable, ledit antigène pouvant induire une réponse immunitaire lorsqu'il est appliqué de manière transcutanée à la zone de la peau du sujet traité au moyen dudit système ; et dans lequel le traitement de la zone de la peau au moyen dudit système améliore la réponse immunitaire.

8. Système selon la revendication 7, dans lequel ladite formulation comprend en outre un adjuvant en quantité suffisante pour améliorer la réponse immunitaire à l'antigène.

9. Système selon la revendication 8, dans lequel l'adjuvant et l'antigène constituent une seule molécule ayant les propriétés à la fois d'un adjuvant et d'un antigène.

10. Système selon la revendication 4 ou 5, dans lequel ladite formulation est constituée d'un adjuvant et d'un véhicule pharmaceutiquement acceptable et peut améliorer une réponse immunitaire spécifique à un antigène lorsque qu'elle est appliquée de manière transcutanée à la zone de la peau d'un sujet traité au moyen dudit système, et dans lequel l'antigène n'est pas administré par voie transcutanée.

11. Système selon l'une quelconque des revendications 1 à 10, dans lequel ladite bande de séparation comprend un abrasif métallique ou céramique.

12. Système selon l'une quelconque des revendications 1 à 11, dans lequel ledit dispositif de fixation comprend un adhésif.

13. Système selon l'une quelconque des revendications 1 à 12, dans lequel ledit masque comprend en outre une ouverture permettant de marquer la peau tandis que ledit masque adhère à la peau, un marquage de la peau réalisé à travers ladite ouverture définissant la position de séparation.

14. Système selon l'une quelconque des revendications 1 à 12, dans lequel ledit masque comprend en outre un indicateur disposé pour être transféré à la peau lorsque ledit masque y est fixé, ledit indicateur désignant la position de séparation de la peau.

15. Système selon l'une quelconque des revendications 5 à 10, dans lequel ladite pièce comprend en outre un adhésif destiné à adhérer à la peau et une pellicule détachable disposée sur ledit adhésif.
